# EUROPÄISCHE PATENTSCHRIFT

(11) **EP 2 569 323 B1**
(45) Veröffentlichungstag und Bekanntmachung des Hinweises auf die Patenterteilung: **25.02.2015**
(21) Anmeldenummer: 11715672.9
(22) Anmeldetag: 20.04.2011
(51) Int. Cl.: C07F 15/00, H01L 51/50

(54) **METALLKOMPLEXE**
METAL COMPLEXES
COMPLEXES MÉTALLIQUES

(30) Priorität: 14.05.2010 DE 102010020567
(43) Veröffentlichungstag der Anmeldung: 20.03.2013
(73) Patentinhaber: Merck Patent GmbH, 64293 Darmstadt (DE)
(72) Erfinder: ANÈMIAN, Rémi, Manouk, 60316 Frankfurt am Main (DE); SCHROEDER, Bernd, 65606 Villmar-Weyer (DE); PARHAM, Amir, Hossain, 65929 Frankfurt am Main (DE); NONANCOURT, Claire de, 65929 Frankfurt am Main (DE)
(86) Internationale Anmeldenummer: PCT/EP2011/002025
(87) Internationale Veröffentlichungsnummer: WO 2011/141120

(56) Entgegenhaltungen:
- WO-A1-2008/009343
- WO-A2-2004/026886
- US-A1- 2004 131 880
- A. V. Usatov ET AL: "Organometallic hydrides as reactants in fullerene chemistry. Interaction of C60 and C70 fullerenes with HIr(CO)(PPh3)3", RUSSIAN CHEMICAL BULLETIN, vol. 43, no. 9, 1 September 1994 (1994-09-01), pages 1572-1576, XP055117469, ISSN: 1066-5285, DOI: 10.1007/BF00697152

## Beschreibung

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, jedoch immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und blau, emittieren. Weiterhin weisen viele phosphoreszierende Emitter keine ausreichende Löslichkeit für eine Verarbeitung aus Lösung auf, so dass es auch hier weiteren Verbesserungsbedarf gibt.

Gemäß dem Stand der Technik werden in phosphoreszierenden OLEDs als Triplettemitter insbesondere Iridium- und Platinkomplexe eingesetzt, welche üblicherweise als cyclometallierte Komplexe eingesetzt werden. Dabei sind die Liganden häufig Derivate von Phenylpyridin. Jedoch ist die Löslichkeit derartiger Komplexe häufig gering, was eine Verarbeitung aus Lösung erschwert oder gänzlich verhindert.

Aus dem Stand der Technik sind Iridiumkomplexe bekannt, welche am Phenylring des Phenylpyridinliganden in para-Position zur Koordination an das Metall mit einer gegebenenfalls substituierten Aryl- oder Heteroarylgruppe substituiert sind (WO 2004/026886 A2). Dadurch wurde eine verbesserte Löslichkeit der Komplexe erzielt. Jedoch gibt es auch hier noch weiteren Verbesserungsbedarf in Bezug auf die Löslichkeit sowie die Effizienz und die Lebensdauer der Komplexe.

Überraschend wurde gefunden, dass bestimmte, unten näher beschriebene Metallchelatkomplexe eine verbesserte Löslichkeit aufweisen und weiterhin zu Verbesserungen der organischen Elektrolumineszenzvorrichtung führen, insbesondere hinsichtlich der Effizienz und der Lebensdauer. Diese Metallkomplexe und organische Elektrolumineszenzvorrichtungen, welche diese Komplexe enthalten, sind daher der Gegenstand der vorliegenden Erfindung.

Gegenstand der Erfindung ist eine Verbindung gemäß Formel (1),

M(L)ₙ(L')ₘ Formel (1)

wobei die Verbindung der allgemeinen Formel (1) eine Teilstruktur M(L)ₙ der Formel (2) enthält: 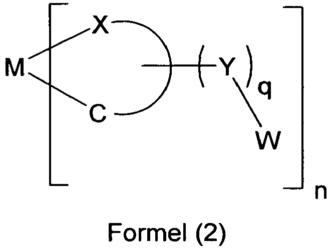 wobei M an einen beliebigen bidentaten Liganden L über X und ein Kohlenstoffatom C bindet und
wobei für die verwendeten Symbole und Indices gilt:
- M: ist ein Metall ausgewählt aus der Gruppe bestehend aus Iridium, Rhodium, Platin und Palladium;
- X: ist gleich oder verschieden bei jedem Auftreten CR¹ oder N;
- Y: ist gleich oder verschieden bei jedem Auftreten eine Einfachbindung oder eine bivalente Gruppe ausgewählt aus C(R¹)₂, C(=O), O, S, SO, SO₂, NR¹, PR¹ oder P(=O)R¹; ein aliphatischer, aromatischer oder heteroaromatischer Kohlenwasserstoff mit 5 bis 60 Atomen, der jeweils durch einen oder mehrere Reste R³ substituiert sein kann;
- R¹: ist gleich oder verschieden bei jedem Auftreten H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen; dabei können zwei oder mehr Reste R¹ auch miteinander ein mono- oder polycyclisches, aliphatisches, aromatisches und/oder benzoannelliertes Ringsystem bilden;
- R²: ist gleich oder verschieden bei jedem Auftreten H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination aus zwei oder mehr dieser Gruppen; dabei können zwei oder mehrere benachbarte Reste R² miteinander ein mono- oder polycyclisches, aliphatisches oder aromatisches Ringsystem bilden;
- R³: ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches oder aromatisches Ringsystem bilden;
- L': ist gleich oder verschieden bei jedem Auftreten ein beliebiger Coligand;
- W: ist gleich der Formel (3) 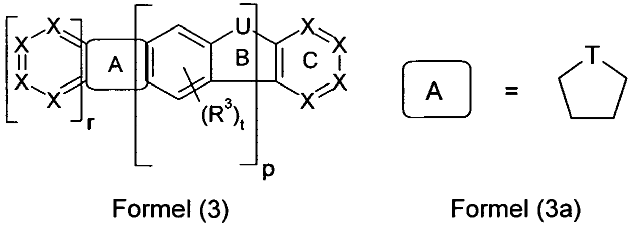 wobei der Ring A ein beliebiger substituierter oder unsubstituierter aliphatischer, aromatischer oder heteroaromatischer Ring mit 5 bis 60 Atomen oder ein substituiertes oder unsubstituiertes polycylisches Ringsystem sein kann, das in jeder möglichen Weise mit den benachbarten Ringen kondensiert sein kann, wobei in einer bevorzugten Ausführungsform der Ring A die Verbindung der Formel (3a) ist, der in jeder möglichen Weise mit den benachbarten Ringen kondensiert sein kann;
- T, U: sind gleich oder verschieden bei jedem Auftreten ausgewählt aus der Gruppe bestehend aus -C(R¹)₂, -Si(R¹)₂, N, -NR¹, -O,- S, -C(=O),-S(=O), -SO₂, -CF₂, -SF₄, -P,- P(=O)R¹, -PF₂, -P(=S)R¹,- As, -As(=O),-As(=S), -Sb, -Sb(=O) und -Sb(=S);
- q, r: sind unabhängig voneinander 0 oder 1;
- p: ist größer oder gleich 1;
- t: ist 0, 1 oder 2;
- n: ist 1, 2 oder 3 für M gleich Iridium oder Rhodium und ist 1 oder 2 für M gleich Platin oder Palladium;
- m: ist 0, 1, 2, 3 oder 4;
dabei werden die Indizes n und m so gewählt, dass die Koordinationszahl am Metall für M gleich Iridium oder Rhodium 6 entspricht und für M gleich Platin oder Palladium 4 entspricht;
dabei können auch mehrere Liganden L miteinander oder L mit L' über eine beliebige Brücke Z verknüpft sein und so ein tridentates, tetradentates, pentadentates oder hexadentates Ligandensystem aufspannen.

In einer bevorzugten Ausführungsform der vorliegenden Erfindung enthält die Verbindung der allgemeinen Formel (1) eine Teilstruktur M(L)ₙ der Formel (4) bis Formel (20) 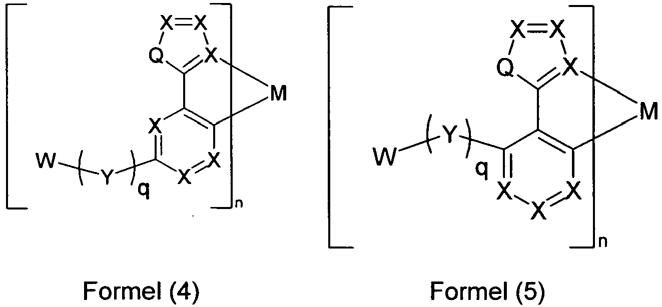 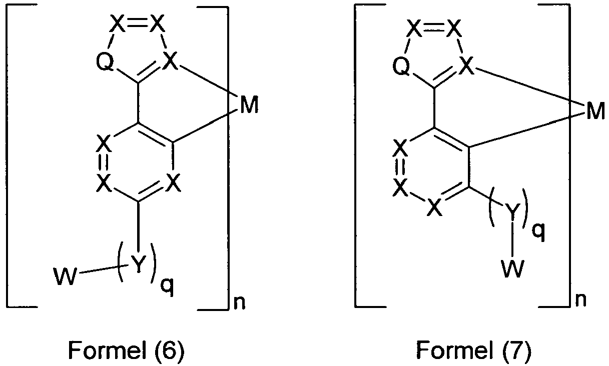 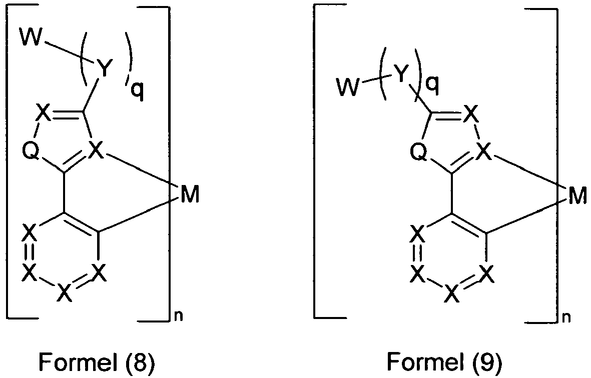 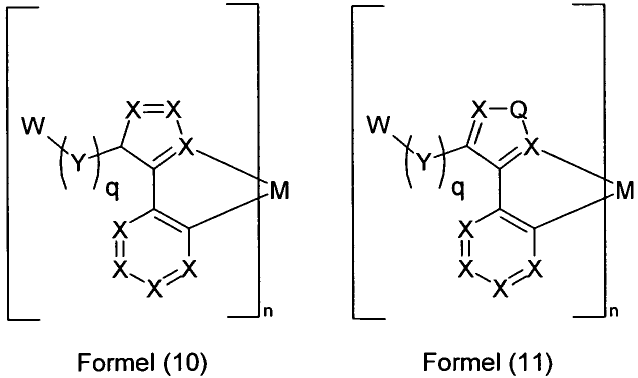 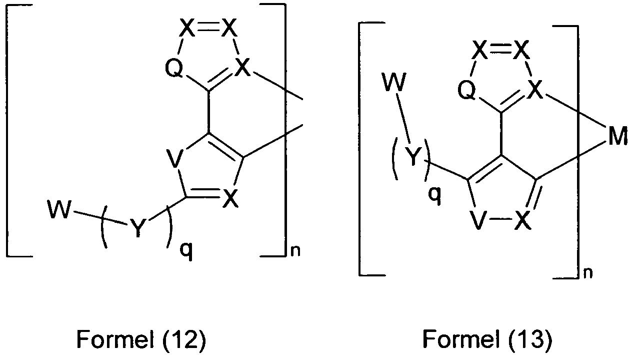 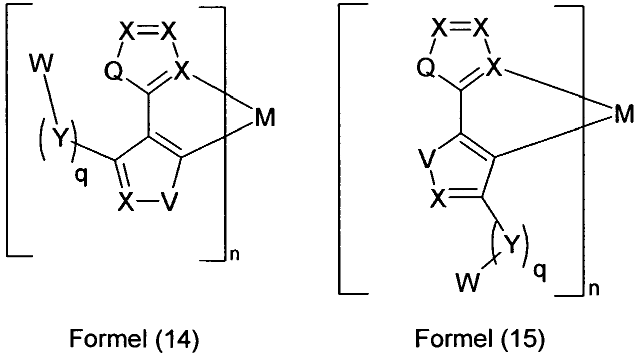 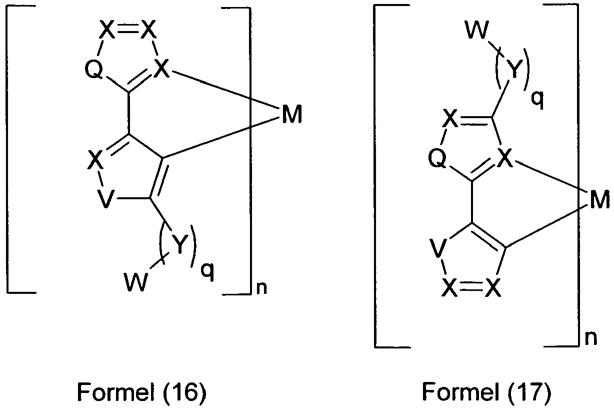 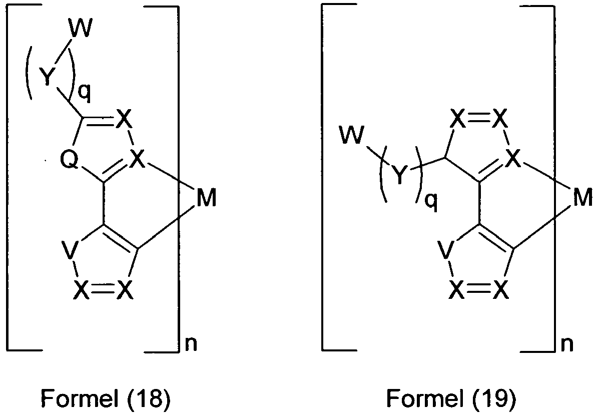 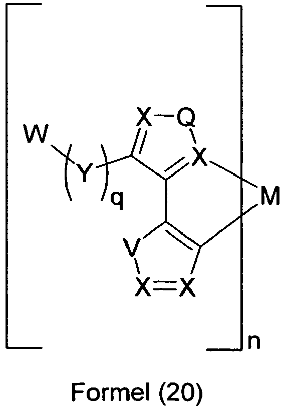 wobei für die verwendeten Symbole und Indizes gilt:
- Q: ist gleich oder verschieden bei jedem Auftreten R¹C=CR¹, R¹C=N, O, S, Se oder NR¹;
- V: ist gleich oder verschieden bei jedem Auftreten O, S, Se, NR¹ oder C(R¹)₂;

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl oder 2,2,2-Trifluorethyl verstanden. Unter einer Alkenylgruppe werden beispielsweise Ethenyl, Propenyl, Butenyl, Pentenyl, Cyclo-pentenyl, Hexenyl, Cyclohexenyl, Heptenyl, Cycloheptenyl, Octenyl, Cyclo-octenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spiro-bifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Verbindungen gemäß Formel (1) können elektrisch geladen oder ungeladen sein. In einer bevorzugten Ausführungsform sind die Verbindungen der Formel (1) elektrisch neutral. Dies wird auf einfache Weise dadurch erreicht, dass die Ladung der Liganden L und L' so gewählt werden, dass sie die Ladung des komplexierten Metallatoms M kompensieren.

Bevorzugt sind weiterhin Verbindungen gemäß Formel (1), dadurch gekennzeichnet, dass die Summe der Valenzelektronen um das Metallatom für Platin und Palladium 16 und für Iridium oder Rhodium 18 beträgt. Diese Bevorzugung ist durch die besondere Stabilität dieser Metallkomplexe begründet.

In einer bevorzugten Ausführungsform der Erfindung steht M für Iridium oder Platin. Besonders bevorzugt steht M für Iridium.

Wenn M für Platin oder Palladium steht, steht der Index n für 1 oder 2. Wenn der Index n = 1 ist, sind noch ein bidentater oder zwei monodentate Liganden L', bevorzugt ein bidentater Ligand L', an das Metall M koor-diniert. Entsprechend ist für einen bidentaten Liganden L' der Index m = 1 und für zwei monodentate Liganden L' der Index m = 2. Wenn der Index n = 2 ist, ist der Index m = 0.

Wenn M für Iridium oder Rhodium steht, steht der Index n für 1, 2 oder 3, bevorzugt für 2 oder 3 und besonders bevorzugt für 3. Wenn der Index n = 1 ist, sind noch vier monodentate oder zwei bidentate oder ein bidentater und zwei monodentate oder ein tridentater und ein monodentater oder ein tetradentater Ligand L', bevorzugt zwei bidentate Liganden L', an das Metall koordiniert. Entsprechend ist der Index m, je nach Liganden L', gleich 1, 2, 3 oder 4. Wenn der Index n = 2 ist, sind noch ein bidentater oder zwei monodentate Liganden L', bevorzugt ein bidentater Ligand L', an das Metall koordiniert. Entsprechend ist der Index m, je nach Liganden L', gleich 1 oder 2. Wenn der Index n = 3 ist, ist der Index m = 0.

In einer bevorzugten Ausführungsform der Erfindung steht das Symbol X gleich oder verschieden bei jedem Auftreten für CR¹.

In einer weiteren bevorzugten Ausführungsform der Erfindung stehen entweder alle Symbole X gleich oder verschieden bei jedem Auftreten für CR¹ oder alle Symbole X stehen für N.

In einer weiteren bevorzugten Ausführungsform der Erfindung steht das Symbol Q gleich oder verschieden bei jedem Auftreten für R¹C=CR¹, R¹C=N, O oder NR¹, besonders bevorzugt für R¹C=CR¹ und R¹C=N und insbesondere bevorzugt für R¹C=CR¹.

In einer weiteren bevorzugten Ausführungsform der Erfindung steht das Symbol V gleich oder verschieden bei jedem Auftreten für O, S oder NR¹, besonders bevorzugt für S oder NR¹ und insbesondere bevorzugt für S.

In einer weiteren bevorzugten Ausführungsform der Erfindung steht das Symbol Y für eine Einfachbindung oder eine bivalente Gruppe ausgewählt aus C(=O) oder NR¹, besonders bevorzugt für eine Einfachbindung.

Es ist besonders bevorzugt, wenn die oben genannten Bevorzugungen gleichzeitig gelten.

In einer besonders bevorzugten Ausführungsform der Erfindung gilt für die verwendeten Symbole:
- M: ist Iridium oder Platin, besonders bevorzugt Iridium;
- X: ist bei jedem Auftreten für alle Positionen, die nicht direkt an M gebunden sind, CR¹;
- Q: ist gleich oder verschieden bei jedem Auftreten R¹C=CR¹ oder R¹C=N, besonders bevorzugt R¹C=CR¹;
- V: ist gleich oder verschieden bei jedem Auftreten O, S oder NR¹, besonders bevorzugt S;
- Y: ist gleich oder verschieden bei jedem Auftreten eine Einfachbindung oder eine bivalente Gruppe ausgewählt aus C(=O) oder NR¹, besonders bevorzugt eine Einfachbindung.

Besonders bevorzugt im Sinne der vorliegenden Erfindung sind Verbindungen der Formeln (4) und (12).

In einer besonders bevorzugten Ausführungsform der Erfindung sind die Teilstrukturen der Formel (4) oder (12) ausgewählt aus den Teilstrukturen der folgenden Formeln (21), (22), (23) oder (24),

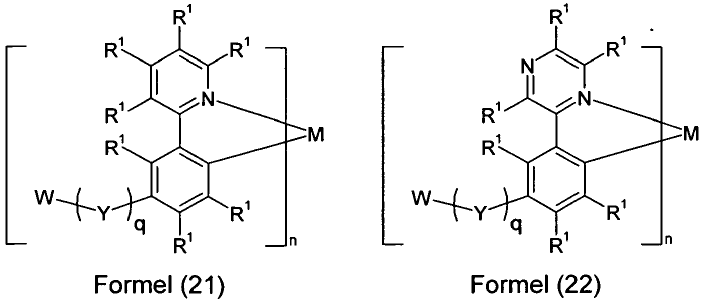

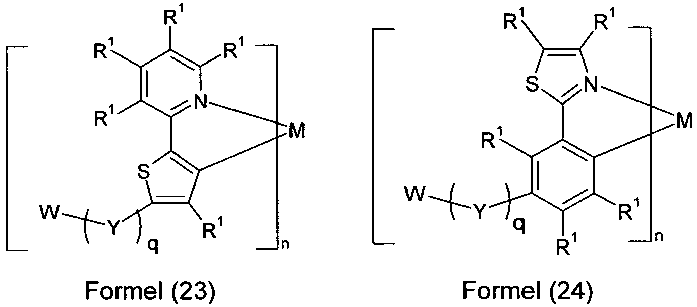

Eine insbesondere bevorzugte Ausführungsform der vorliegenden Erfindung sind Verbindungen der Formel (25).

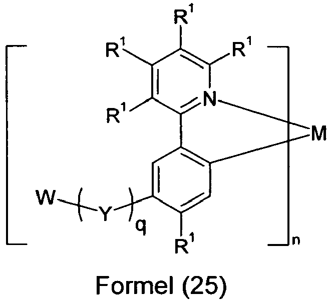

Eine weitere insbesondere bevorzugte Ausführungsform der vorliegenden Erfindung sind Verbindungen der Formel (26).

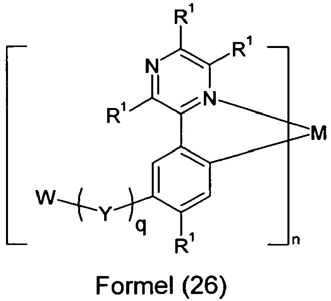

Eine weitere insbesondere bevorzugte Ausführungsform der vorliegenden Erfindung sind Verbindungen der Formel (27).

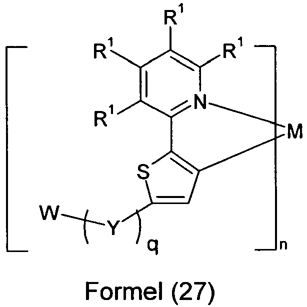

Eine weitere insbesondere bevorzugte Ausführungsform der vorliegenden Erfindung sind Verbindungen der Formel (28).

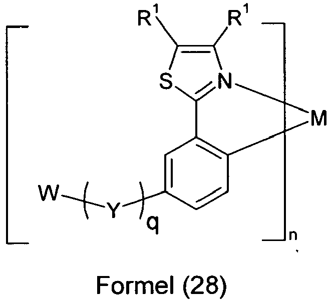

Weiterhin sind die folgenden Verbindungen besonders bevorzugt.

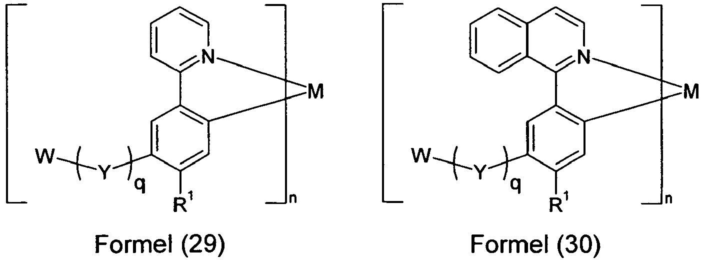

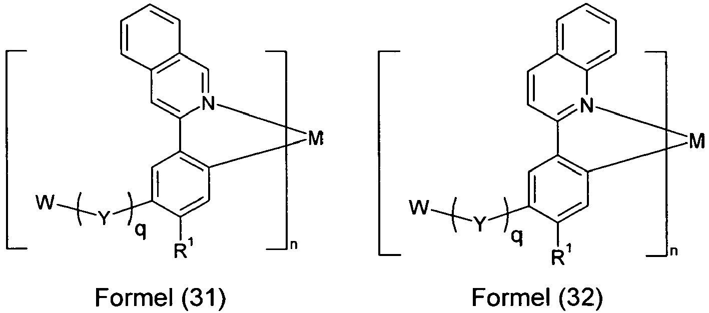

In einer bevorzugten Ausführungsform der vorliegenden Erfindung ist Y ausgewählt aus der Gruppe der aromatischen oder heteroaromatischen Kohlenwasserstoffe mit 5 bis 60 Atomen, der jeweils durch einen oder mehrere Reste R³ substituiert sein kann.

Besonders bevorzugt für Y sind die Verbindungen der folgenden Formeln (33) bis (63). 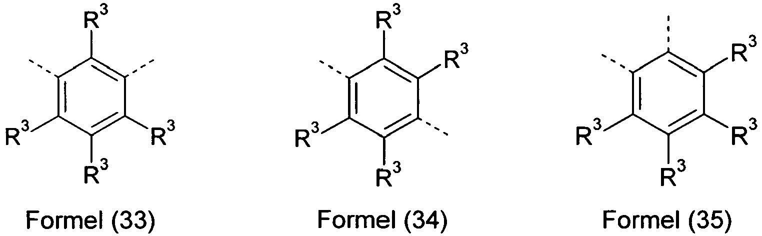 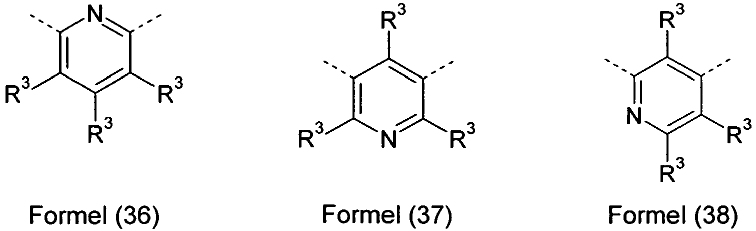 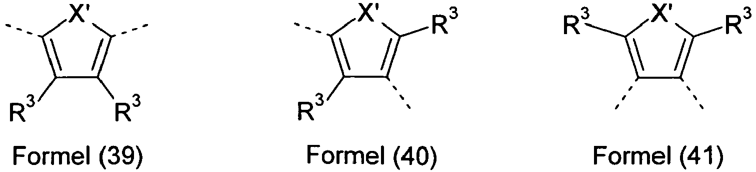 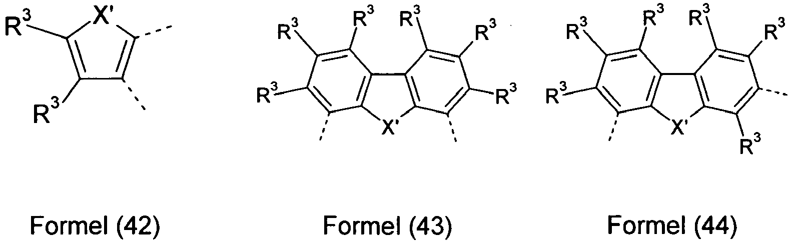 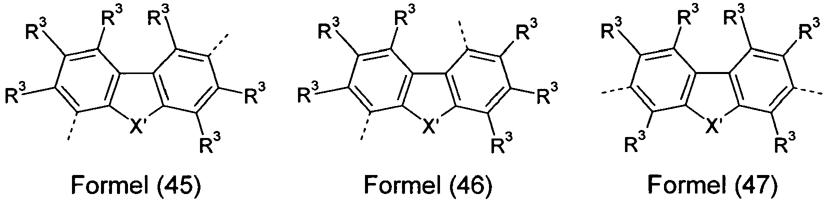 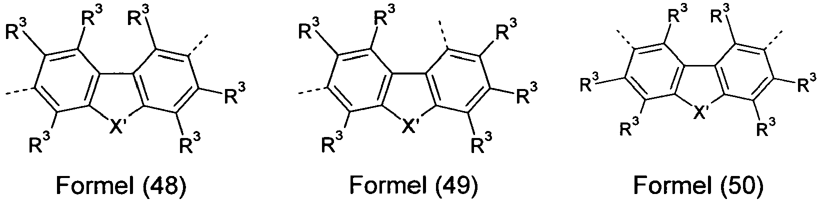 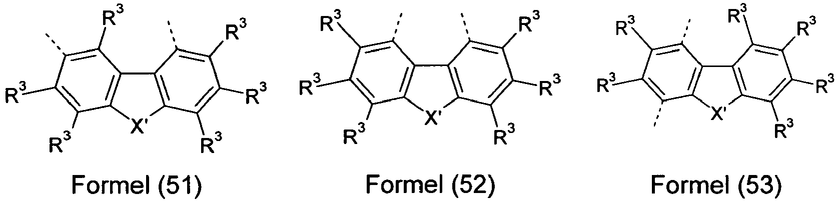 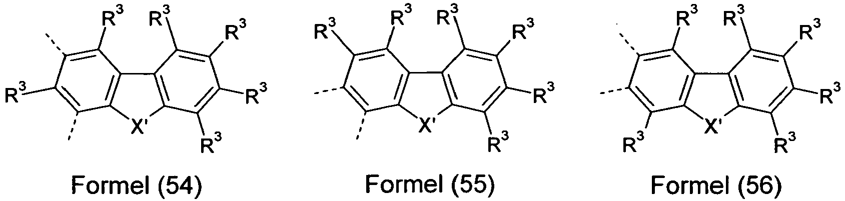 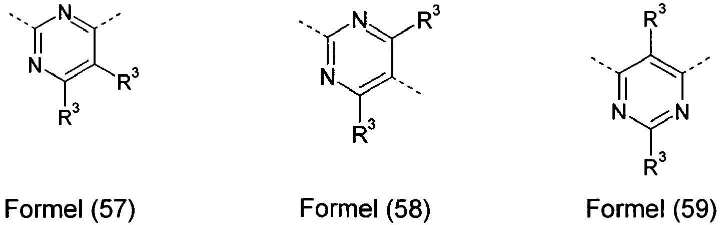 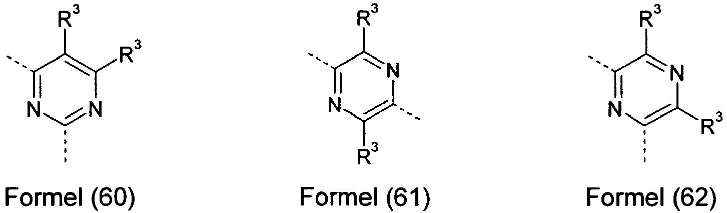 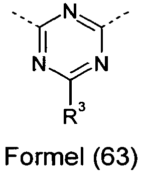 X' ist hierbei O, S, Se, NR¹, C(R¹)₂ oder S(=O)₂.
Die gepunktete Linie steht für die Verknüpfungen zu W bzw. zu dem direkt an das Metall M gebundenen Teil des organischen Liganden.

Ganz besonders bevorzugt für Y sind die Verbindungen der Formeln (33), (36), (37), (39), (40), und (48).

Eine weiterhin besonders bevorzugte Verbindung für Y ist die Verbindungen der Formeln (63).

In einer bevorzugten Ausführungsform der vorliegenden Erfindung ist W dadurch gekennzeichnet, dass T und U unabhängig voneinander ausgewählt werden aus der Gruppe bestehend aus -C(R¹)₂, -N, -NR¹, -O, oder-S.

Die Verknüpfung von W und Y erfolgt bevorzugt über den Ring B oder den Ring C. Erfolgt die Verknüpfung von W und Y über den Ring B so stellt die Verbindung der folgenden Formel (64) eine besonders bevorzugte Ausführungsform von W dar.

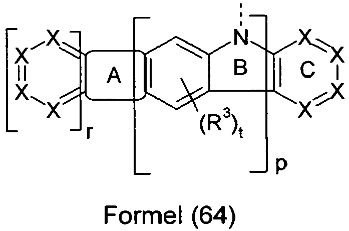

Weitere besonders bevorzugte Verbindungen der Formel (3) sind Verbindungen der Formel (65).

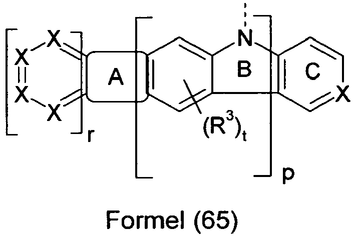

Wenn p > 1 ist, kann die Verknüpfung von W und Y über jeden der auftretenden B-Ringe erfolgen. Besonders bevorzugt ist, wenn die Verknüpfung zwischen W und Y an dem B-Ring erfolgt, der dem C-Ring direkt benachbart ist.

In einer ganz besonders bevorzugten Ausführungsform der vorliegenden Erfindung gilt r = 1 und p = 1 und besonders bevorzugte Verbindungen der Formel (3) sind die Verbindungen mit den Formeln (66) bis (71),

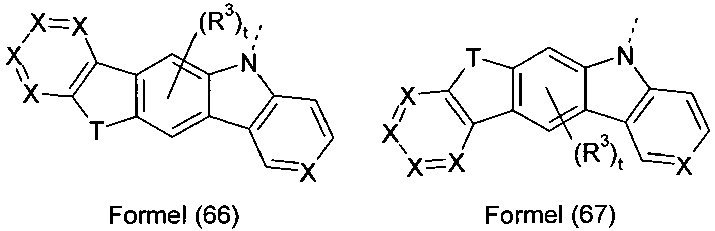

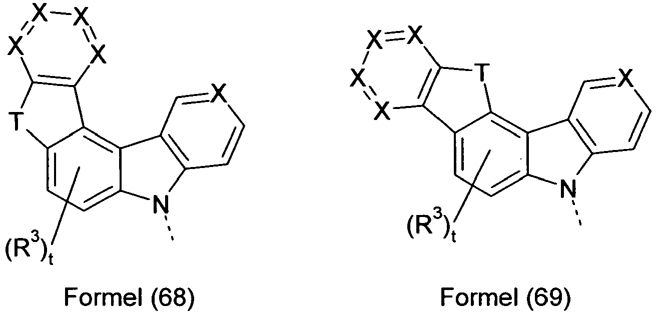

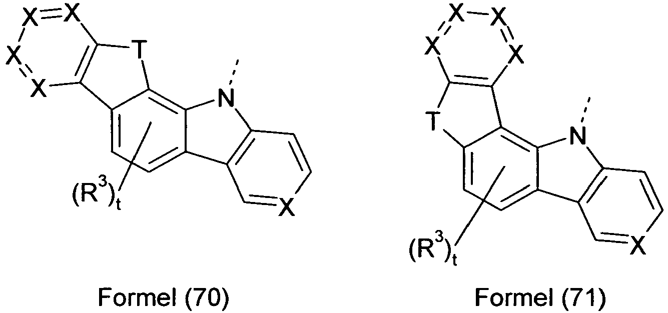

Weiterhin bevorzugt für die Verbindungen der Formel (3) sind die Verbindungen der folgenden Formeln (72) to (77).

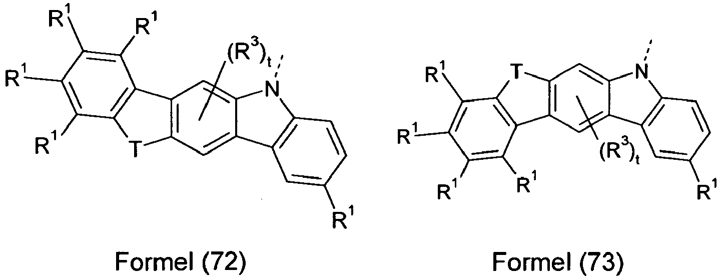

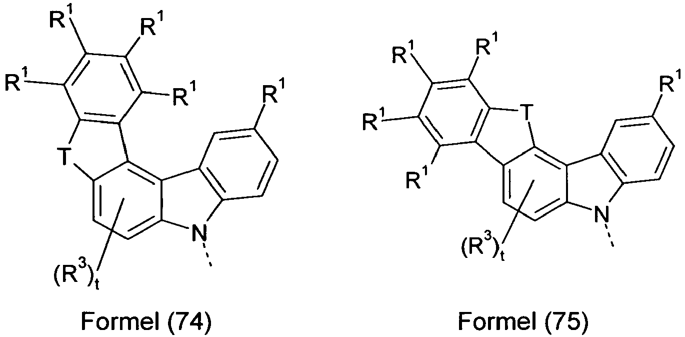

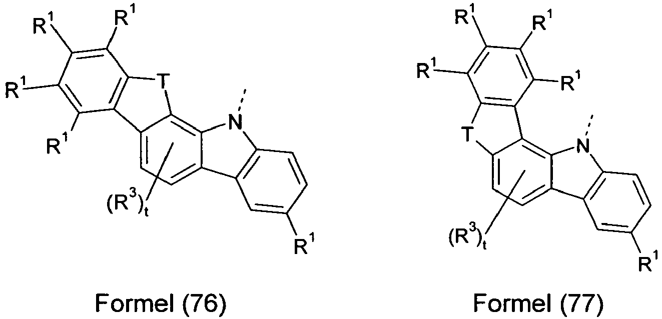

Wobei die verwendeten Symbole und Indizes die oben genannten Bedeutungen aufweisen, insbesondere die oben genannten bevorzugten Bedeutungen.

W kann an Y auch über den Ring C verknüpft sein, dabei ist jede frei substituierbare Position des Rings C als Verknüpfungspunkt geeignet. Bevorzugt sind die hierbei Verbindungen der folgenden Formeln (78) bis (83).

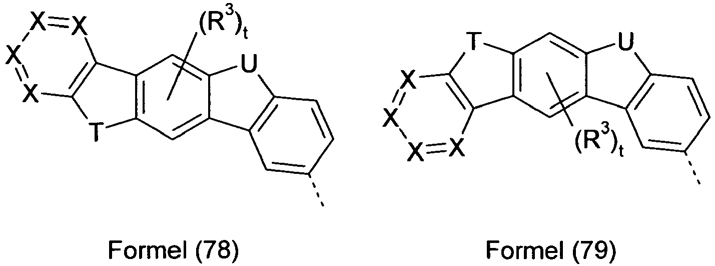

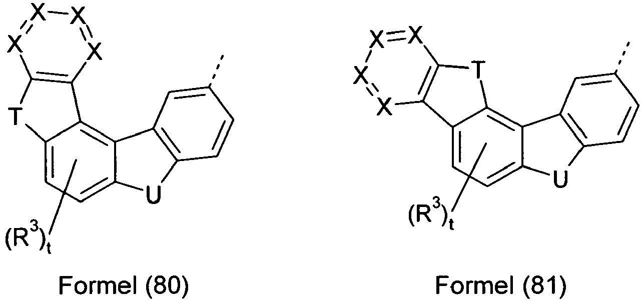

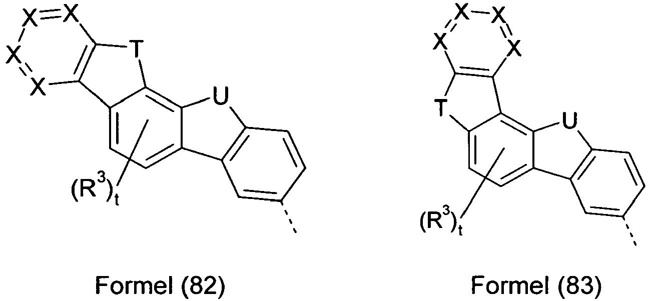

Weiterhin bevorzugte Verbindungen für W sind die Verbindungen der Formeln (84) bis (89).

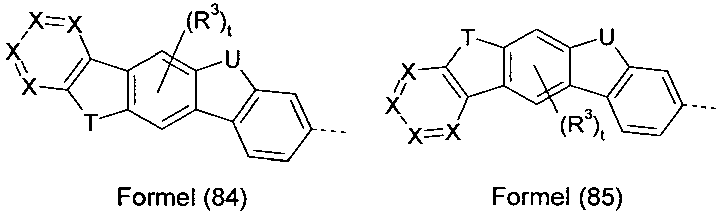

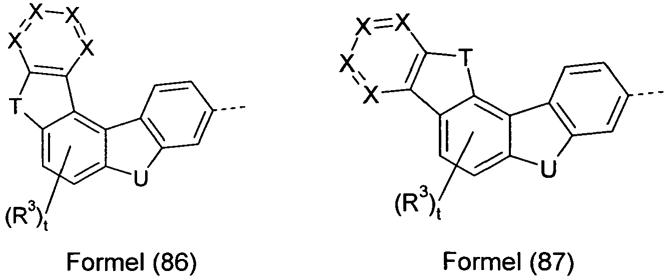

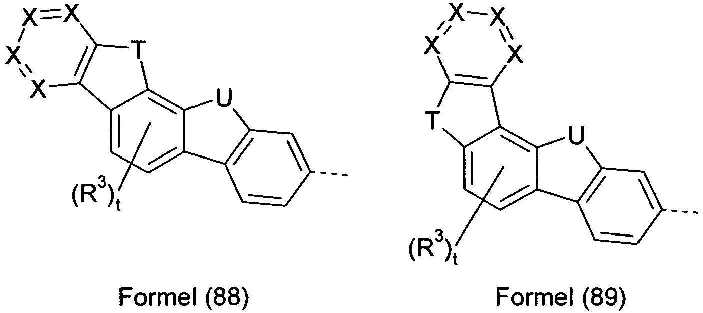

Die Verbindungen der Formeln (84) bis (89) sind insbesondere dann bevorzugt, wenn U gleich C(R¹)₂ ist.

Weiterhin bevorzugt für die Verbindungen der Formel (3) sind die Verbindungen der folgenden Formeln (90) bis (95).

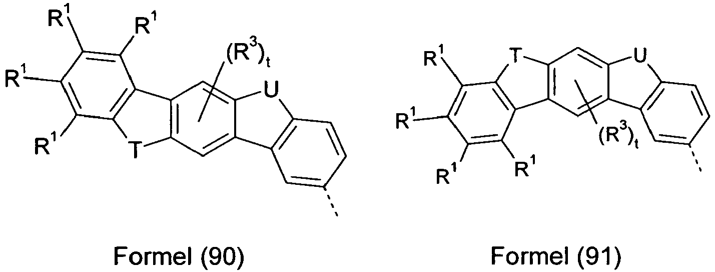

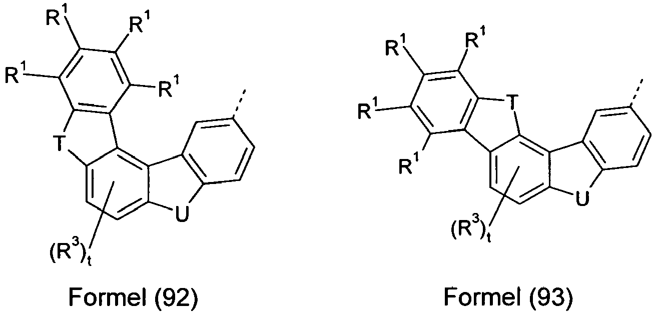

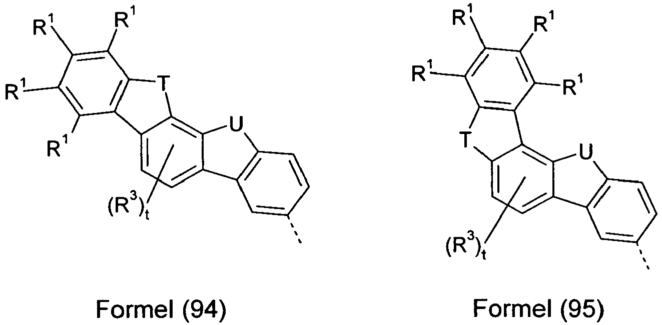

Weiterhin bevorzugte Verbindungen für W sind die Verbindungen der Formeln (96) bis (101).

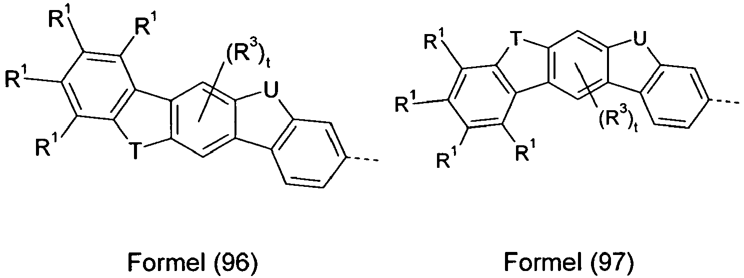

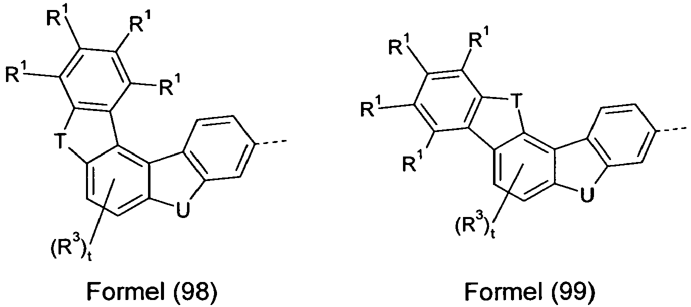

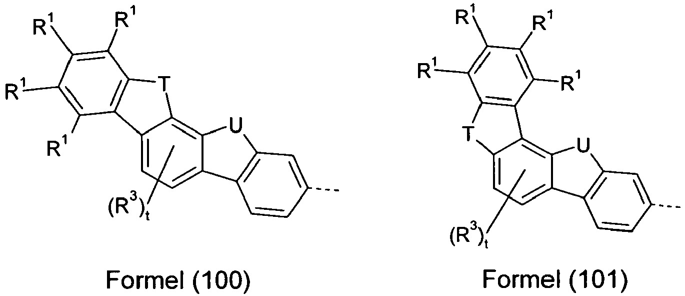

Die Verbindungen der Formeln (96) bis (101) sind insbesondere dann bevorzugt, wenn U gleich C(R¹)₂ ist.

In einer weiteren bevorzugten Ausführungsform der vorliegenden Erfindung gilt für die erfindungsgemäßen Verbindungen t = 0.

An einem der Reste R¹ kann auch eine verbrückende Einheit Z vorhanden sein, die den Liganden L mit einem oder mehreren weiteren Liganden L bzw. L' verknüpft. In einer bevorzugten Ausführungsform der Erfindung ist statt einem der Reste R¹ eine verbrückende Einheit Z vorhanden, so dass die Liganden dreizähnigen oder mehrzähnigen oder polypodalen Charakter aufweisen. Es können auch zwei solcher verbrückenden Einheiten Z vorhanden sein. Dies führt zur Bildung makrocyclischer Liganden bzw. zur Bildung von Kryptaten.

Bevorzugte Strukturen mit mehrzähnigen Liganden bzw. mit polydentaten Liganden sind die Metallkomplexe der folgenden Formeln (102) bis (109), 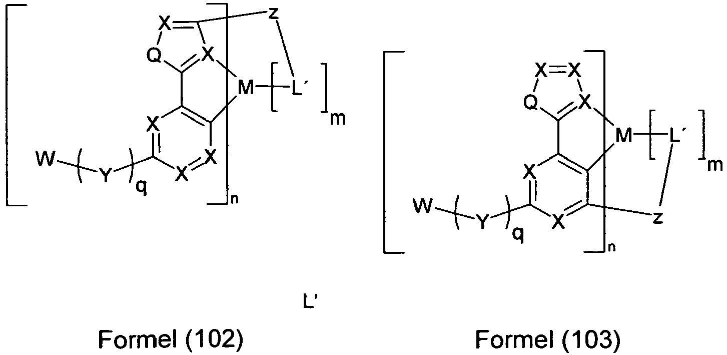 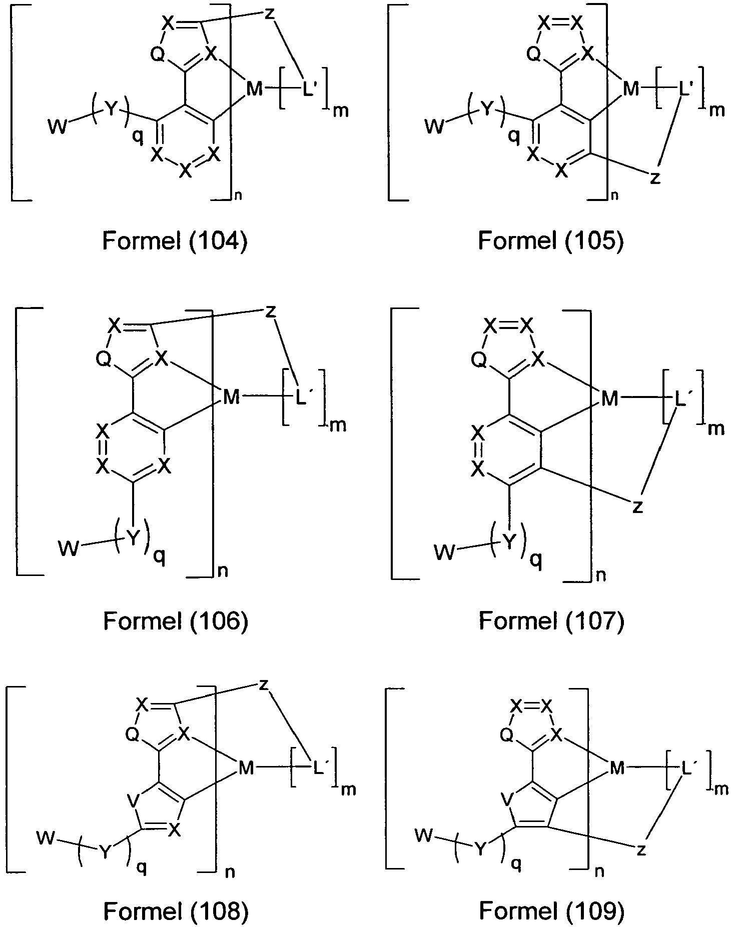 wobei die verwendeten Symbole die oben genannten Bedeutungen aufweisen und Z bevorzugt eine verbrückende Einheit darstellt, enthaltend 1 bis 80 Atome aus der dritten, vierten, fünften und/oder sechsten Hauptgruppe (Gruppe 13, 14, 15 oder 16 gemäß IUPAC) oder einen 3- bis 6-gliedrigen Homo- oder Heterocyclus, die die Teilliganden L miteinander oder L mit L' miteinander kovalent verbindet. Dabei kann die verbrückende Einheit Z auch unsymmetrisch aufgebaut sein, d. h. die Verknüpfung von Z zu L bzw. L' muss nicht identisch sein.

Die verbrückende Einheit Z kann neutral, einfach, zweifach oder dreifach negativ oder einfach, zweifach oder dreifach positiv geladen sein. Bevorzugt ist Z neutral oder einfach negativ oder einfach positiv geladen. Dabei wird die Ladung von Z bevorzugt so gewählt, dass insgesamt ein neutraler Komplex entsteht.

Wenn Z eine trivalente Gruppe ist, also drei Liganden L miteinander bzw. zwei Liganden L mit L' oder einen Liganden L mit zwei Liganden L' verbrückt, ist Z bevorzugt gleich oder verschieden bei jedem Auftreten gewählt aus der Gruppe bestehend aus B, B(R²)-, B(C(R²)₂)₃, (R²)B(C(R²)₂)₃⁻, B(O)₃, (R²)B(O)₃⁻, B(C(R²)₂C(R²)₂)₃, (R²)B(C(R²)₂C(R²)₂)₃⁻, B(C(R²)₂O)₃, (R²)B(C(R²)₂O)₃⁻, B(OC(R²)₂)₃, (R²)B(OC(R²)₂)₃⁻, C(R²), CO⁻, CN(R²)₂, (R²)C(C(R²)₂)₃, (R²)C(O)₃, (R²)C(C(R²)₂C(R²)₂)₃, (R²)C(C(R²)₂O)₃, (R²)C(OC(R²)₂)₃, (R²)C(Si(R²)₂)₃, (R²)C(Si(R²)₂C(R²)₂)₃, (R²)C(C(R²)₂Si(R²)₂)₃, (R²)C(Si(R²)₂Si(R²)₂)₃, Si(R²), (R²)Si(C(R²)₂)₃, (R²)Si(O)₃, (R²)Si(C(R²)₂C(R²)₂)₃, (R²)Si(OC(R²)₂)₃, (R²)Si(C(R²)₂O)₃, (R²)Si(Si(R²)₂)₃, (R²)Si(Si(R²)₂C(R²)₂)₃, (R²)Si(C(R²)₂Si(R²)₂)₃, (R²)Si(Si(R²)₂Si(R²)₂)₃, N, NO, N(R²)⁺, N(C(R²)₂)₃, (R²)N(C(R²)₂)₃⁺, N(C=O)₃, N(C(R²)₂C(R²)₂)₃, (R²)N(C(R²)₂C(R²)₂)⁺, P, P(R²)⁺, PO, PS, PSe, PTe, P(O)₃, PO(O)₃, P(OC(R²)₂)₃, PO(OC(R²)₂)₃, P(C(R²)₂)₃, P(R²)(C(R²)₂)₃⁺, PO(C(R²)₂)₃, P(C(R²)₂C(R²)₂)₃, P(R²)(C(R²)₂C(R²)₂)₃⁺, PO(C(R²)₂C(R²)₂)₃, S⁺, S(C(R²)₂)₃⁺, S(C(R²)₂C(R²)₂)₃⁺, oder eine Einheit gemäß Formel (110), (111), (112) oder (113), 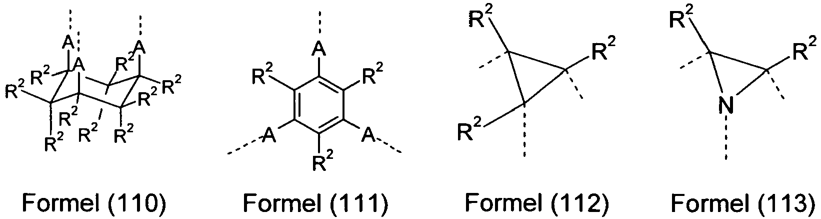 wobei die gestrichelten Bindungen jeweils die Bindung zu den Teilliganden L bzw. L' andeuten und A gleich oder verschieden bei jedem Auftreten ausgewählt ist aus der Gruppe bestehend aus einer Einfachbindung, O, S, S(=O), S(=O)₂, NR², PR², P(=O)R², P(=NR²), C(R²)₂, C(=O), C(=NR²), C(=C(R²)₂), Si(R²)₂ oder BR². Die weiteren verwendeten Symbole haben die oben genannten Bedeutungen.

Wenn Z eine bivalente Gruppe ist, also zwei Liganden L miteinander bzw. einen Liganden L mit L' verbrückt, ist Z bevorzugt gleich oder verschieden bei jedem Auftreten gewählt aus der Gruppe bestehend aus BR², B(R²)₂⁻, C(R²)₂, C(=O), Si(R²)₂, NR², PR², P(R²)₂⁺, P(=O)(R²), P(=S)(R²), AsR², As(=O)(R²), As(=S)(R²), O, S, Se, oder eine Einheit gemäß Formel (114) bis (122), 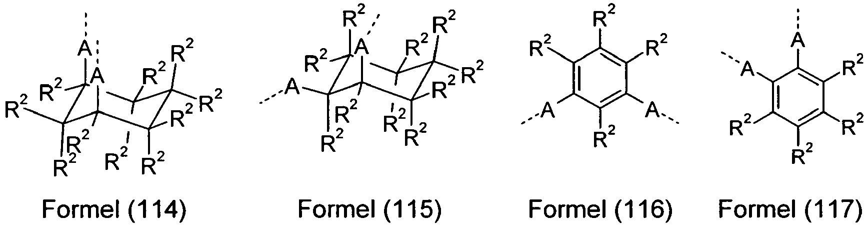 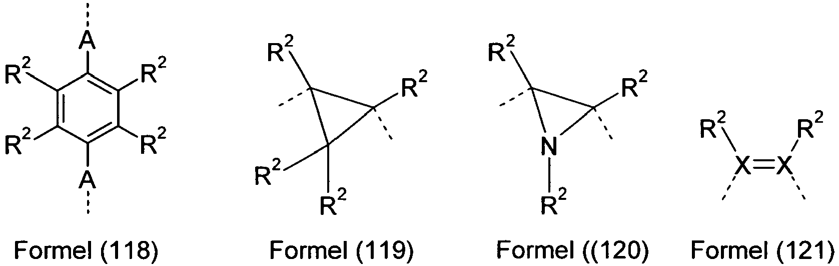 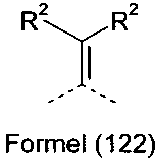 wobei die gestrichelten Bindungen jeweils die Bindung zu den Teilliganden L bzw. L' andeuten und die weiteren verwendeten Symbole jeweils die oben aufgeführten Bedeutungen haben.

Im Folgenden werden bevorzugte Liganden L' beschrieben, wie sie in Formel (1) vorkommen. Entsprechend können auch die Ligandengruppen L' gewählt sein, wenn diese über eine verbrückende Einheit Z an L gebunden sind.

Die Liganden L' sind bevorzugt neutrale, monoanionische, dianionische oder trianionische Liganden, besonders bevorzugt neutrale oder monoanionische Liganden. Sie können monodentat, bidentat, tridentat oder tetradentat sein und sind bevorzugt bidentat, weisen also bevorzugt zwei Koordinationsstellen auf. Wie oben beschrieben, können die Liganden L' auch über eine verbrückende Gruppe Z an L gebunden sein.

Bevorzugte neutrale, monodentate Liganden L' sind ausgewählt aus Kohlenmonoxid, Stickstoffmonoxid, Alkylcyaniden, wie z. B. Acetonitril, Arylcyaniden, wie z. B. Benzonitril, Alkylisocyaniden, wie z. B. Methylisonitril, Arylisocyaniden, wie z. B. Benzoisonitril, Aminen, wie z. B. Trimethylamin, Triethylamin, Morpholin, Phosphinen, insbesondere Halogenphosphine, Trialkylphosphine, Triarylphosphine oder Alkylarylphosphine, wie z. B. Trifluorphosphin, Trimethylphosphin, Tricyclohexylphosphin, Tri-*tert*-butylphosphin, Triphenylphosphin, Tris(pentafluorphenyl)phosphin, Phosphiten, wie z. B. Trimethylphosphit, Triethylphosphit, Arsinen, wie z. B. Trifluorarsin, Trimethylarsin, Tricyclohexylarsin, Tri-*tert-*butylarsin, Triphenylarsin, Tris(pentafluorphenyl)arsin, Stibinen, wie z. B. Trifluorstibin, Trimethylstibin, Tricyclohexylstibin, Tri-*tert*-butylstibin, Triphenylstibin, Tris(pentafluorphenyl)stibin, stickstoffhaltigen Heterocyclen, wie z. B. Pyridin, Pyridazin, Pyrazin, Pyrimidin, Triazin, und Carbenen, insbesondere Arduengo-Carbenen.

Bevorzugte monoanionische, monodentate Liganden L' sind ausgewählt aus Hydrid, Deuterid, den Halogeniden F⁻, Cl⁻, Br⁻ und I⁻, Alkylacetyliden, wie z. B. Methyl-C≡C⁻, tert-Butyl-C≡C⁻, Arylacetyliden, wie z. B. Phenyl-C≡C⁻, Cyanid, Cyanat, Isocyanat, Thiocyanat, Isothiocyanat, aliphatischen oder aromatischen Alkoholaten, wie z. B. Methanolat, Ethanolat, Propanolat, *iso*-Propanolat, *tert*-Butylat, Phenolat, aliphatischen oder aromatischen Thioalkoholaten, wie z. B. Methanthiolat, Ethanthiolat, Propanthiolat, *iso-*Propanthiolat, *tert*-Thiobutylat, Thiophenolat, Amiden, wie z. B. Dimethylamid, Diethylamid, Di-*iso-*propylamid, Morpholid, Carboxylaten, wie z. B. Acetat, Trifluoracetat, Propionat, Benzoat, Arylgruppen, wie z. B. Phenyl, Naphthyl, und anionischen, stickstoffhaltigen Heterocyclen, wie Pyrrolid, Imidazolid, Pyrazolid. Dabei sind die Alkylgruppen in diesen Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte di- bzw. trianionische Liganden sind O²⁻, S²⁻, Carbide, welche zu einer Koordination der Form R-C=M führen, und Nitrene, welche zu einer Koordination der Form R-N=M führen, wobei R allgemein für einen Substituenten steht, oder N³⁻.

Bevorzugte neutrale oder mono- oder dianionische, bidentate oder höherdentate Liganden L' sind ausgewählt aus Diaminen, wie z. B. Ethylendiamin, N,N,N',N'-Tetramethylethylendiamin, Propylendiamin, N,N,N',N'-Tetramethylpropylendiamin, cis- oder trans-Diaminocyclohexan, cis- oder trans-N,N,N',N'-Tetramethyldiaminocyclohexan, Iminen, wie z. B. 2[(1-(Phenylimino)ethyl]pyridin, 2[(1-(2-Methylphenylimino)ethyl]pyridin, 2[(1-(2,6-Di-*iso-*propylphenylimino)ethyl]pyridin, 2[(1-(Methylimino)ethyl]pyridin, 2[(1-(ethylimino)ethyl]pyridin, 2[(1-(*Iso*-Propylimino)ethyl]pyridin, 2[(1-(*Tert-*Butylimino)ethyl]pyridin, Diminen, wie z. B. 1,2-Bis(methylimino)ethan, 1,2-Bis(ethylimino)ethan, 1,2-Bis(*iso*-propylimino)ethan, 1,2-Bis(*tert*-butyl-imino)ethan, 2,3-Bis(methylimino)butan, 2,3-Bis(ethylimino)butan, 2,3-Bis-(iso-propylimino)butan, 2,3-Bis(*tert*-butylimino)butan, 1,2-Bis(phenylimino)-ethan, 1,2-Bis(2-methylphenylimino)ethan, 1,2-Bis(2,6-di-iso-propylphenyl-imino)ethan, 1,2-Bis(2,6-di-*tert*-butylphenylimino)ethan, 2,3-Bis(phenyl-imino)butan, 2,3-Bis(2-methylphenylimino)butan, 2,3-Bis(2,6-di-*iso*-propyl-phenylimino)butan, 2,3-Bis(2,6-di-*tert*-butylphenylimino)butan, Heterocyclen enthaltend zwei Stickstoffatome, wie z. B. 2,2'-Bipyridin, o-Phenanthrolin, Diphosphinen, wie z. B. Bis-diphenylphosphinomethan, Bis-diphenylphosphinoethan, Bis(diphenylphosphino)propan, Bis(diphenyl-phosphino)butan, Bis(dimethylphosphino)methan, Bis(dimethylphosphino)-ethan, Bis(dimethylphosphino)propan, Bis(diethylphosphino)methan, Bis-(diethylphosphino)ethan, Bis(diethylphosphino)propan, Bis(di-*tert-*butyl-phosphino)methan, Bis(di-*tert*-butylphosphino)ethan, Bis(*tert*-butyl-phosphino)propan, 1,3-Diketonaten abgeleitet von 1,3-Diketonen, wie z. B. Acetylaceton, Benzoylaceton, 1,5-Diphenylacetylaceton, Dibenzoylmethan, Bis(1,1,1-trifluoracetyl)methan, 3-Ketonaten abgeleitet von 3-Ketoestern, wie z. B. Acetessigsäureethylester, Carboxylate, abgeleitet von Aminocarbonsäuren, wie z. B. Pyridin-2-carbonsäure, Chinolin-2-carbonsäure, Glycin, N,N-Dimethylglycin, Alanin, N,N-Dimethylaminoalanin, Salicyliminaten abgeleitet von Salicyliminen, wie z. B. Methylsalicylimin, Ethylsalicylimin, Phenylsalicylimin, Dialkoholaten abgeleitet von Dialkoholen, wie z. B. Ethylenglykol, 1,3-Propylenglykol und Dithiolaten abgeleitet von Dithiolen, wie z. B. 1,2-Ethylendithiol, 1,3-Propylendithiol.

Bevorzugte tridentate Liganden sind Borate stickstoffhaltiger Heterocyclen, wie z. B. Tetrakis(1-imidazolyl)borat und Tetrakis(1-pyrazolyl)borat.

Besonders bevorzugt sind weiterhin bidentate monoanionische Liganden L', welche mit dem Metall einen cyclometallierten Fünfring oder Sechsring mit mindestens einer Metall-Kohlenstoff-Bindung aufweisen, insbesondere einen cyclometallierten Fünfring. Dies sind insbesondere Liganden, wie sie allgemein im Gebiet der phosphoreszierenden Metallkomplexe für organische Elektrolumineszenzvorrichtungen verwendet werden, also Liganden vom Typ Phenylpyridin, Naphthylpyridin, Phenylchinolin, Phenylisochinolin, etc., welche jeweils durch einen oder mehrere Reste R¹ bis R⁷ substituiert sein können. Dem Fachmann auf dem Gebiet der phosphoreszierenden Elektrolumineszenzvorrichtungen ist eine Vielzahl derartiger Liganden bekannt, und er kann ohne erfinderisches Zutun weitere derartige Liganden als Ligand L' für Verbindungen gemäß Formel (1) auswählen. Generell eignet sich dafür besonders die Kombination aus zwei Gruppen, wie sie durch die folgenden Formeln (123) bis (150) dargestellt sind, wobei eine Gruppe über ein neutrales Stickstoffatom oder ein Carbenatom bindet und die andere Gruppe über ein negativ geladenes Kohlenstoffatom oder ein negativ geladenes Stickstoffatom bindet. Der Ligand L' kann dann aus den Gruppen der Formeln (123) bis (150) gebildet werden, indem diese Gruppen jeweils an der durch # gekennzeichneten Position aneinander binden. Die Position, an der die Gruppen an das Metall koordinieren, sind durch * gekennzeichnet. Diese Gruppen können auch über eine oder zwei verbrückende Einheiten Z an den Liganden L gebunden sein.

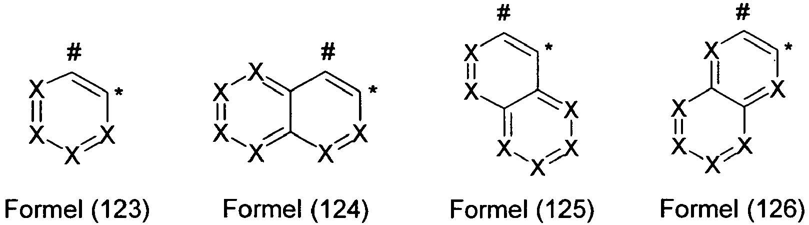

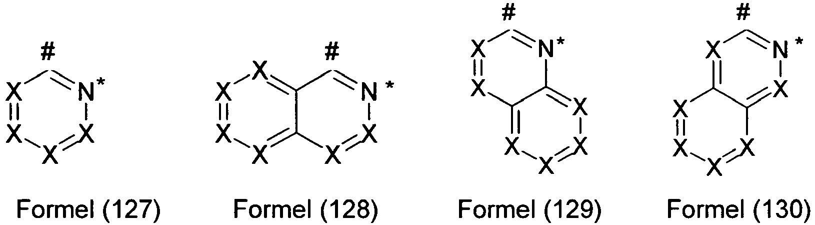

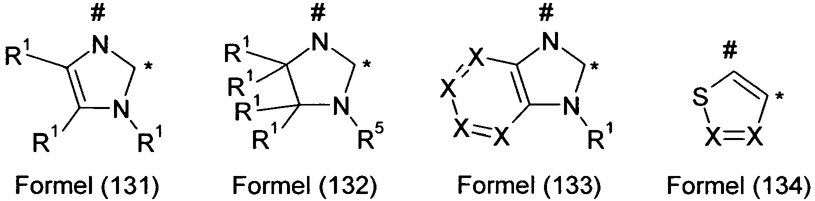

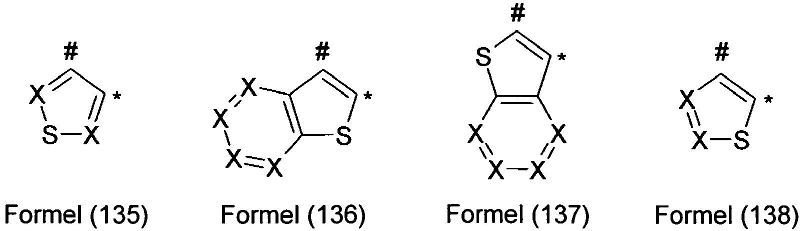

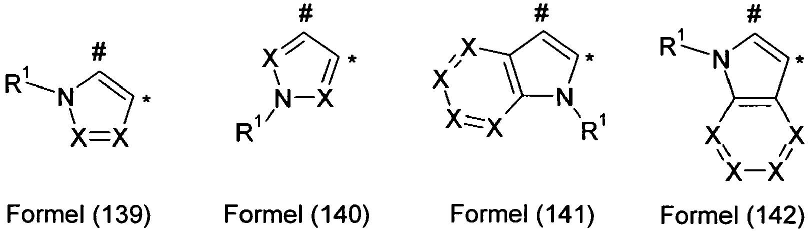

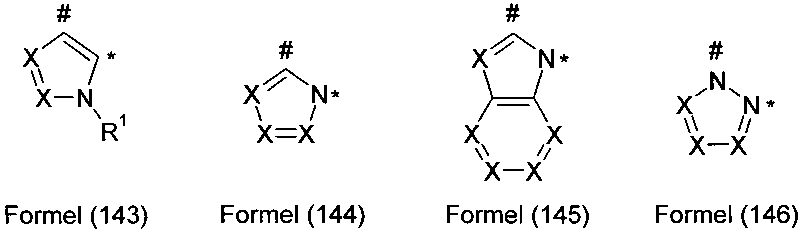

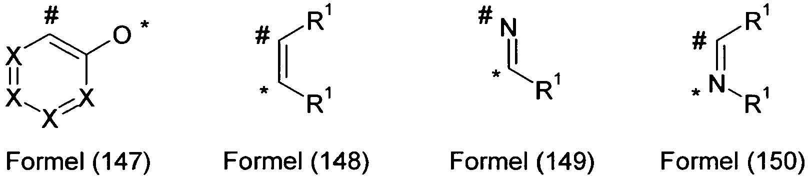

Dabei haben die verwendeten Symbole dieselbe Bedeutung wie oben beschrieben, und bevorzugt stehen maximal drei Symbole X in jeder Gruppe für N, besonders bevorzugt stehen maximal zwei Symbole X in jeder Gruppe für N, ganz besonders bevorzugt steht maximal ein Symbol X in jeder Gruppe für N. Insbesondere bevorzugt stehen alle Symbole X gleich oder verschieden bei jedem Auftreten für CR¹.

In einer besonders bevorzugten Ausführungsform der vorliegenden Erfindung werden zwei Fragmente eines Liganden L' der Formel (123) bis (150) derart über die Position # miteinander kombiniert, dass wenigstens eines der Fragmente ein Heteroatom an der Position * aufweist.

In einer ganz besonders bevorzugten Ausführungsform der vorliegenden Erfindung setzt sich der Ligand L' aus genau einem Fragment ohne Heteroatom aus der Liste der Formel (123) bis (150) und genau einem Fragment mit Heteroatom, bevorzugte einem Stickstoffatom, aus der Liste der Fragmente mit den Formeln (123) bis (150) zusammen.

Ebenfalls bevorzugte Liganden L' sind η⁵-Cyclopentadienyl, η⁵-Penta-methylcyclopentadienyl, η⁶-Benzol oder η⁷-Cycloheptatrienyl, welche jeweils durch einen oder mehrere Reste R¹ substituiert sein können. Ebenfalls bevorzugte Liganden L' sind 1,3,5-cis-Cyclohexanderivate, insbesondere der Formel (150), 1,1,1-Tri(methylen)methanderivate, insbesondere der Formel (152) und 1,1,1-trisubstituierte Methane, insbesondere der Formel (153) und (154), 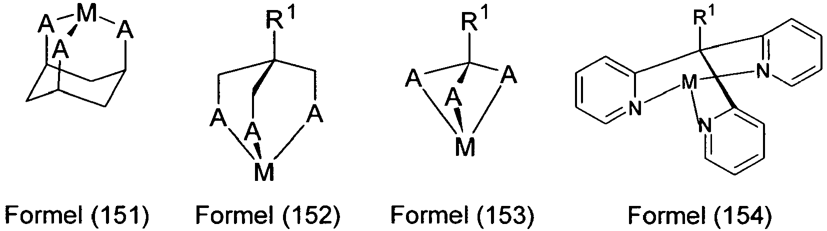 wobei in den Formeln jeweils die Koordination an das Metall M dargestellt ist, R¹ die oben genannte Bedeutung hat und A, gleich oder verschieden bei jedem Auftreten, für O⁻, S⁻, COO⁻, P(R¹)₂ oder N(R¹)₂ steht.

Bevorzugte Reste R¹ in den oben aufgeführten Strukturen sind bei jedem Auftreten gleich oder verschieden ausgewählt aus der Gruppe bestehend aus H, D, F, Br, N(R²)₂, CN, B(OR²)₂, C(=O)R², P(=O)(R²)₂, einer geradkettige Alkylgruppe mit 1 bis 10 C-Atomen oder einer geradkettige Alkenyl- oder Alkinylgruppe mit 2 bis 10 C-Atomen oder einer verzweigten oder cyclischen Alkyl-, Alkenyl- oder Alkinylgruppe mit 3 bis 10 C-Atomen, die jeweils mit einem oder mehreren Resten R² substituiert sein kann, wobei ein oder mehrere H-Atome durch F ersetzt sein können, oder ein aromatisches oder heteroaromatisches Ringsystem mit 5 bis 14 aromatischen Ringatomen, das jeweils durch einen oder mehrere Reste R² substituiert sein kann; dabei können mehrere Reste R¹ auch miteinander ein mono- oder polycyclisches, aliphatisches, aromatisches und/oder benzoannelliertes Ringsystem bilden. Besonders bevorzugte Reste R¹ sind bei jedem Auftreten gleich oder verschieden ausgewählt aus der Gruppe bestehend aus H, F, Br, CN, B(OR²)₂, einer geradkettigen Alkylgruppe mit 1 bis 6 C-Atomen, insbesondere Methyl, oder einer verzweigten oder cyclischen Alkylgruppe mit 3 bis 10 C-Atomen, insbesondere iso-Propyl oder tert-Butyl, wobei ein oder mehrere H-Atome durch F ersetzt sein können, oder ein aromatisches oder heteroaromatisches Ringsystem mit 5 bis 12 aromatischen Ringatomen, das jeweils durch einen oder mehrere Reste R² substituiert sein kann; dabei können mehrere Reste R¹ auch miteinander ein mono- oder polycyclisches, aliphatisches, aromatisches und/oder benzoannelliertes Ringsystem bilden.

Die erfindungsgemäßen Metallkomplexe sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Metallkomplex-Verbindungen gemäß Formel (1) durch Umsetzung der entsprechenden freien Liganden mit Metallalkoholaten der Formel (155), mit Metallketoketonaten der Formel (156) oder mit Metallhalogeniden der Formel (157),

M(OR¹)ₙ Formel (155)

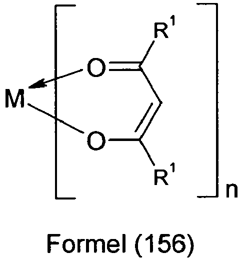

MHalₙ Formel (157)

wobei die Symbole M, n und R¹ die oben angegebenen Bedeutungen haben und Hal = F, Cl, Br oder I ist.

Es können ebenfalls Metallverbindungen, insbesondere Iridiumverbin-dungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 04/085449 offenbart. Besonders geeignet ist [IrCl₂(acac)₂]⁻, beispielsweise Na[IrCl₂(acac)₂].

Die Synthese der Komplexe wird bevorzugt durchgeführt wie in WO 02/060910 und in WO 04/085449 beschrieben. Heteroleptische Komplexe können beispielsweise auch gemäß WO 05/042548 synthetisiert werden. Dabei kann die Synthese beispielsweise auch thermisch, photochemisch und/oder durch Mikrowellenstrahlung aktiviert werden.

Durch diese Verfahren lassen sich die erfindungsgemäßen Verbindungen gemäß Formel (1) in hoher Reinheit, bevorzugt mehr als 99 % (bestimmt mittels ¹H-NMR und/oder HPLC) erhalten.

Mit den hier erläuterten Synthesemethoden lassen sich unter anderem die im Folgenden dargestellten erfindungsgemäßen Verbindungen der Formeln (158) bis (335) herstellen.

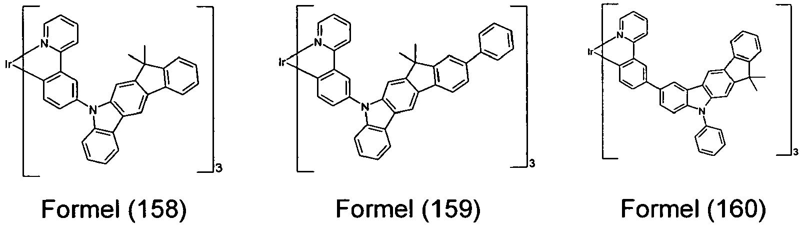

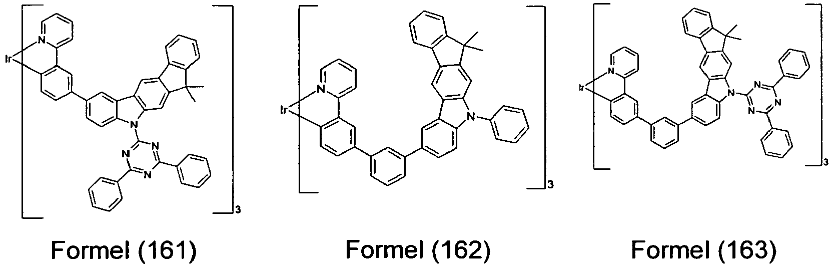

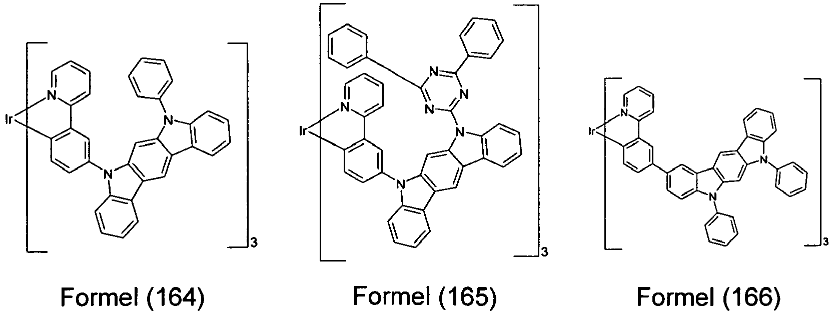

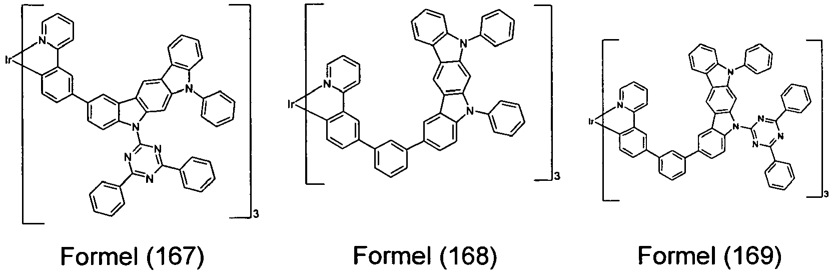

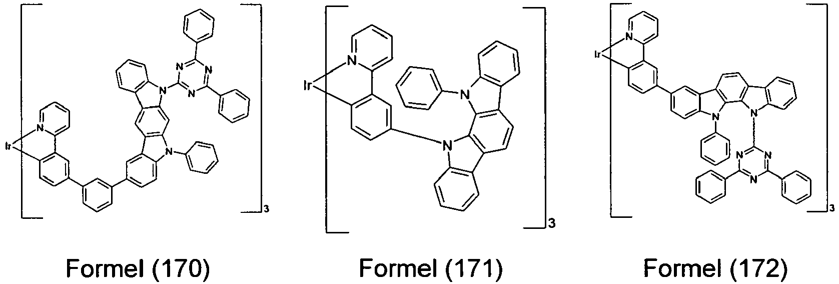

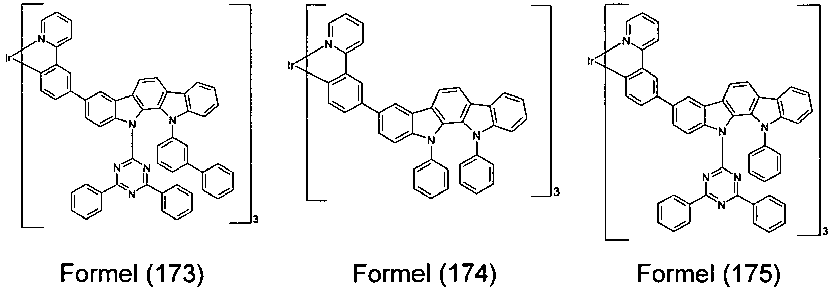

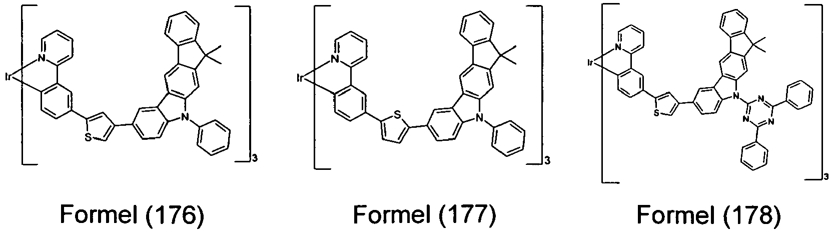

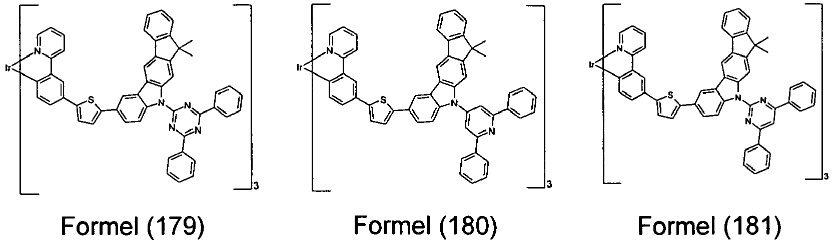

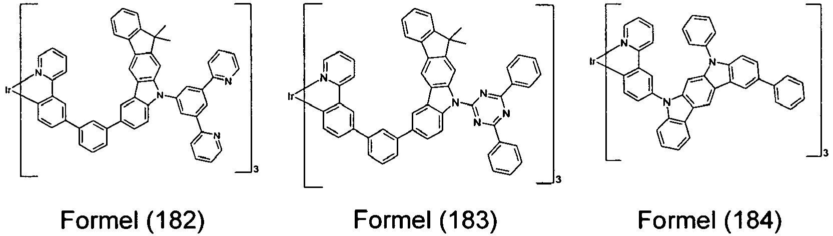

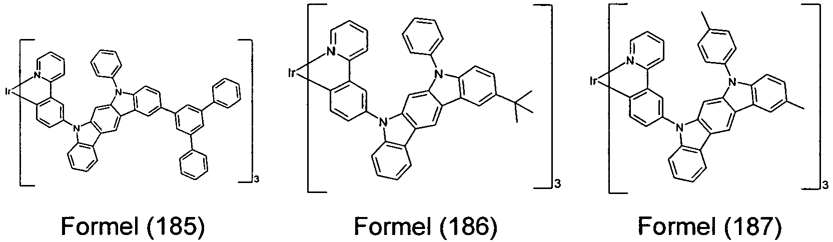

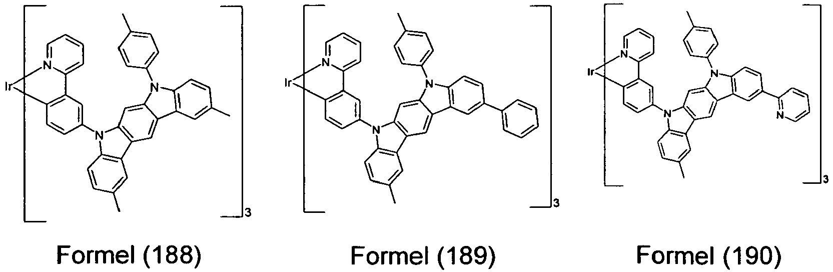

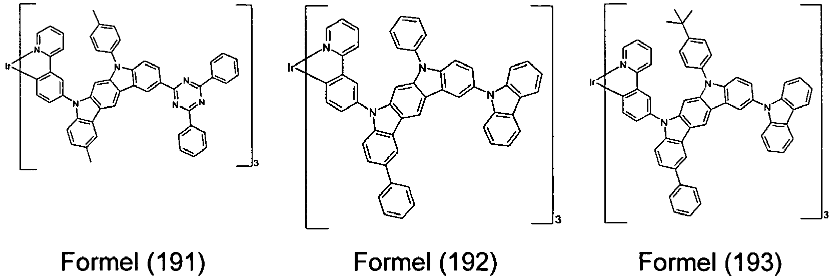

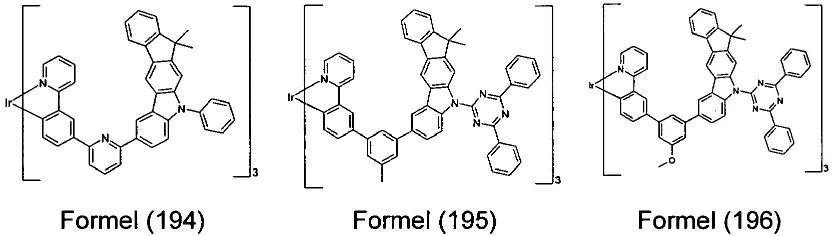

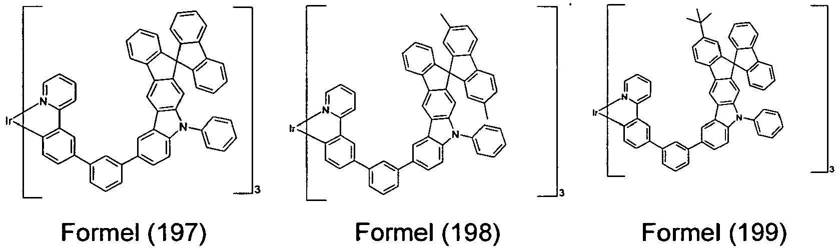

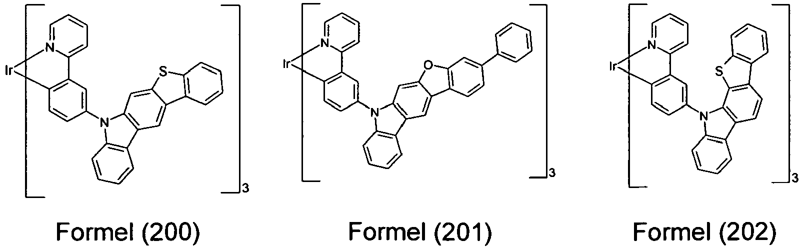

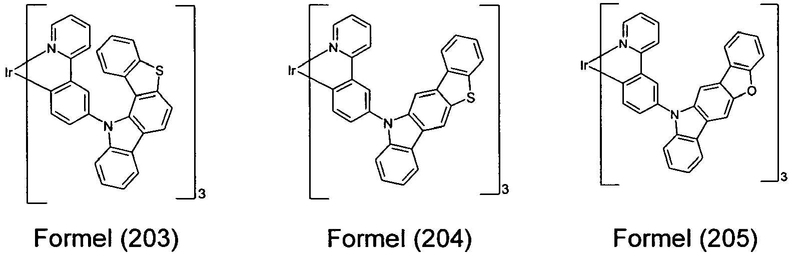

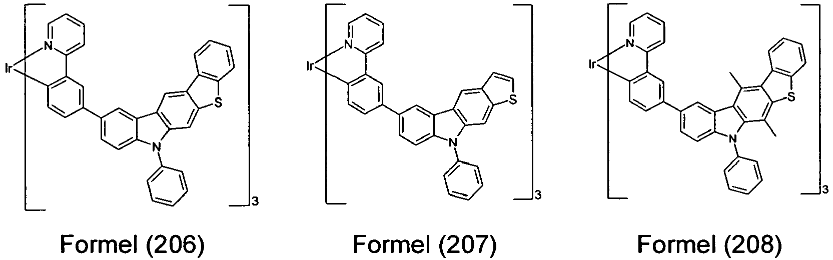

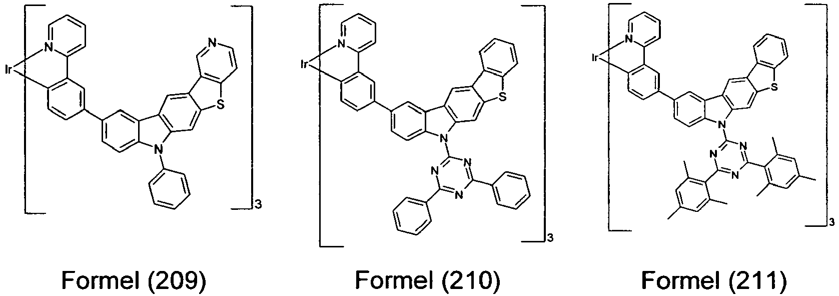

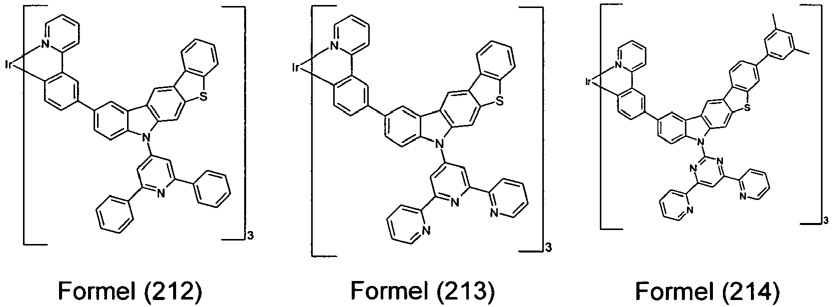

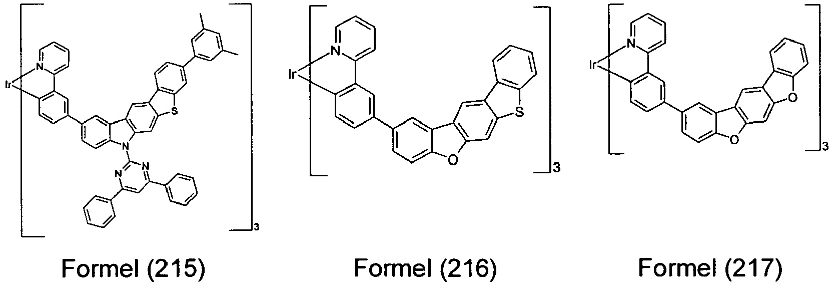

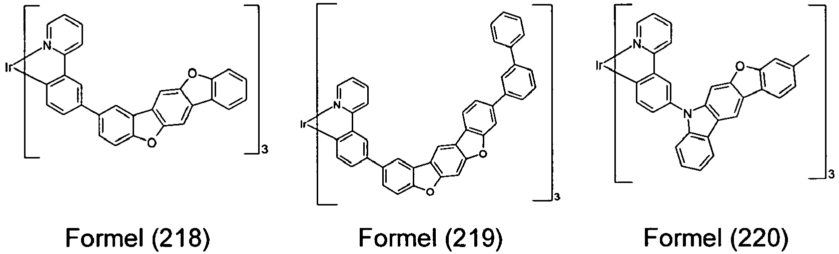

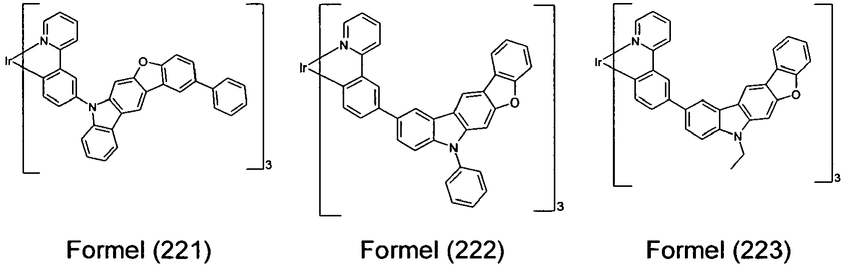

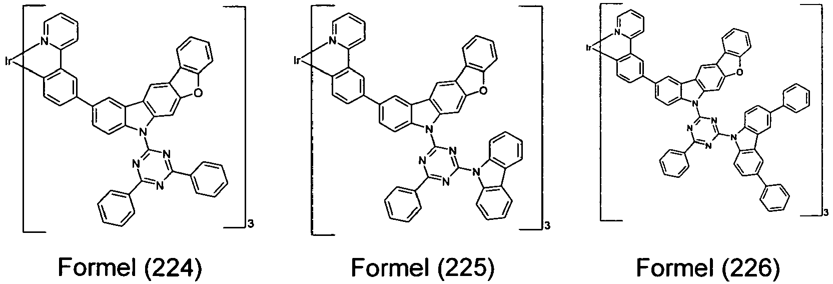

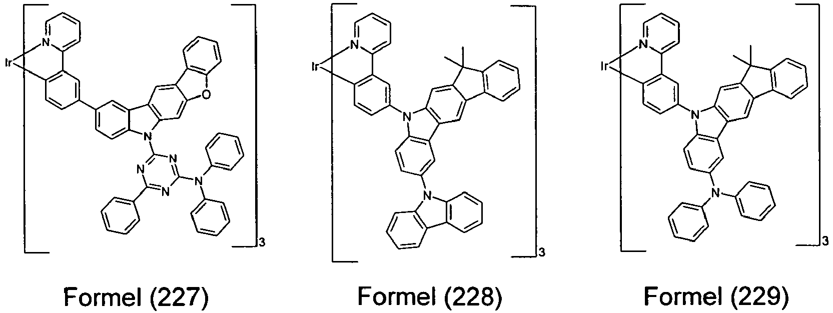

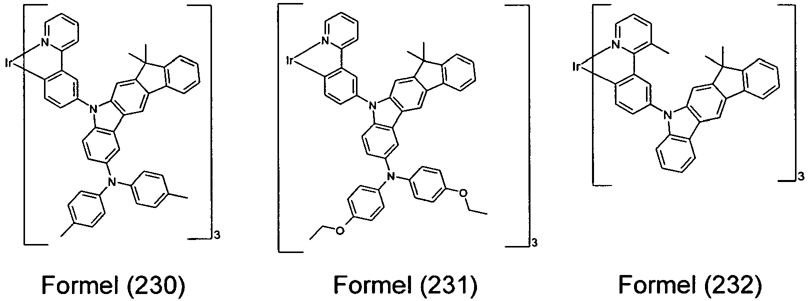

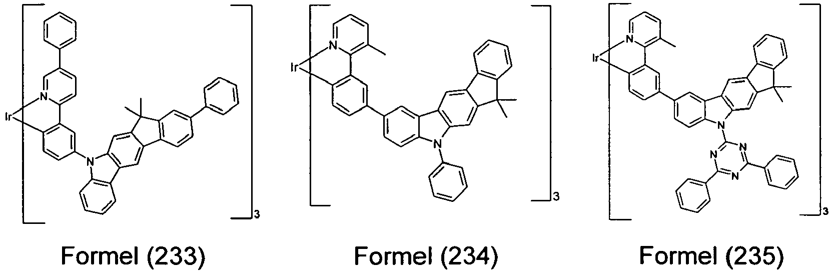

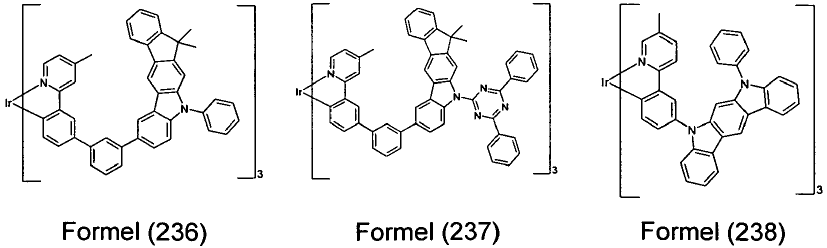

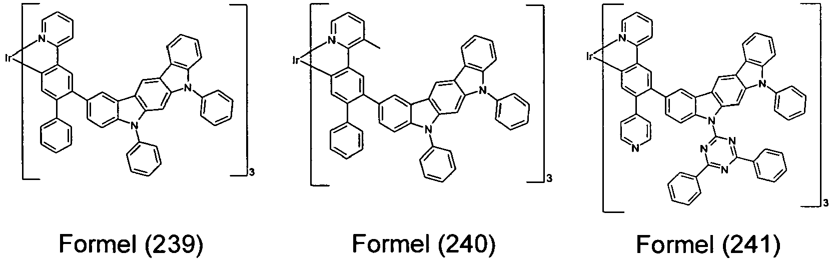

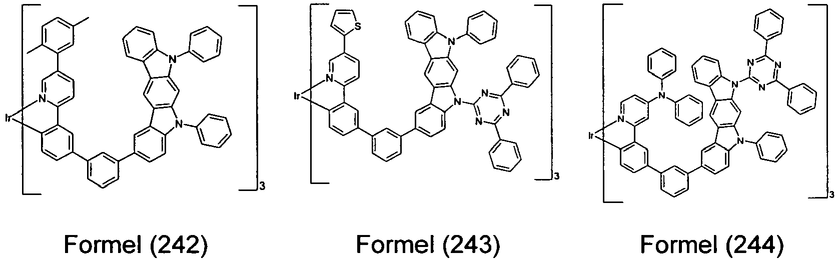

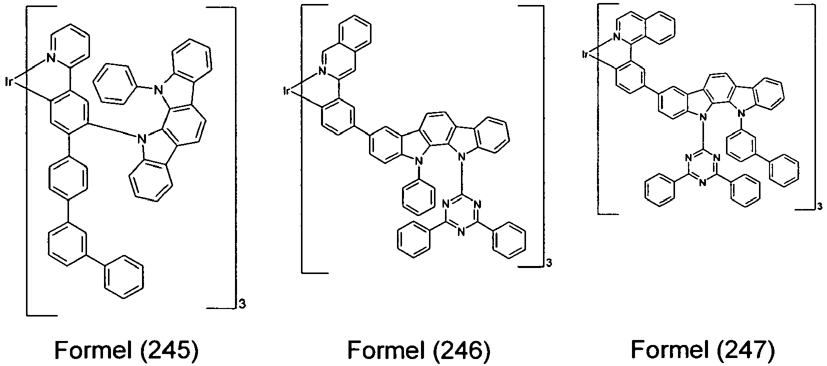

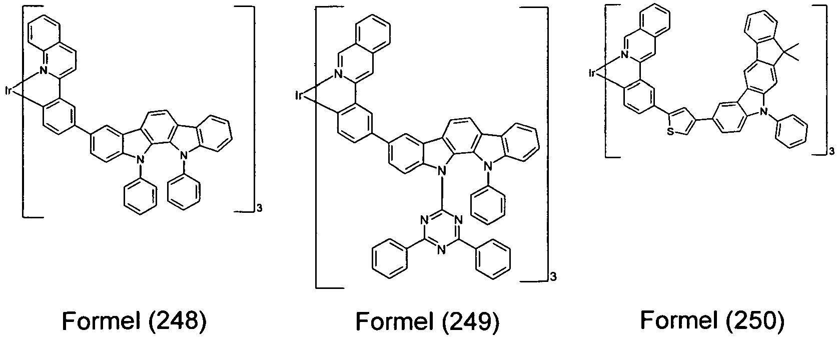

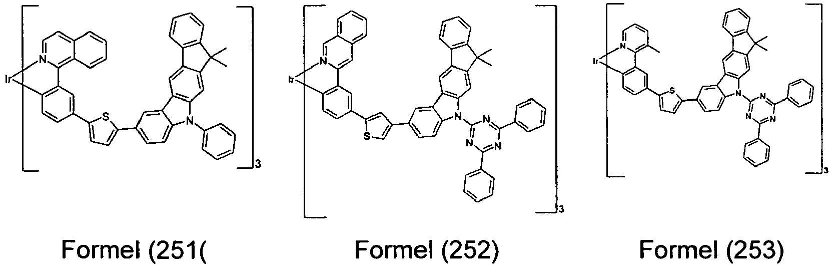

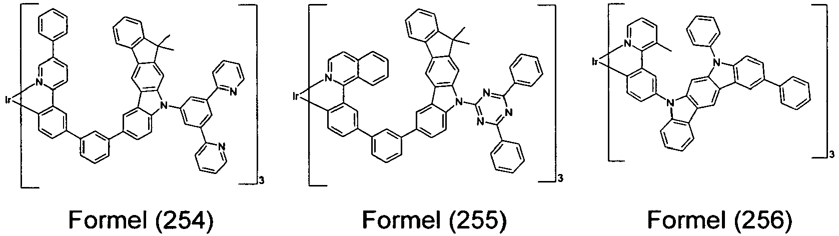

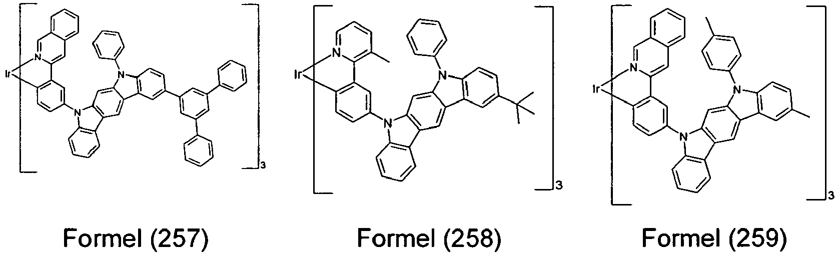

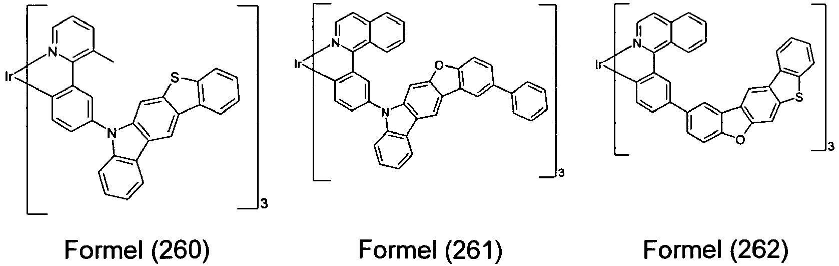

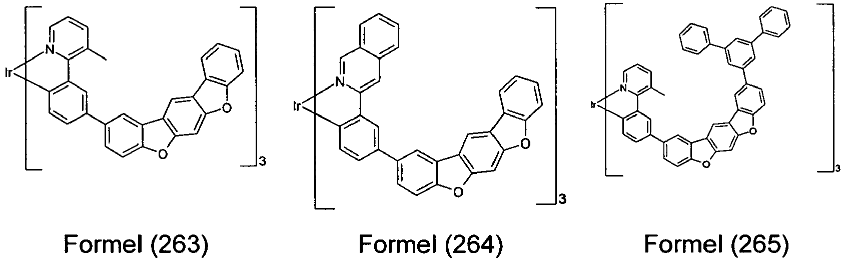

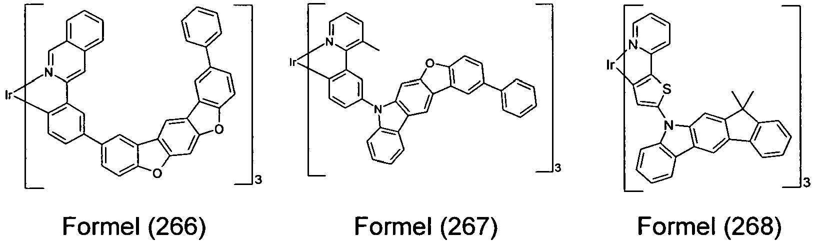

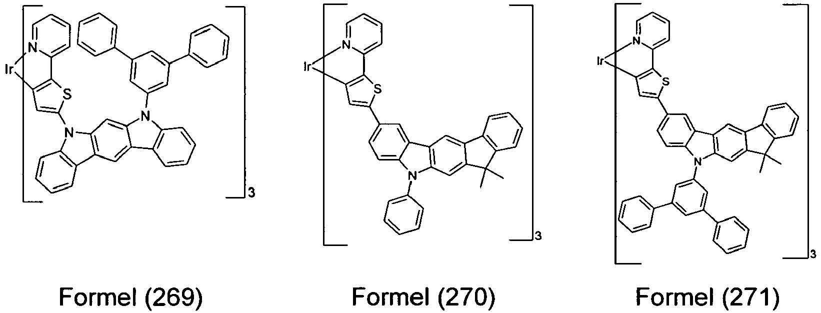

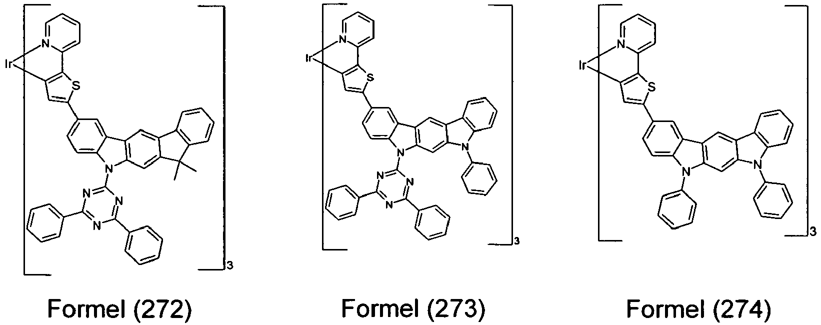

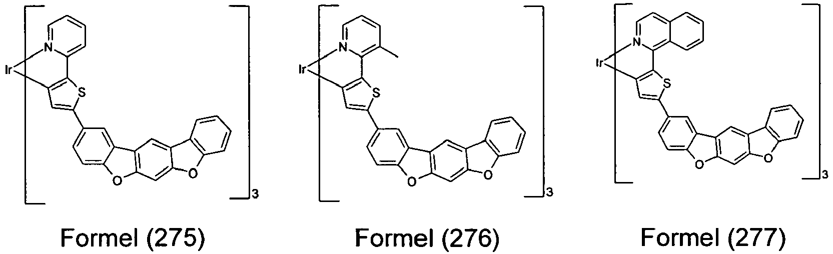

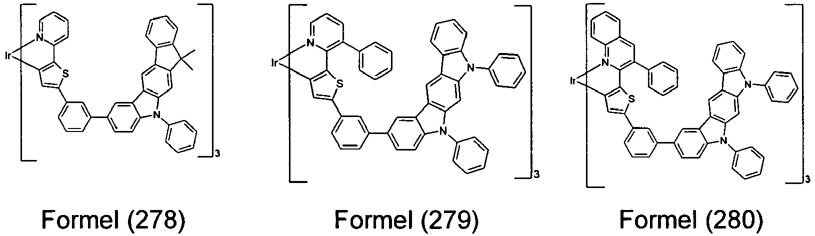

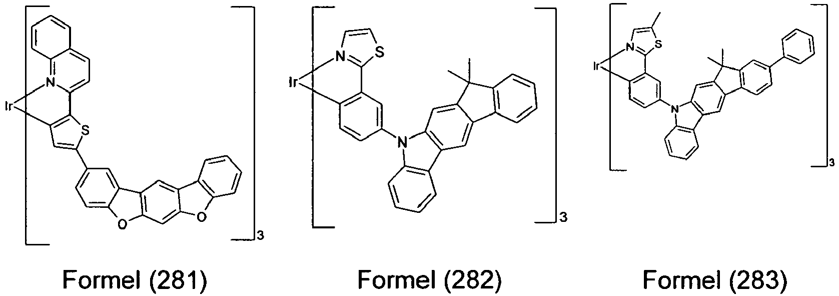

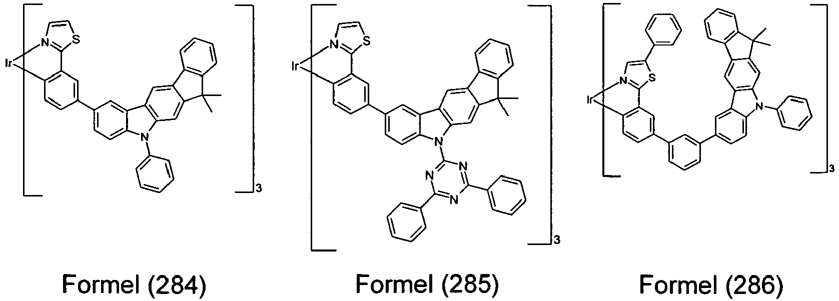

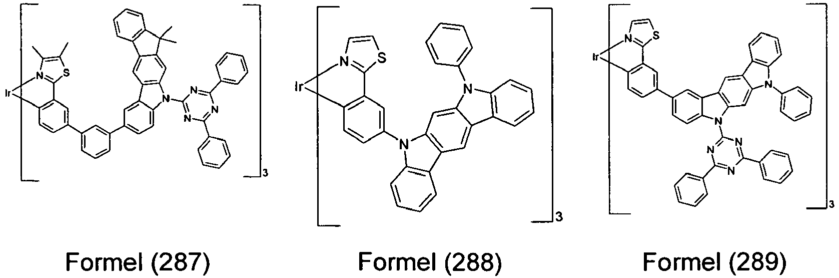

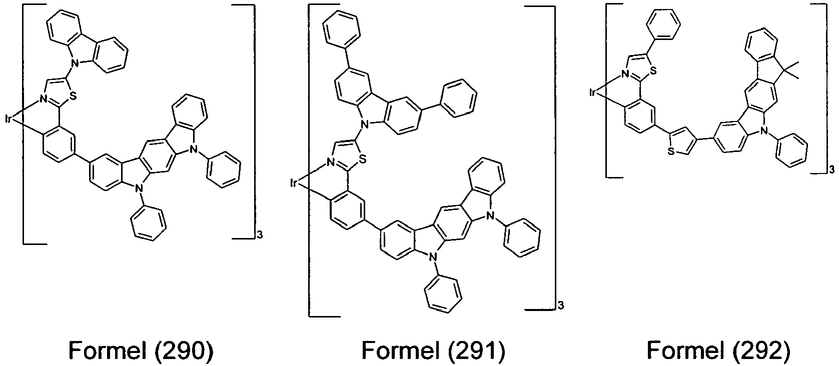

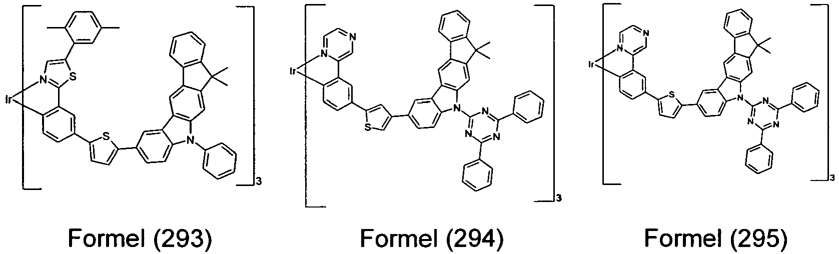

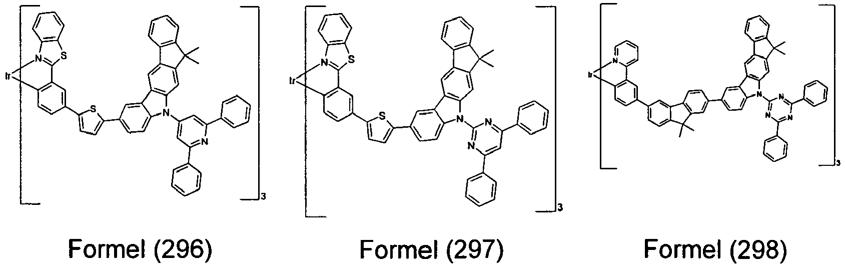

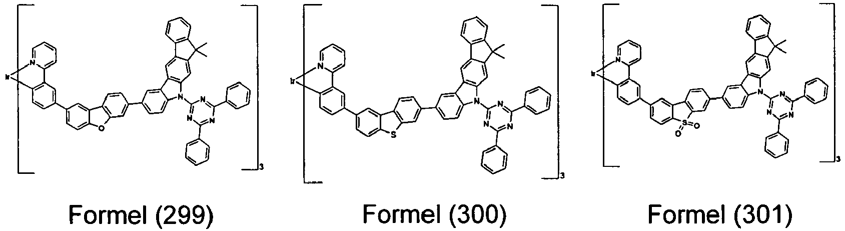

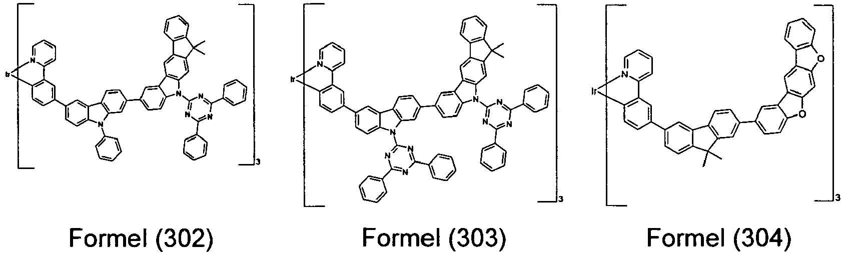

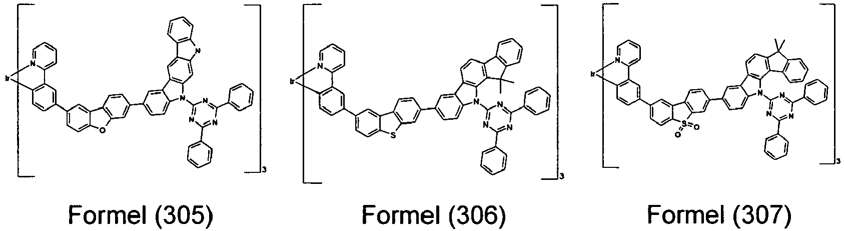

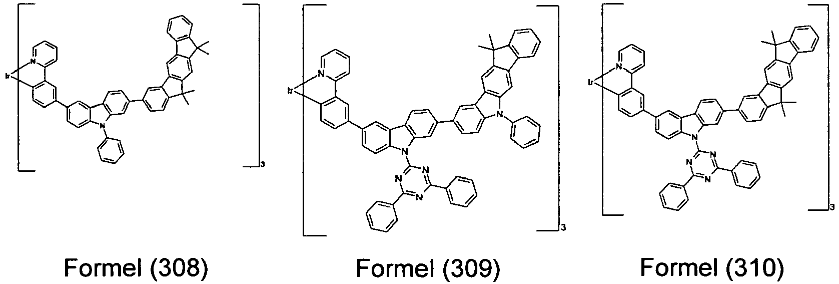

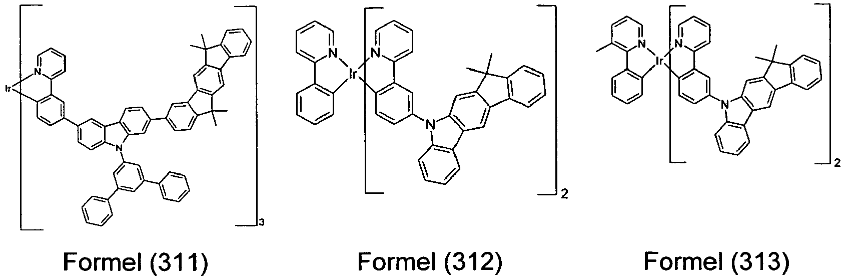

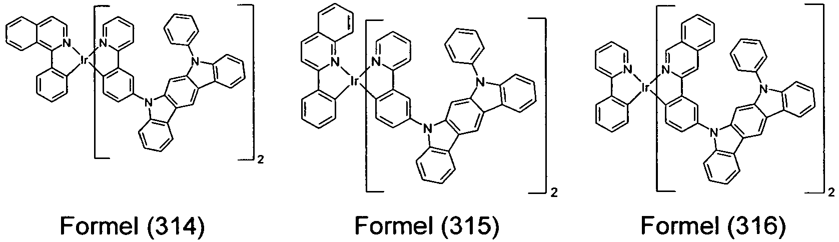

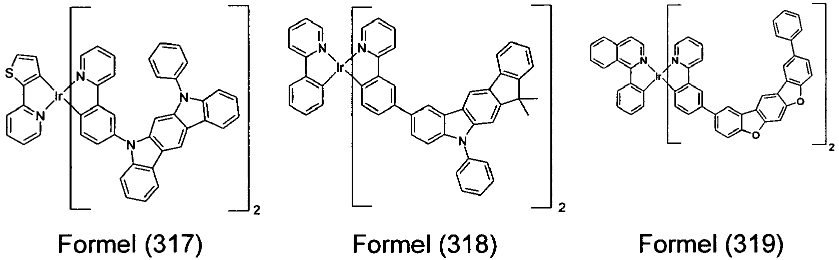

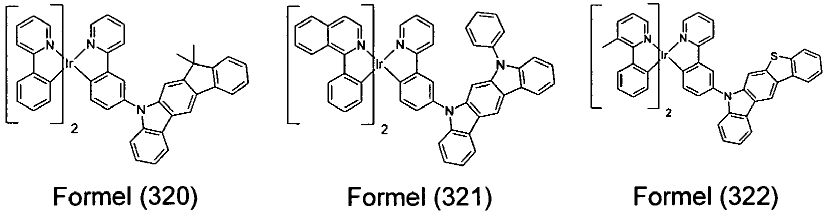

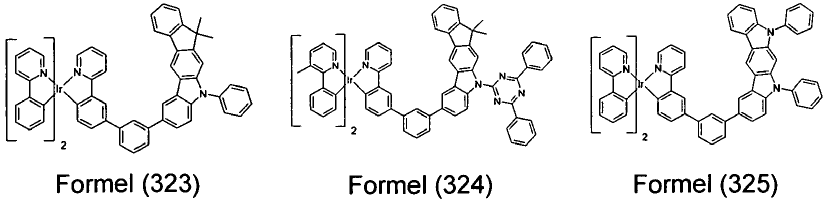

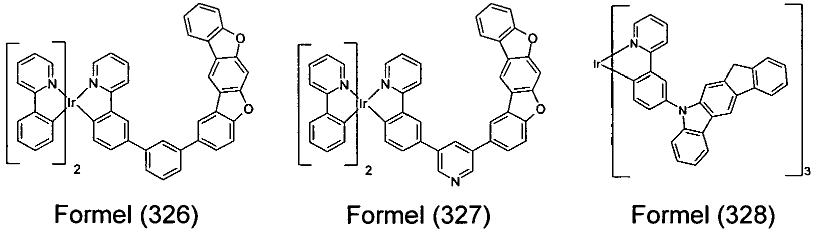

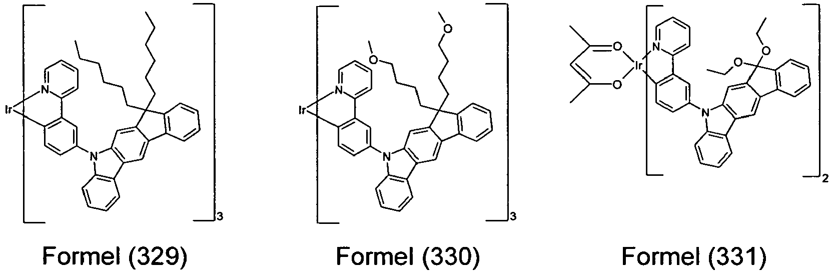

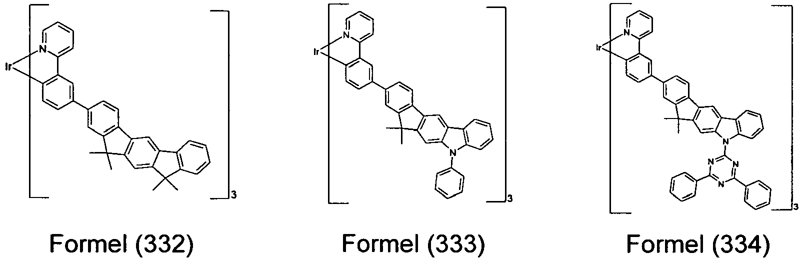

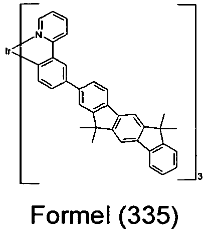

Die oben beschriebenen Komplexe gemäß Formel (1) bzw. die oben aufgeführten bevorzugten Ausführungsformen können in der elektronischen Vorrichtung als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung der oben aufgeführten Formel (1) enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine Verbindung gemäß der oben aufgeführten Formel (1). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Excitonenblockierschichten, Ladungserzeugungsschichten und/oder organische oder anorganische p/n-Übergänge. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 05/011013) bzw. Systeme, welche mehr als drei emittierende Schichten aufweisen.

In einer bevorzugten Ausführungsform der Erfindung enthält die organische Elektrolumineszenzvorrichtung die Verbindung gemäß Formel (1) bzw. die oben aufgeführten bevorzugten Ausführungsformen als emittierende Verbindung in einer oder mehreren emittierenden Schichten.

Wenn die Verbindung gemäß Formel (1) als emittierende Verbindung in einer emittierenden Schicht eingesetzt wird, wird sie bevorzugt in Kombination mit einem oder mehreren Matrixmaterialien eingesetzt. Die Mischung aus der Verbindung gemäß Formel (1) und dem Matrixmaterial enthält zwischen 1 und 99 Gew.-%, vorzugsweise zwischen 2 und 40 Gew.-%, besonders bevorzugt zwischen 3 und 30 Gew.-%, insbesondere zwischen 5 und 25 Gew.-% der Verbindung gemäß Formel (1) bezogen auf die Gesamtmischung aus Emitter und Matrixmaterial. Entsprechend enthält die Mischung zwischen 99 und 1 Gew.-%, vorzugsweise zwischen 98 und 60 Gew.-%, besonders bevorzugt zwischen 97 und 70 Gew.-%, insbesondere zwischen 95 und 75 Gew.-% des Matrixmaterials bezogen auf die Gesamtmischung aus Emitter und Matrixmaterial.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder der nicht offen gelegten Anmeldung DE 102008033943.1, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß den nicht offen gelegten Anmeldungen DE 102009023155.2 und DE 102009031021.5, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß der nicht offen gelegten Anmeldung DE 102008036982.9, WO 07/063754 oder WO 08/056746, Zinkkomplexe, z. B. gemäß EP 652273 oder gemäß WO 09/062578, Diaza- oder Tetraazasilolderivate, z. B. gemäß der nicht offen gelegten Anmeldung DE 102008056688.8, oder Diazaphospholderivate, z. B. gemäß der nicht offen gelegten Anmeldung DE 102009022858.6.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons oder eines Triazins mit einem Triarylamin-Derivat oder einem Carbazol-Derivat als gemischte Matrix für den erfindungsgemäßen Metallkomplex. Ebenfalls bevorzugt sind auch Mischungen aus einen loch- oder elektronentransportierenden Material mit einem Material, welches weder am Loch- noch am Elektronentransport beteiligt ist, wie beispielsweise in DE 102009014513 offenbart.

In einer weiteren bevorzugten Ausführungsform der vorliegenden Erfindung können die erfindungsgemäßen Verbindungen in Mischungen mit einem oder mehreren weiteren Emittern eingesetzt werden. Ganz besonders bevorzugt ist dabei eine Mischung der erfindungsgemäßen Verbindungen mit einem oder mehreren fluoreszierenden Emittern. Weiterhin bevorzugt ist eine Mischung mit einem oder mehreren phosphoreszierenden Emittern. Dabei emittieren fluoreszierende Emitter hauptsächlich aus angeregten Singulett-Zuständen, wohingegen phosphoreszierende Emitter hauptsächlich aus höheren Spinzuständen (z.B. Triplett und Quintett) Licht emittieren. Die Komplexe organischer Übergangsmetalle werden im Sinne dieser Erfindung als phosphoreszierende Emitter verstanden. Vorzugsweise sind die weiteren Emitter organische Verbindungen.

In einer besonders bevorzugten Ausführungsform der vorliegenden Erfindung werden die erfindungsgemäßen Verbindungen mit 3 weiteren, in einer insbesondere bevorzugten Ausführungsform mit 2 weiteren und in einer ganz besonders bevorzugten Ausführungsform mit einem weiteren Emitter gemischt.

In einer weiteren bevorzugten Ausführungsform der vorliegenden Erfindung enthalten die Emitter-Mischungen 3, insbesondere bevorzugt 2 und ganz besonders bevorzugt eine erfindungsgemäße Verbindung.

In einer besonders bevorzugten Ausführungsform der vorliegenden Erfindung enthalten die Emitter-Mischungen genau eine der erfindungsgemäßen Verbindung und genau einen weiteren Emitter.

Es ist weiterhin bevorzugt im Sinne der vorliegenden Erfindung, dass die Absorptionsspektren wenigstens eines Emitters und das Emissionsspektrum wenigstens eines anderen Emitters der Mischung überlappen, so dass ein Energietransfer (Double Doping) zwischen den Emittern erleichtert wird. Der Energietransfer kann dabei nach unterschiedlichen Mechanismen erfolgen. Nicht abschließende Beispiele hierfür sind der Energietransfer nach Förster oder Dexter.

Die beschriebenen Emitter-Mischungen enthalten bevorzugt wenigstens zwei Emitter, die beide rotes Licht emittieren. Weiterhin bevorzugt sind Emitter-Mischungen enthaltend wenigstens zwei Emitter, die beide grünes Licht emittieren. Weiterhin bevorzugt sind Emitter-Mischungen enthaltend wenigstens einen Emitter, der rotes Licht emittiert und wenigstens einen Emitter, der grünes Licht emittiert.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektronen (z. B. Al/Ni/NiOₓ, Al/PtOₓ) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

In den weiteren Schichten können generell alle Materialien verwendet werden, wie sie gemäß dem Stand der Technik für die Schichten verwendet werden und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Da die erfindungsgemäßen Verbindungen der Formel (1) eine sehr gute Löslichkeit in organischen Lösemitteln aufweisen, eignen sie sich besonders gut für die Verarbeitung aus Lösung.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (1) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Für die Verarbeitung aus Lösung sind Lösungen bzw. Formulierungen der Verbindungen gemäß Formel (1) erforderlich. Es kann auch bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Lösung bzw. eine Formulierung enthaltend mindestens eine Verbindung gemäß Formel (1) und ein oder mehrere Lösemittel, insbesondere organische Lösemittel. Wie solche Lösungen hergestellt werden können, ist dem Fachmann bekannt und beispielsweise in der WO 02/072714, der WO 03/019694 und der darin zitierten Literatur beschrieben.

Die erfindungsgemäßen elektronischen Vorrichtungen, insbesondere organische Elektrolumineszenzvorrichtungen, zeichnen sich durch folgende überraschende Vorteile gegenüber dem Stand der Technik aus:
1. Die Verbindungen gemäß Formel (1) weisen eine sehr gute Löslichkeit in einer Vielzahl gängiger organischer Lösemittel auf und sind daher sehr gut für die Verarbeitung aus Lösung geeignet. Insbesondere weisen die erfindungsgemäßen Verbindungen eine höhere Löslichkeit auf als die im Stand der Technik beschriebenen ähnlichen Verbindungen.
2. Organische Elektrolumineszenzvorrichtungen enthaltend Verbindungen gemäß Formel (1) als emittierende Materialien weisen eine exzellente Lebensdauer auf. Insbesondere ist die Lebensdauer besser als bei ähnlichen Verbindungen gemäß dem Stand der Technik.
3. Organische Elektrolumineszenzvorrichtungen enthaltend Verbindungen gemäß Formel (1) als emittierende Materialien weisen eine hervorragende Effizienz auf. Insbesondere ist die Effizienz besser als bei ähnlichen Verbindungen gemäß dem Stand der Technik.

Diese oben genannten Vorteile gehen nicht mit einer Verschlechterung der weiteren elektronischen Eigenschaften einher.

Die erfindungsgemäßen Verbindungen sind in der Lage unter bestimmten Voraussetzungen Licht zu emittieren. Somit sind diese Verbindungen sehr vielseitig einsetzbar. Einige der Hauptanwendungsgebiete sind dabei Display- oder Beleuchtungs-Technologien. Weiterhin ist es besonders vorteilhaft, die Verbindungen sowie Vorrichtungen enthaltend diese Verbindungen im Bereich der Phototherapie einzusetzen.

Ein weiterer Gegenstand der vorliegenden Erfindung bezieht sich daher auf die Verwendung der erfindungsgemäßen Verbindungen und Vorrichtungen enthaltend die Verbindungen zur Behandlung, Prophylaxe und Diagnose von Erkrankungen. Noch ein weiterer Gegenstand der vorliegenden Erfindung bezieht sich auf die Verwendung, der erfindungsgemäßen Verbindungen und Vorrichtungen enthaltend die Verbindungen zur Behandlung und Prophylaxe kosmetischen Umstände.

Ein weiterer Gegenstand der vorliegenden Erfindung bezieht sich auf die erfindungsgemäßen Verbindungen zu Herstellung von Vorrichtungen zur Therapie, Prophylaxe und/oder Diagnose therapeutischer Erkrankungen.

Dabei sind viele Erkrankungen sind mit kosmetischen Aspekten assoziiert. So leidet ein Patient mit schwerer Akne in der Gesichtspartie nicht nur an den medizinischen Ursachen und Folgen der Erkrankung, sondern auch an den kosmetischen Begleitumständen.

Phototherapie oder Lichttherapie findet in vielen medizinischen und/oder kosmetischen Bereichen Anwendung. Die erfindungsgemäßen Verbindungen sowie die Vorrichtungen enthaltend diese Verbindungen können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Bestrahlung auch die photodynamischen Therapie (PDT) sowie das Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser und Trinkwasser.

Der Begriff Phototherapie beinhaltet auch jede Art der Kombination von Lichttherapie und anderen Therapiearten, wie bspw. die Behandlung mit Wirkstoffen. Viele Lichttherapien habe zum Ziel äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die erfindungsgemäßen therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt im Sinne der Erfindung sind die Behandlung und/oder Prophylaxe von Psoriasis, Akne, Cellulite, Hautfaltenbildung, Hautalterung, Ikterus und Vitiligo.

Weitere erfindungsgemäße Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw.-veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt im Sinne der Erfindung sind die Behandlung und/oder Prophylaxe von rheumatoider Arthritis, viraler Infektionen, und Schmerzen.

Weitere erfindungsgemäße Anwendungsgebiete für die Verbindungen und/oder Vorrichtungen enthaltend die erfindungsgemäßen Verbindungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere erfindungsgemäße Anwendungsgebiete für die Verbindungen und/oder Vorrichtungen enthaltend die erfindungsgemäßen Verbindungen sind ausgewählt aus der Gruppe der Desinfektionen. Mit den erfindungsgemäßen Verbindungen und/oder mit den erfindungsgemäßen Vorrichtungen können jegliche Art von Objekten (unbelebte Materie) oder Subjekten (lebende Materie wie bspw. Mensch und Tier) zum Zweck der Desinfektion behandelt werden. Hierzu zählt, zum Beispiel, die Desinfektion von Wunden, die Reduktion von Bakterien, das Desinfizieren chirurgischer Instrumente oder anderer Gegenstände, das Desinfizieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie emittieren Vorrichtungen enthaltend die erfindungsgemäßen Verbindungen bevorzugt Licht der Wellenlänge zwischen 250 and 1250 nm, besonders bevorzugt zwischen 300 and 1000 nm und insbesondere bevorzugt zwischen 400 and 850 nm.

In einer besonders bevorzugten Ausführungsform der vorliegenden Erfindung werden die erfindungsgemäßen Verbindungen in einer organischen lichtemittierenden Diode (OLED) oder einer organischen lichtemittierenden elektrochemischen Zelle (OLEC) zum Zwecke der Phototherpie eigesetzt. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten,-intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken,Textilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringerer Bestrahlungsintensitäten an nahezu jedem Ort und zu jeder Tageszeit möglich sind. Die Bestrahlungen können stationär, ambulant und/oder selbst, d.h., ohne Einleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen mit kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer erfindungsgemäßen Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Verbindung **I** kann nach WO 2002/068435 dargestellt werden. Verbindungen **II, IV** und VI können nach DE 102009023155 dargestellt werden. Verbindung **IX** kann nach WO 2006/003000 dargestellt werden.

### Beispiel 1:

### Herstellung der Verbindung III

### Syntheseprozedur für die Darstellung von Verbindung III:

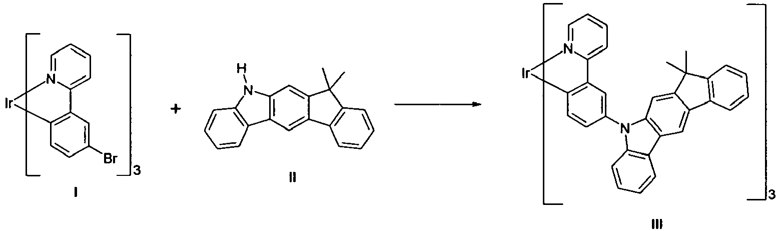

15.0 g (16.8 mmol) Verbindung I, 38.1 g (134.6 mmol) Verbindung **II** und 145 g (685 mmol) K₃PO₄ werden in 2100 mL p-Xylol, suspendiert. Zu dieser Suspension werden 117 mg (0.5 mmol) Pd(OAc)₂ und 6.7 ml eine 1 M Tri.tert-butylphosphin Lösung gegeben. Die Reaktionsmischung wird 27 h unter Rückfluss erhitzt. Nach Erkalten wird die organische Phase abgetrennt, dreimal mit 300 mL Wasser gewaschen und anschließend zur Trockene eingeengt. Der Rückstand wird aus DMSO umkristallisiert und abschließend bei vermindertem Druck getrocknet. Die Ausbeute beträgt 8.5 g (6.7 mmol), entsprechend 34% der Theorie.

### Beispiel 2:

### Herstellung der Verbindung V

### Syntheseprozedur für die Darstellung von Verbindung V:

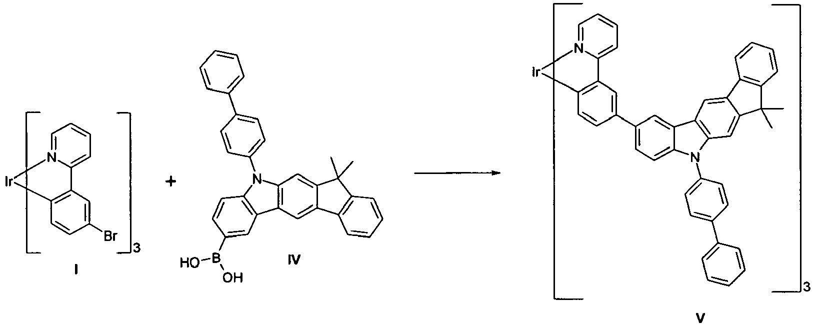

17.0 g (190 mmol) Verbindung I, 74.2 g (479 mmol) Verbindung **IV,** 27.2 g (182 mmol) Kaliumcarbonat werden in 350 mL Toluol und 300 mL Wasser suspendiert. Zu dieser Suspension werden 211 mg (0.18 mmol) Palladium(0)tetrakis(triphenylphosphin) gegeben, und die Reaktionsmischung wird 24 h unter Rückfluss erhitzt. Nach Erkalten wird die organische Phase abgetrennt, dreimal mit 200 mL Wasser gewaschen, mit Natriumsulfat getrocknet und anschließend zur Trockene eingeengt. Der Rückstand wird mit Ethanol gewaschen und aus DMSO umkristallisiert und abschließend bei vermindertem Druck getrocknet. Die Ausbeute beträgt 21.9 g (11.2 mmol), entsprechend 58.7% der Theorie.

### Beispiel 3:

### Herstellung der Verbindung VIII

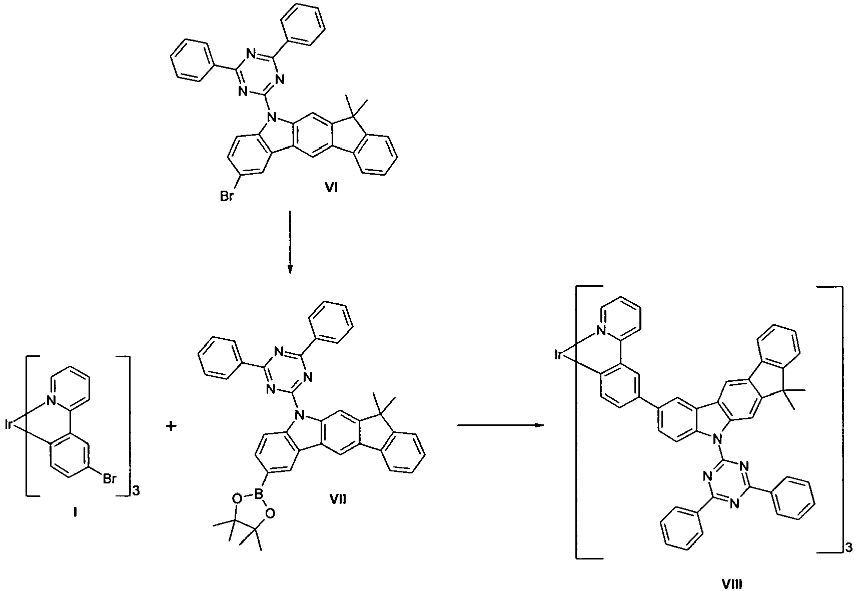

### a) Synthese von der Verbindung VII

22.4 g (37.7 mmol) Verbindung **VI,** 10.5 g (41.5 mmol) Bispinakolatodiboron, 9.8 g (100 mmol) Kaliumacetat werden in 300 mL Dioxan suspendiert. Zu dieser Suspension werden 0.9 g (1.1 mmol) 1,1-Bis(diphenylphosphino)ferrocen-dichloro-palladium(II)*DCM gegeben, die Reaktionsmischung wird 8 h unter Rückfluss erhitzt. Nach Erkalten werden 200 ml Ethylacetat und 400 ml Wasser hinzugegeben und die organische Phase abgetrennt, dreimal mit 300 mL Wasser gewaschen, mit Natriumsulfat getrocknet und anschließend zur Trockene eingeengt. Der Rückstand wird mit Ethanol gewaschen und aus Ethylacetat umkristallisiert und abschließend bei vermindertem Druck getrocknet. Die Ausbeute beträgt 19.3 g (30 mmol), entsprechend 79,8% der Theorie.

### b) Synthese von der Verbindung VIII

27.2 g (30.5 mmol) Verbindung **I**, 118.7 g (185.3 mmol) Verbindung **VII,** 30.3 g (286.6 mmol) Kaliumcarbonat werden in 700 mL Toluol und 600 mL Wasser suspendiert. Zu dieser Suspension werden 290 mg (0.25 mmol) Palladium(0)tetrakis(triphenylphosphin) gegeben, und die Reaktionsmischung wird 48h unter Rückfluss erhitzt. Nach Erkalten wird die organische Phase abgetrennt, dreimal mit 300 mL Wasser gewaschen, mit Natriumsulfat getrocknet und anschließend zur Trockene eingeengt. Der Rückstand wird mit Ethanol gewaschen und aus Toluol umkristallisiert und abschließend bei vermindertem Druck getrocknet. Die Ausbeute beträgt 43.6 g (19.9 mmol), entsprechend 65.1% der Theorie.

### Beispiel 4:

### Herstellung der Verbindung X

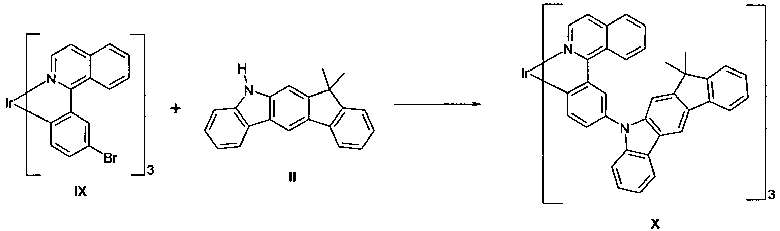

21.0 g (23.6 mmol) Verbindung **IX,** 53.4 g (188.4 mmol) Verbindung **II** und 203 g (960 mmol) K₃PO₄ werden in 3000 mL p-Xylol, suspendiert. Zu dieser Suspension werden 164 mg (0.73 mmol) Pd(OAc)₂ und 9.4 ml eine 1 M Tri.tert-butylphosphin Lösung gegeben. Die Reaktionsmischung wird 42h unter Rückfluss erhitzt. Nach Erkalten wird die organische Phase abgetrennt, dreimal mit 400 mL Wasser gewaschen und anschließend zur Trockene eingeengt. Der Rückstand wird mit Ethanol gewaschen und aus Toluol umkristallisiert und abschließend bei vermindertem Druck getrocknet. Die Ausbeute beträgt 20.6 g (12.3 mmol), entsprechend 53% der Theorie.

### Beispiel 5:

### Herstellung der Verbindung XI

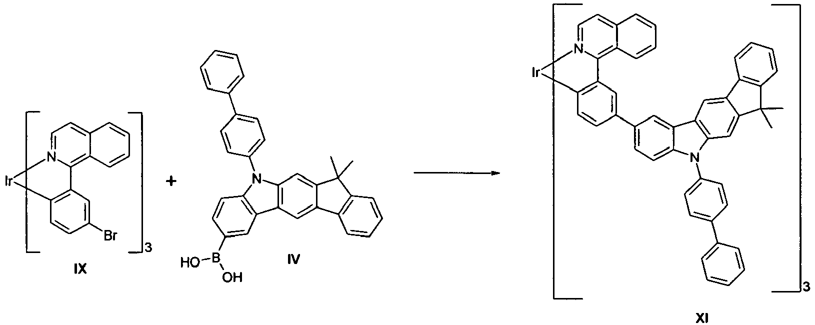

33,8 g (36,2 mmol) Verbindung **IX,** 141 g (480 mmol) Verbindung **IV,** 54 g (365 mmol) Kaliumcarbonat werden in 1300 mL Toluol und 1000 mL Wasser suspendiert. Zu dieser Suspension werden 430 mg (0.36 mmol) Palladium(0)tetrakis(triphenylphosphin) gegeben, und die Reaktionsmischung wird 35 h unter Rückfluss erhitzt. Nach Erkalten wird die organische Phase abgetrennt, dreimal mit 200 mL Wasser gewaschen, mit Natriumsulfat getrocknet und anschließend zur Trockene eingeengt. Der Rückstand wird mit Ethanol gewaschen und aus Toluol umkristallisiert und abschließend bei vermindertem Druck getrocknet. Die Ausbeute beträgt 34 g (16,2 mmol), entsprechend 44,6% der Theorie.

### Beispiel 6:

### Herstellung der Verbindung XII

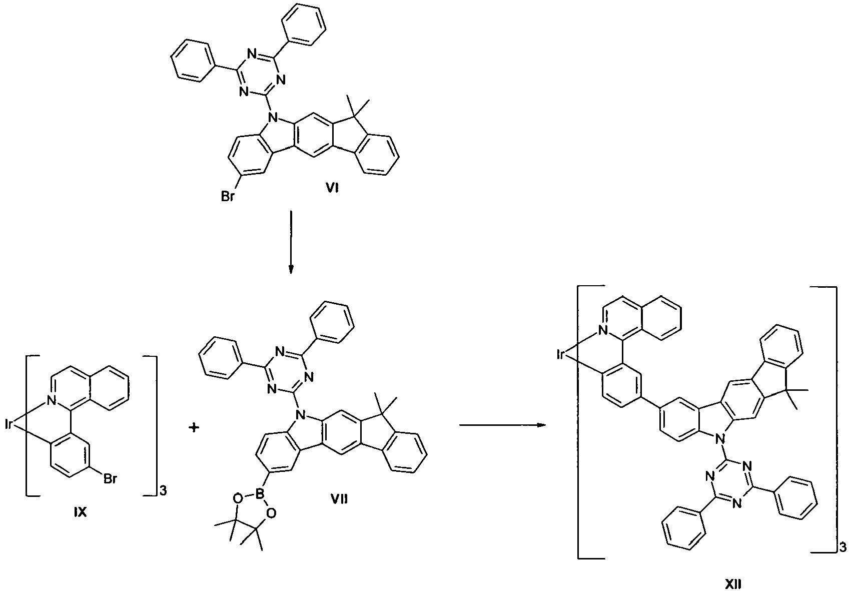

30.4 g (32.6 mmol) Verbindung **IX,** 126.8 g (293.3 mmol) Verbindung **VII,** 30.3 g (286.6 mmol) Kaliumcarbonat werden in 800 mL Toluol und 700 mL Wasser suspendiert. Zu dieser Suspension werden 290 mg (0.25 mmol) Palladium(0)tetrakis(triphenylphosphin) gegeben, und die Reaktionsmischung wird 45 h unter Rückfluss erhitzt. Nach Erkalten wird die organische Phase abgetrennt, dreimal mit 300 mL Wasser gewaschen, mit Natriumsulfat getrocknet und anschließend zur Trockene eingeengt. Der Rückstand wird mit Ethanol gewaschen und aus Toluol umkristallisiert und abschließend bei vermindertem Druck getrocknet. Die Ausbeute beträgt 44 g (18.8 mmol), entsprechend 57.6% der Theorie.

### Beispiel 7 bis 12:

### Herstellung und Charakterisierung organischer Elektrolumineszenzvorrichtungen

Die Strukturen von den erfindungsgemäßen Verbindungen TE-1 bis TE-6, TMM-1 (synthetisiert nach DE 102008036982.9) und TMM-2 (synthetisiert nach DE 102008017591.9) sind der Übersichtlichkeit halber im Folgenden abgebildet.

### Strukturen der Emitter

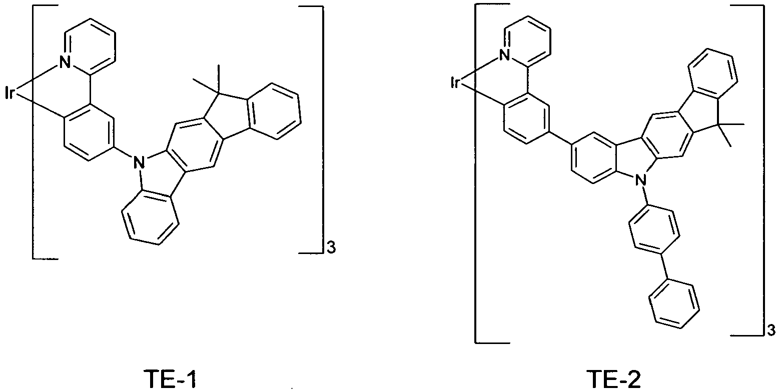

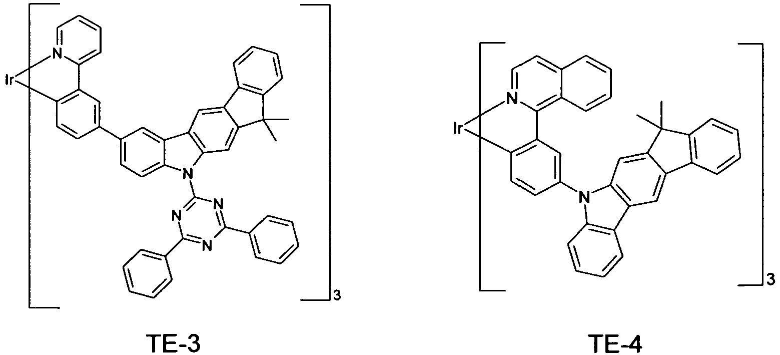

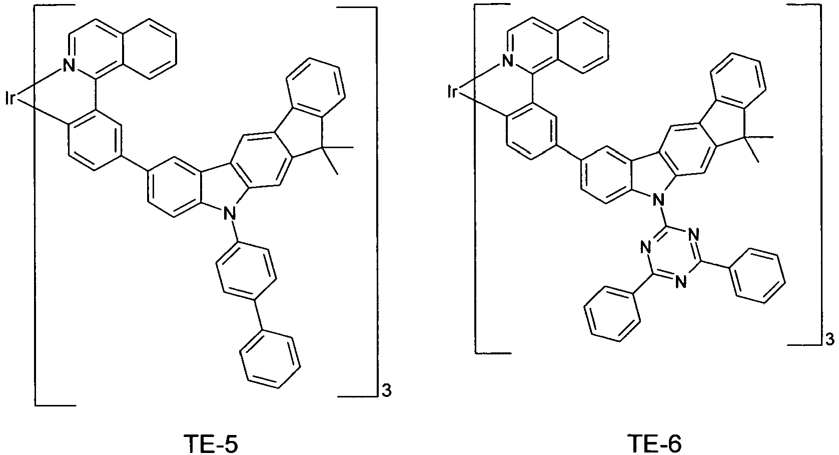

### Strukturen der Matrices

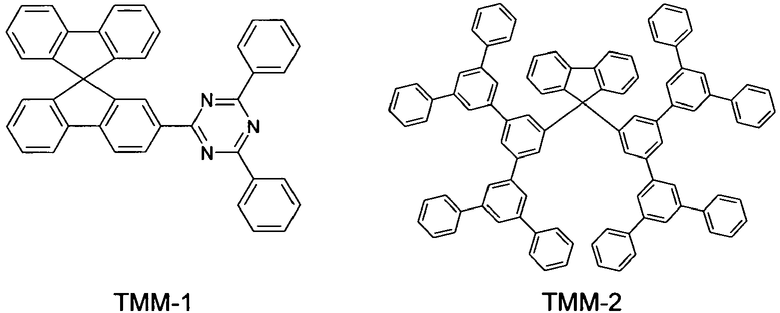

Erfindungsgemäße Materialien können aus Lösung verwendet werden und führen dort zu einfachen Vorrichtungen mit guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 04/037887). Im vorliegenden Fall werden die erfindungsgemäßen Verbindungen TE-1 bis TE-4 in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Strukturierte ITO-Substrate und das Material für die sogenannte Pufferschicht (PEDOT, eigentlich PEDOT:PSS) sind käuflich erhältlich (ITO von Technoprint und anderen, PEDOT:PSS als wässrige Dispersion Clevios Baytron P von H.C. Starck). Die verwendete Interlayer dient der Lochinjektion; in diesem Fall wurde HIL-012 von Merck verwendet. Die Emissionsschicht wird in einer Inertgasatmosphäre, im vorliegenden Fall Argon, aufgeschleudert und 10 min bei 160°C ausgeheizt. Zuletzt wird eine Kathode aus Barium und Aluminium im Vakuum aufgedampft. Zwischen der emittierenden Schicht und der Kathode können auch eine Lochblockierschicht und/oder eine Elektronentransportschicht per Bedampfung aufgebracht werden, auch kann die Interlayer durch eine oder mehrere Schichten ersetzt werden, die lediglich die Bedingung erfüllen müssen, durch den nachgelagerten Prozessierungs-schritt der Abscheidung der emittierenden Schicht aus Lösung nicht wieder abgelöst zu werden.
Die Devices werden standardmäßig charakterisiert, die genannten OLED-Beispiele sind noch nicht optimiert. Tabelle 1 fasst die erhaltenen Daten zusammen. Bei den prozessierten Devices zeigt sich hier, dass die erfindungsgemäßen Materialien den zuvor zur Verfügung stehenden in Effizienz und/oder Lebensdauer überlegen sind. Die OLEd zeigt dabei den folgenden Schichtaufbau: I) Kathode (Ba/Al: 3 nm/150 nm), II) emittierende Schicht (80 nm; 41.5Gew.-% TMM-1 + 41.5 Gew.-% TMM-2 + 17 Gew.-% TE), III) Zwischenschicht (20 nm), IV) Pufferschicht (80 nm; PEDOT) und V) Anode.

**Tabelle 1: Ergebnisse mit aus Lösung prozessierten Materialien in der angegebenen Devicekonfiquration**

| Bsp. | EML 80 nm | Max. Eff. [cd/A] | Spannung [V] bei 100 cd/m² | CIE (x, y) | Lebensdauer [h], Anfangshelligkeit 1000 cd/m² |
|---|---|---|---|---|---|
| 1 | TMM-1 :TMM-2 : TE-1 | 28 | 3.6 | 0.33/0.63 | 38000 |
| 2 | TMM-1 :TMM-2 : TE-2 | 29 | 3.8 | 0.33/0.63 | 35000 |
| 3 | TMM-1 :TMM-2 : TE-3 | 32 | 4.1 | 0.32/0.63 | 32000 |
| 4 | TMM-1 :TMM-2 : TE-4 | 9 | 3.8 | 0.66/0.34 | 21000 |
| 5 | TMM-1 :TMM-2 : TE-5 | 10 | 4.0 | 0.66/0.34 | 25000 |
| 6 | TMM-1 :TMM-2 : TE-6 | 12 | 4.1 | 0.66/0.34 | 20000 |

## Patentansprüche

1. Verbindung gemäß Formel (1)
M(L)n(L')m Formel (1)
wobei die Verbindung eine Teilstruktur M(L)ₙ der Formel (2) enthält 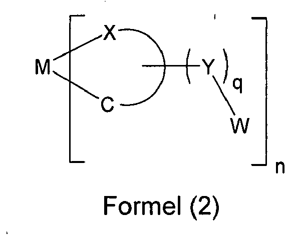 wobei M an einen beliebigen bidentaten Liganden L über X und ein Kohlenstoffatom C bindet und
wobei für die verwendeten Symbole und Indizes gilt:
M ist ein Metall ausgewählt aus der Gruppe bestehend aus Iridium, Rhodium, Platin und Palladium;
X ist gleich oder verschieden bei jedem Auftreten CR¹ oder N;
Y ist gleich oder verschieden bei jedem Auftreten eine Einfachbindung oder eine bivalente Gruppe ausgewählt aus C(R¹)₂, C(=O), O, S, SO, SO₂, NR¹, PR¹ oder P(=O)R¹; ein aliphatischer, aromatischer oder heteroaromatischer Kohlenwasserstoff mit 5 bis 60 Atomen, der jeweils durch einen oder mehrere Reste R³ substituiert sein kann;
R¹ ist gleich oder verschieden bei jedem Auftreten H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen; dabei können zwei oder mehr Reste R¹ auch miteinander ein mono- oder polycyclisches, aliphatisches, aromatisches und/oder benzoannelliertes Ringsystem bilden;
R² ist gleich oder verschieden bei jedem Auftreten H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination aus zwei oder mehr dieser Gruppen; dabei können zwei oder mehrere benachbarte Reste R² miteinander ein mono- oder polycyclisches, aliphatisches oder aromatisches Ringsystem bilden;
R³ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches oder aromatisches Ringsystem bilden;
L' ist gleich oder verschieden bei jedem Auftreten ein beliebiger Coligand;
W ist gleich der Formel (3) 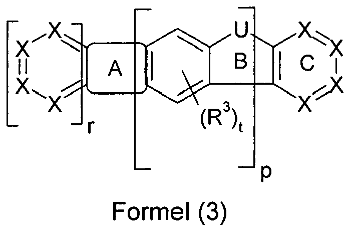 wobei der Ring A in W gegeben ist durch die Verbindung der Formel (3a), die in jeder möglichen Weise mit den benachbarten Ringen kondensiert sein kann 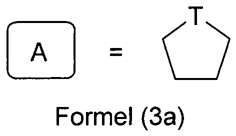 wobei
T ausgewählt wird aus der Gruppe bestehend aus -C(R¹)₂, -N, -NR¹, -O, oder -S;
U sind gleich oder verschieden bei jedem Auftreten ausgewählt aus der Gruppe bestehend aus -C(R¹)₂, -Si(R¹)₂, N, -NR¹,-O,- S,-C(=O), -S(=O), -SO₂, -CF₂, -SF₄, -P,- P(=O)R¹, -PF₂, -P(=S)R¹,- As,-As(=O), -As(=S), -Sb, -Sb(=O) und -Sb(=S);
q, r sind unabhängig voneinander 0 oder 1;
p ist größer oder gleich 1;
t ist 0, 1 oder 2;
n ist 1, 2 oder 3 für M gleich Iridium oder Rhodium und ist 1 oder 2 für M gleich Platin oder Palladium;
m ist 0, 1, 2, 3 oder 4;
dabei werden die Indizes n und m so gewählt, dass die Koordinationszahl am Metall für M gleich Iridium oder Rhodium 6 entspricht und für M gleich Platin oder Palladium 4 entspricht;
dabei können auch mehrere Liganden L miteinander oder L mit L' über eine beliebige Brücke Z verknüpft sein und so ein tridentates, tetradentates, pentadentates oder hexadentates Ligandensystem aufspannen.

2. Verbindung gemäß Anspruch 1, **dadurch gekennzeichnet, dass** die Teilstruktur M(L)ₙ ausgewählt ist aus der Gruppe der Verbindungen mit den Formeln der Formel (4) bis (20) 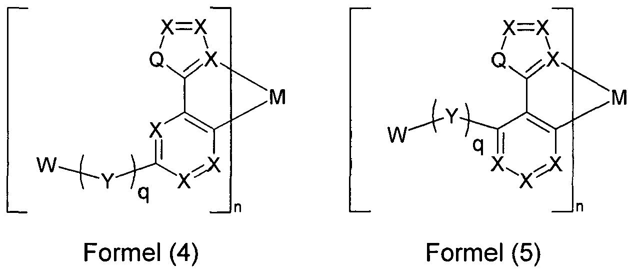 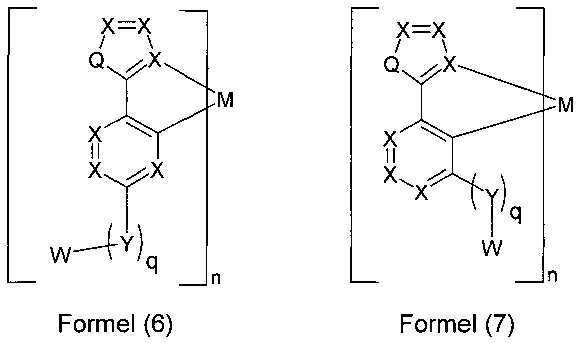 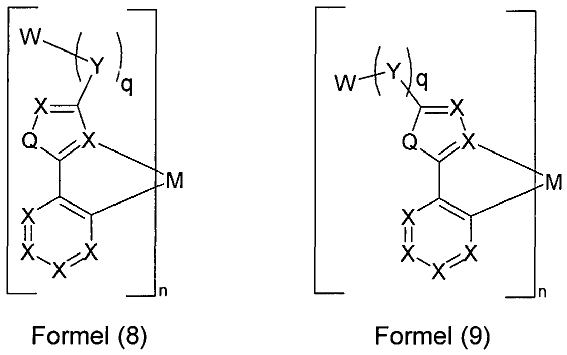 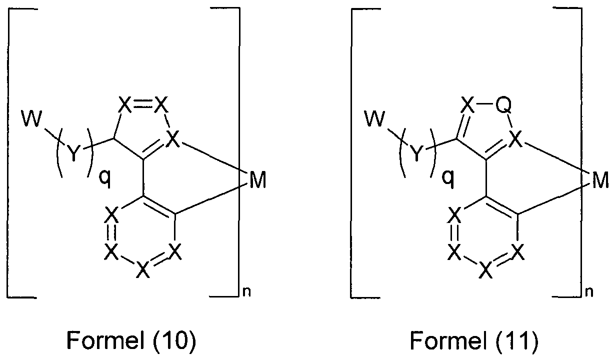 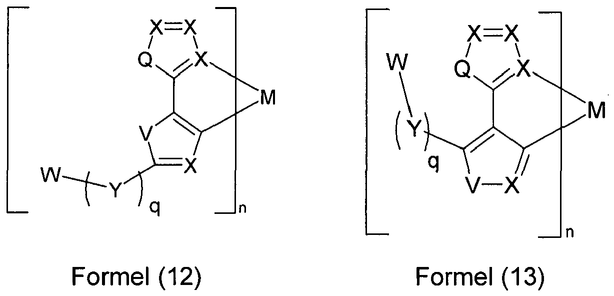 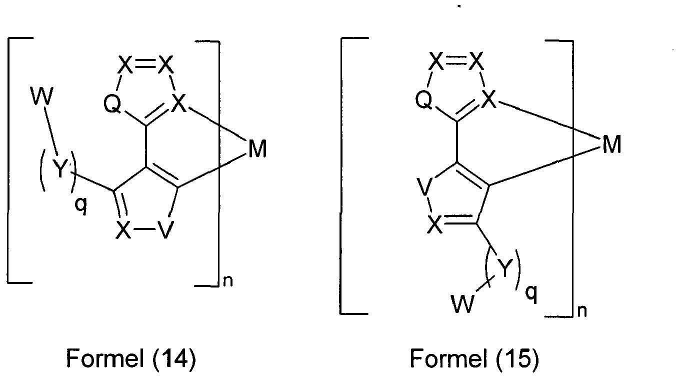 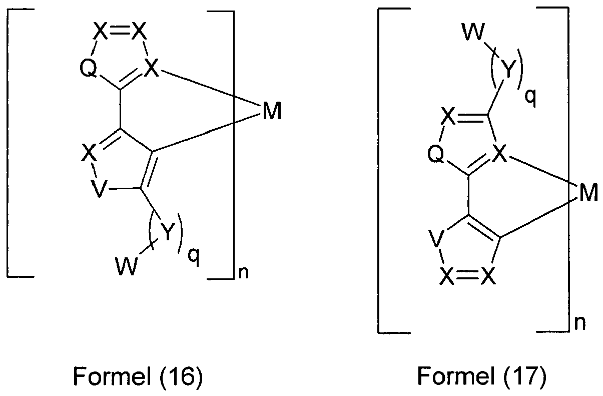 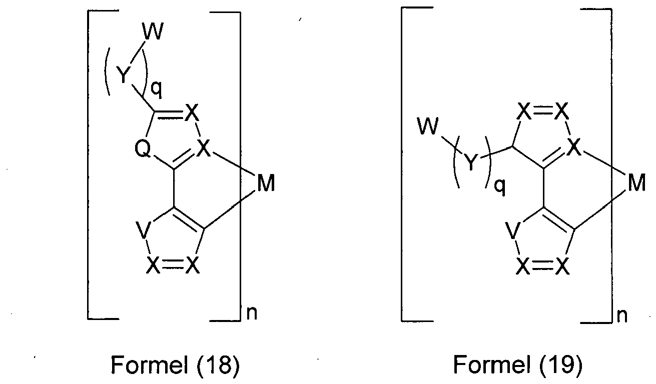 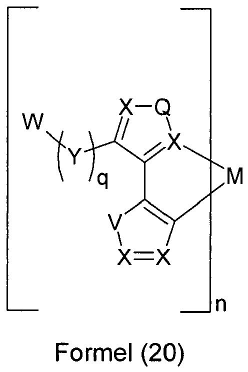 wobei für die verwendeten Symbole und Indizes gilt:
Q ist gleich oder verschieden bei jedem Auftreten R¹C=CR¹, R¹C=N, O, S, Se oder NR¹;
V ist gleich oder verschieden bei jedem Auftreten O, S, Se, NR¹ oder C(R¹)₂.

3. Verbindung nach Anspruch 1 oder 2, **dadurch gekennzeichnet, dass** die Teilstruktur M(L)ₙ ausgewählt ist auch den Formel (4), (9), (12) und (18)

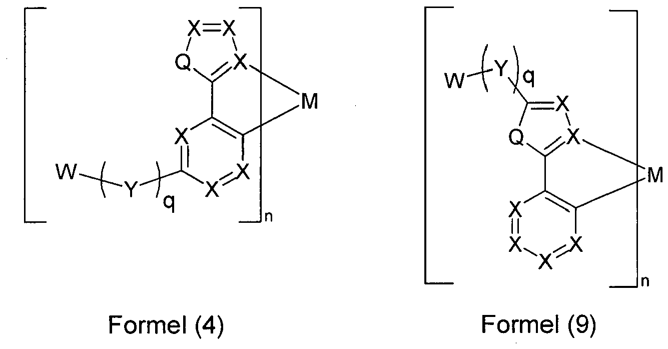

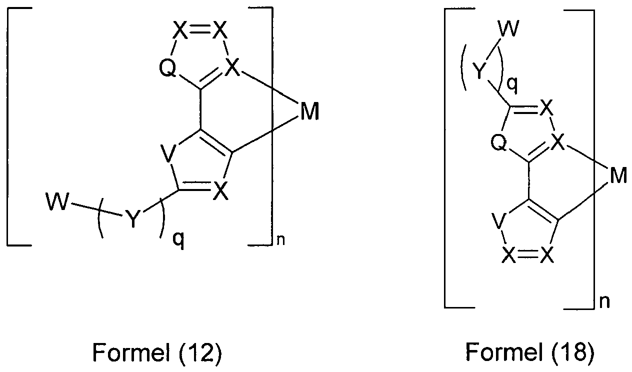

4. Verbindung nach einem oder mehreren der Ansprüche 1 bis 3, **dadurch gekennzeichnet, dass** Teilstruktur M(L)ₙ ausgewählt ist aus den Formel (4) und (12)

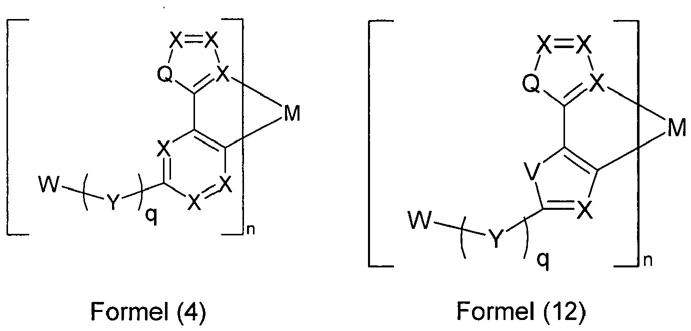

5. Verbindung nach einem oder mehreren der Ansprüche 1 bis 4, **dadurch gekennzeichnet, dass** das Symbol X bei jedem Auftreten für alle nicht direkt an M gebundene Atome für CR¹ steht.

6. Verbindung nach einem oder mehreren der Ansprüche 1 bis 5, **dadurch gekennzeichnet, dass** die Teilstruktur M(L)ₙ ausgewählt ist aus den Formeln (25) bis (28)

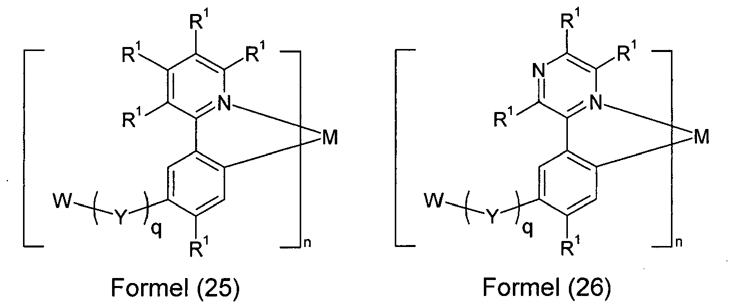

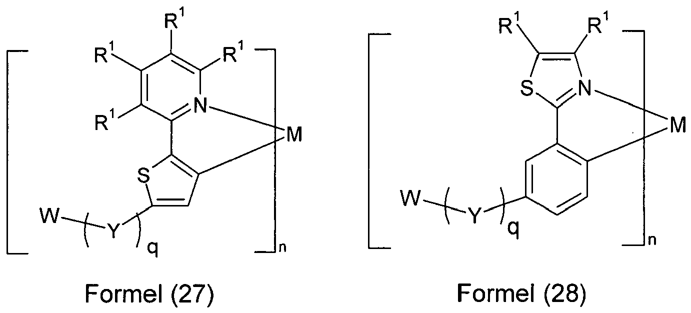

7. Verbindung nach einem oder mehreren der Ansprüche 1 bis 6, **dadurch gekennzeichnet, dass** W ausgewählt wird aus den Verbindungen der Formeln (66) bis (71) und (78) bis (89) 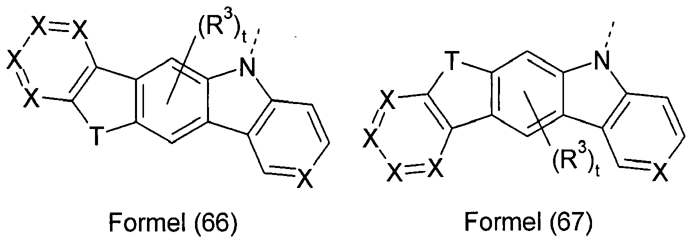 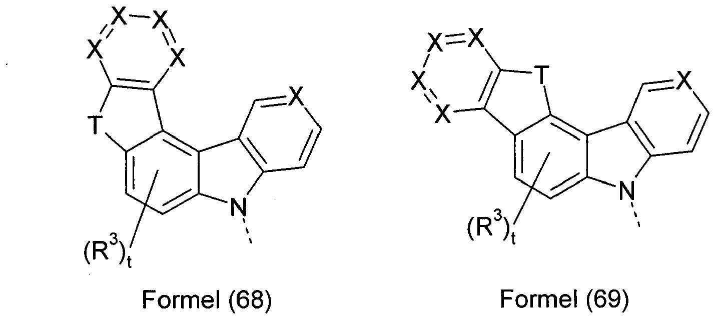 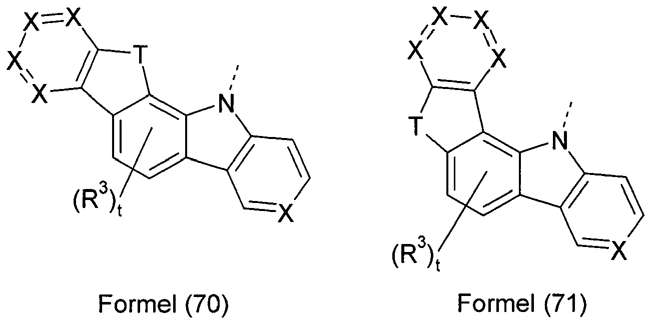 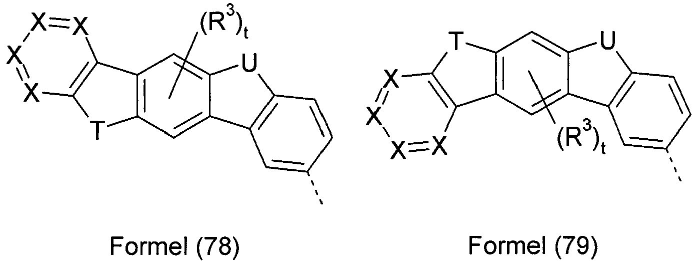 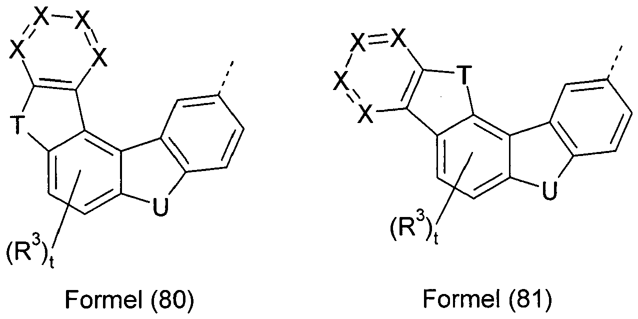 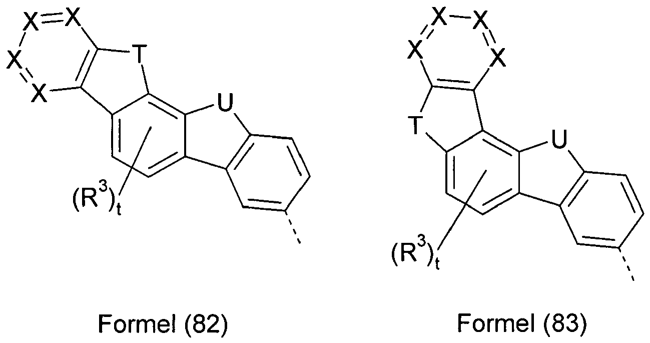 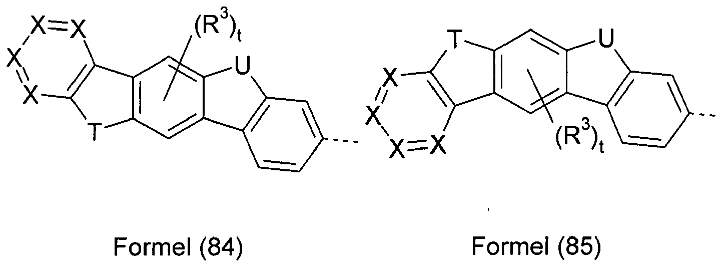 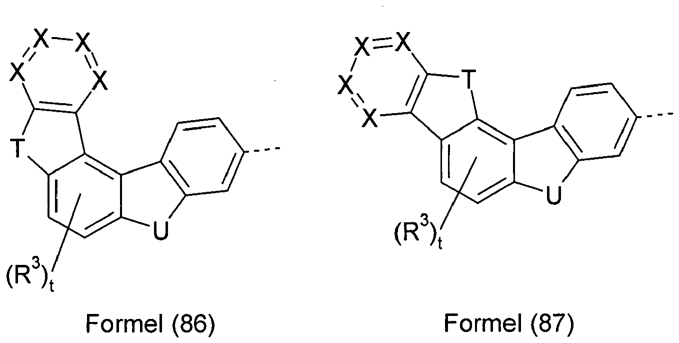 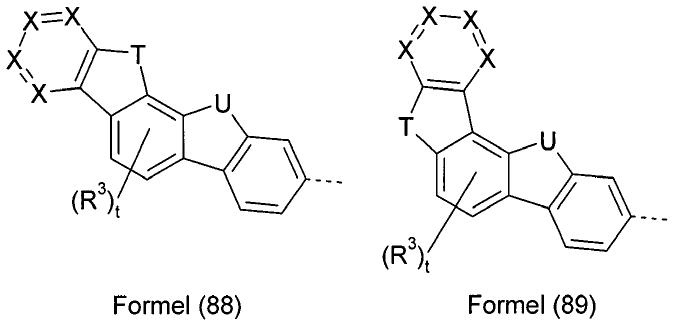 wobei die gepunktete Linie für die Verbindung zwischen W und Y stehen.

8. Verbindung nach einem oder mehreren der Ansprüche 1 bis 7, **dadurch gekennzeichnet, dass** Y gleich oder verschieden bei jedem Auftreten eine Einfachbindung oder ein aliphatischer, aromatischer oder heteroaromatischer Kohlenwasserstoff mit 5 bis 60 Atomen, der jeweils durch einen oder mehrere Reste R³ substituiert sein kann ist.

9. Verbindung nach einem oder mehreren der Ansprüche 1 bis 8, **dadurch gekennzeichnet, dass** der Rest Y ausgewählt ist aus den Verbindungen der Formeln (33) bis (63) 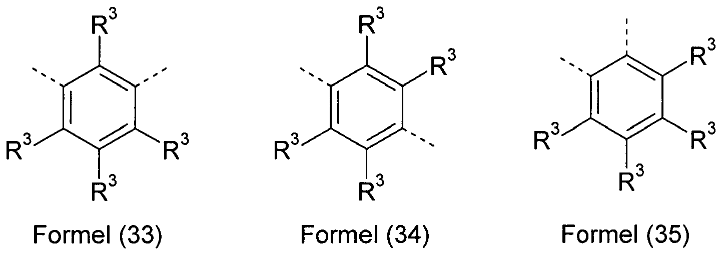 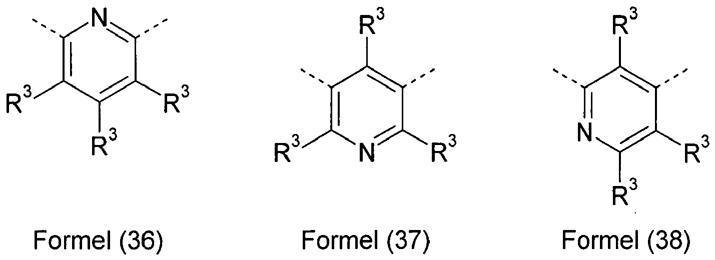 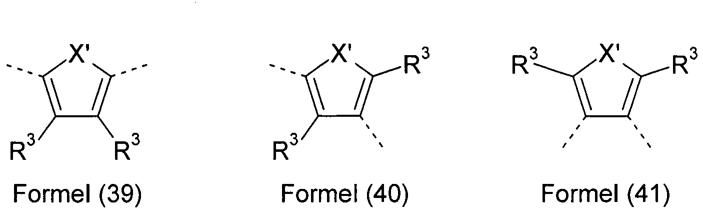 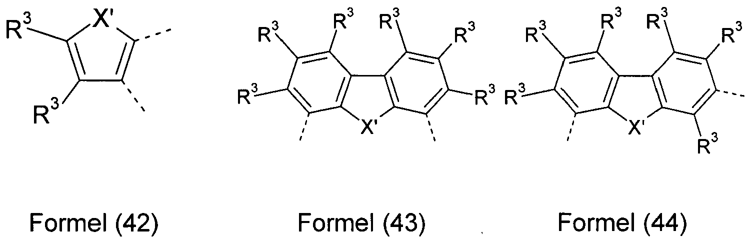 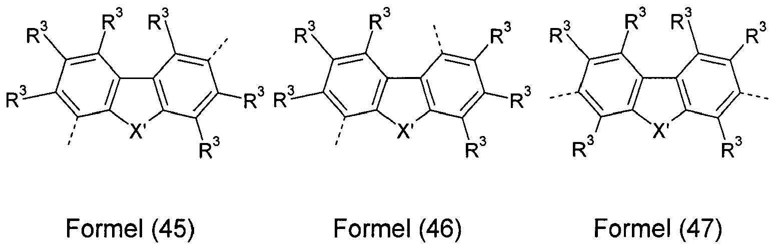 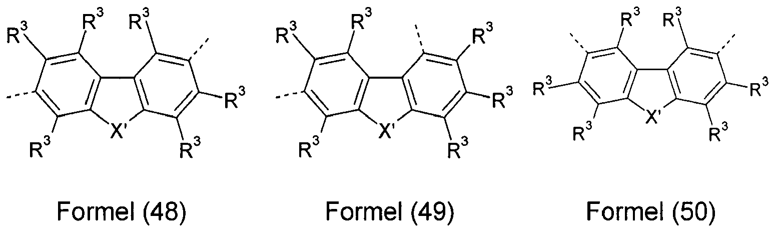 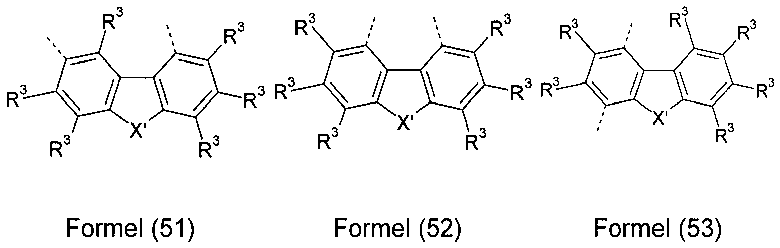 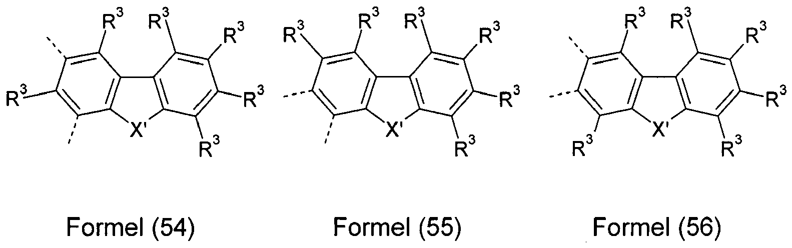 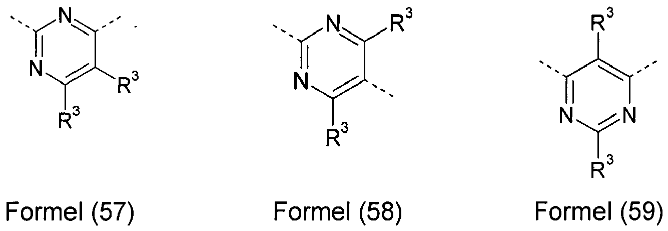 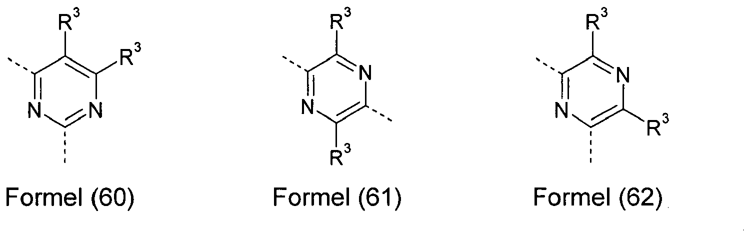 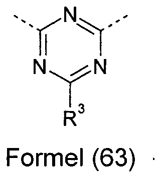 wobei X' ausgewählt ist aus der Gruppe O, S, Se, NR¹, C(R¹)₂ oder S(=O)₂ und die gepunktete Linien für die Verknüpfungen zu W bzw. zu dem direkt an das Metall M gebundenen Teil des organischen Liganden stehen.

10. Verfahren zur Herstellung einer Verbindung nach einem oder mehreren der Ansprüche 1 bis 9 durch Umsetzung der entsprechenden freien Liganden mit Metall alkoholaten der Formel (155), mit Metallketoketonaten der Formel (156) oder mit Metallhalogeniden der Formel (157)
M(OR¹)ₙ Formel (155) 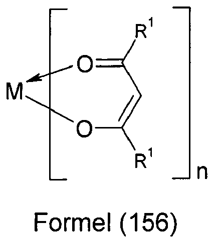
MHalₙ Formel (157)
wobei wobei die Symbole M, n und R¹ die oben angegebenen Bedeutungen haben und Hal = F, Cl, Br oder I ist.

11. Formulierung enthaltend wenigstens eine Verbindung nach einem oder mehreren der Ansprüche 1 bis 9 und wenigstens ein Lösungsmittel.

12. Verwendung einer Verbindung nach einem oder mehreren der Ansprüche 1 bis 9 in einer elektronischen Vorrichtung, bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

13. Elektronische Vorrichtung, enthaltend mindestens eine Verbindung nach einem oder mehreren der Ansprüche 1 bis 9, bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

14. Organische Elektrolumineszenzvorrichtung nach Anspruch 13, **dadurch gekennzeichnet, dass** die Verbindung nach einem oder mehreren der Ansprüche 1 bis 9 als emittierende Verbindung in einer oder mehreren emittierenden Schichten eingesetzt wird, bevorzugt in Kombination mit einem Matrixmaterial, wobei das Matrixmaterial bevorzugt ausgewählt ist aus der Gruppe bestehend aus Ketonen, Phosphinoxiden, Sulfoxiden, Sulfonen, Triarylaminen, Carbazolderivaten, Indolocarbazolderivaten, Indenocarbazolderivaten, Azacarbazolderivaten, bipolaren Matrixmaterialien, Silanen, Azaborolen, Boronestern, Triazinderivaten, Zinkkomplexen, Diaza- oder Tetraazasilolderivaten oder Diazaphospholderivaten oder Mischungen dieser Matrixmaterialien.

15. Verbindung nach einem oder mehreren der Ansprüche 1 bis 9 oder Vorrichtung nach Anspruch 13 oder 14 zur Therapie, Prophylaxe und /oder Diagnose von Erkrankungen und/oder kosmetischer Umstände.

## Claims

1. Compound of the formula (1)
M(L)ₙ(L')ₘ formula (1)
where the compound contains a moiety M(L)ₙ of the formula (2) 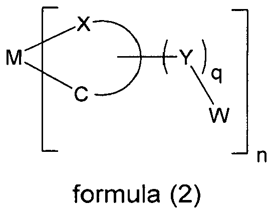 where M is bonded to any desired bidentate ligand L via X and a carbon atom C, and
where the following applies to the symbols and indices used:
M is a metal selected from the group consisting of iridium, rhodium, platinum and palladium;
X is, identically or differently on each occurrence, CR¹ or N;
Y is, identically or differently on each occurrence, a single bond or a divalent group selected from C(R')₂, C(=O), O, S, SO, SO₂, NR¹, PR¹ or P(=O)R¹; an aliphatic, aromatic or heteroaromatic hydrocarbon having 5 to 60 atoms, which may in each case be substituted by one or more radicals R³;
R¹ is, identically or differently on each occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C≡C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups; two or more radicals R¹ here may also form a mono- or polycyclic, aliphatic, aromatic and/or benzo-fused ring system with one another;
R² is, identically or differently on each occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R³, where one or more non-adjacent CH₂ groups may be replaced by R³C=CR³, C=C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, or a diarylamino group, diheteroarylamino group or arylheteroarylamino group having 10 to 40 aromatic ring atoms, which may be substituted by one or more radicals R³, or a combination of two or more of these groups; two or more adjacent radicals R² here may form a mono- or polycyclic, aliphatic or aromatic ring system with one another;
R³ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents R³ here may also form a mono- or polycyclic, aliphatic or aromatic ring system with one another;
L' is, identically or differently on each occurrence, any desired co-ligand;
W is equal to the formula (3) 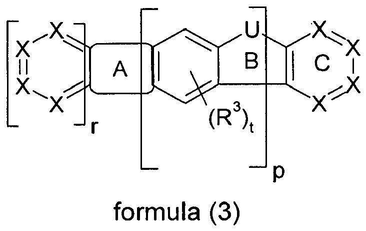 where the ring A in W is given by the compound of the formula (3a), which can be condensed with the adjacent rings in any possible manner 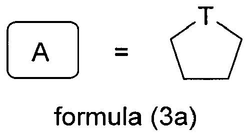 where
T is selected from the group consisting of -C(R¹)₂, -N, -NR¹, -O, or -S.
U is selected, identically or differently on each occurrence, from the group consisting of -C(R¹)₂, -Si(R¹)₂, N, -NR¹,-O,- S, -C(=O), -S(=O), -SO₂, -CF₂, -SF₄, -P,- P(=O)R¹, -PF₂, -P(=S)R¹, -As,- As(=O), -As(=S), -Sb, -Sb(=O) and -Sb(=S);
q, r are, independently of one another, 0 or 1;
p is greater than or equal to 1;
t is 0, 1 or 2;
n is 1, 2 or 3 for M equal to iridium or rhodium and is 1 or 2 for M equal to platinum or palladium;
m is 0, 1, 2, 3 or 4;
the indices n and m here are selected so that the coordination number on the metal corresponds to 6 for M equal to iridium or rhodium and corresponds to 4 for M equal to platinum or palladium;
a plurality of ligands L here may also be linked to one another or L may be linked to L' via any desired bridge Z and thus form a tridentate, tetradentate, pentadentate or hexadentate ligand system.

2. Compound according to Claim 1, **characterised in that** the moiety M(L)ₙ is selected from the group of the compounds of the formulae (4) to (20) 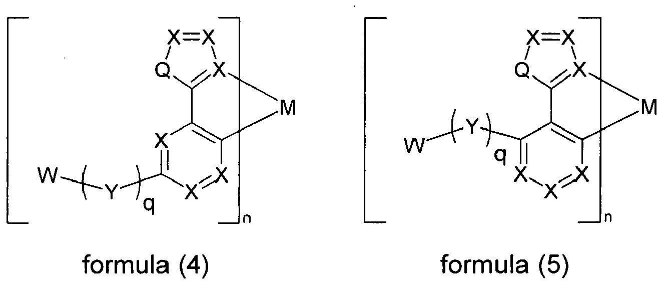 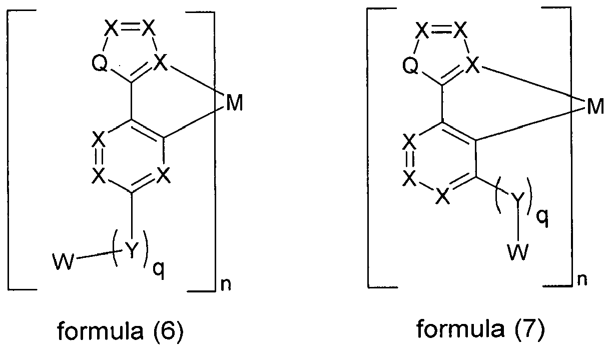 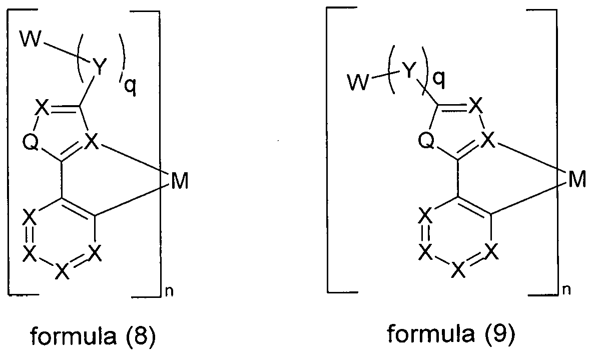 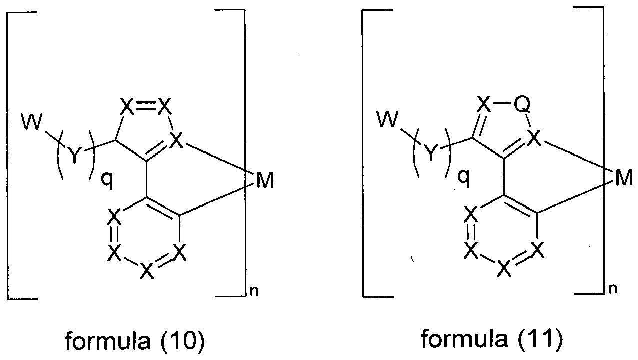 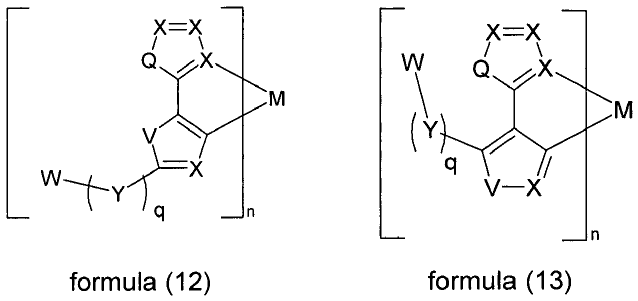 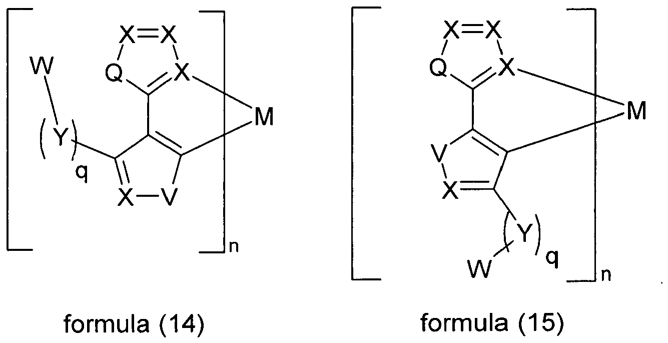 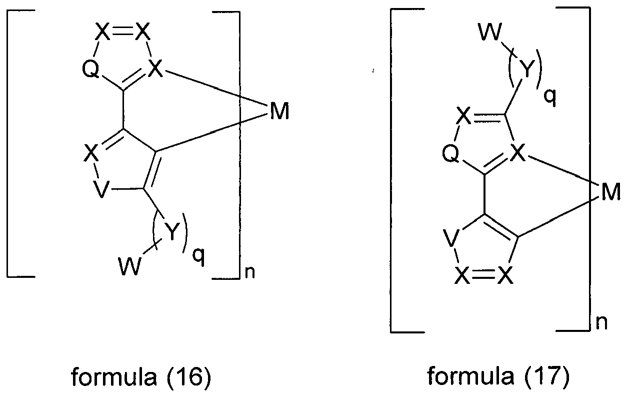 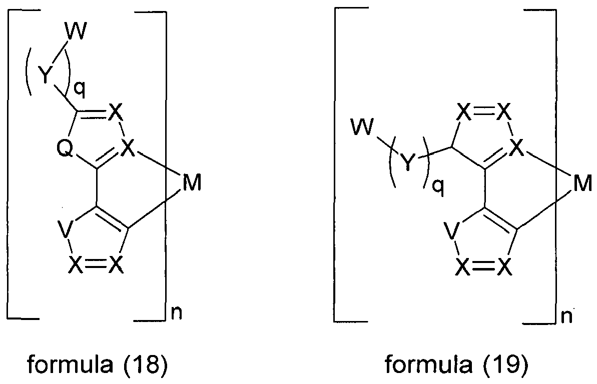 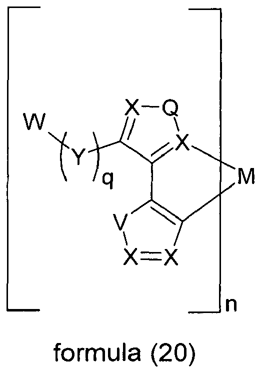 where the following applies to the symbols and indices used:
Q is, identically or differently on each occurrence, R¹C=CR¹, R¹C=N, O, S, Se or NR¹;
V is, identically or differently on each occurrence, O, S, Se, NR¹ or C(R¹)_{2.}

3. Compound according to Claim 1 or 2, **characterised in that** the moiety M(L)ₙ is selected from the formulae (4), (9), (12) and (18)

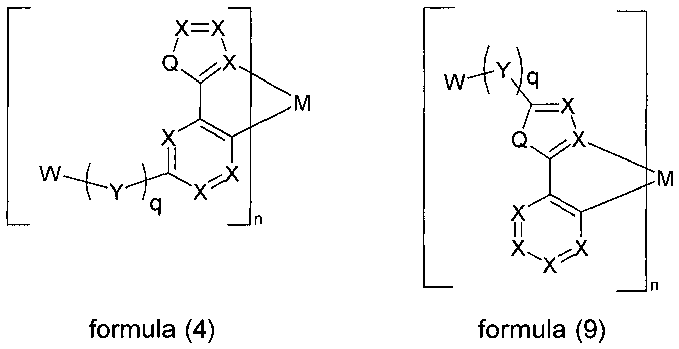

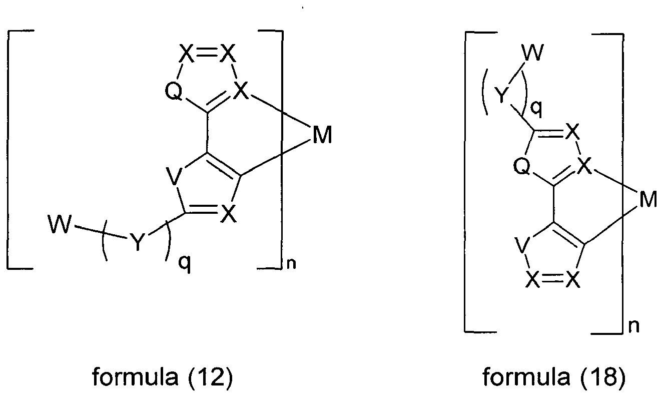

4. Compound according to one or more of Claims 1 to 3, **characterised in that** moiety M(L)ₙ is selected from the formulae (4) and (12)

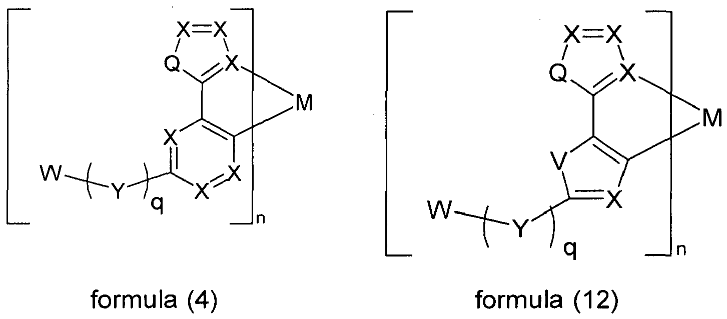

5. Compound according to one or more of Claims 1 to 4, **characterised in that** the symbol X stands on each occurrence for CR¹ for all atoms which are not bonded directly to M.

6. Compound according to one or more of Claims 1 to 5, **characterised in that** the moiety M(L)ₙ is selected from the formulae (25) to (28)

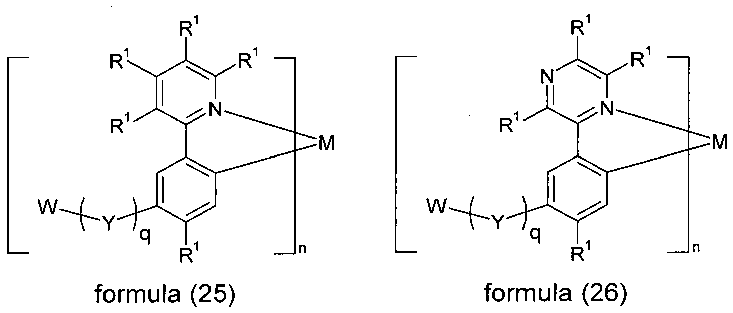

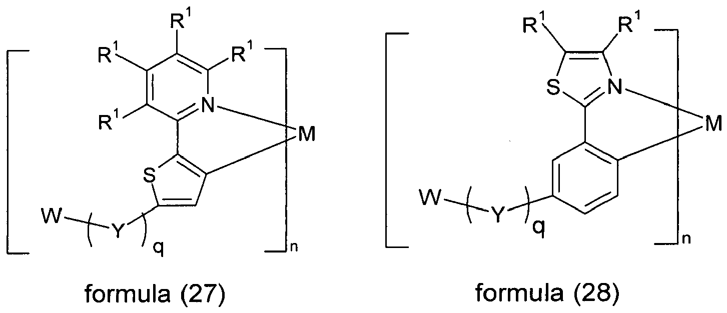

7. Compound according to one or more of Claims 1 to 6, **characterised in that** W is selected from the compounds of the formulae (66) to (71) and (78) to (89) 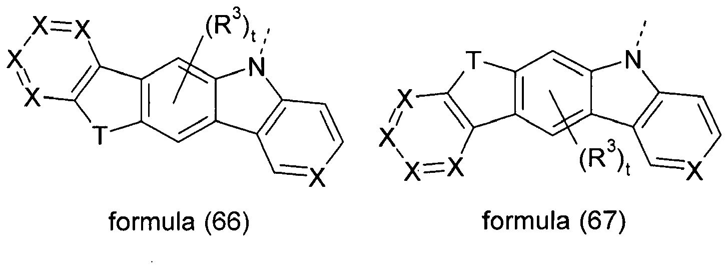 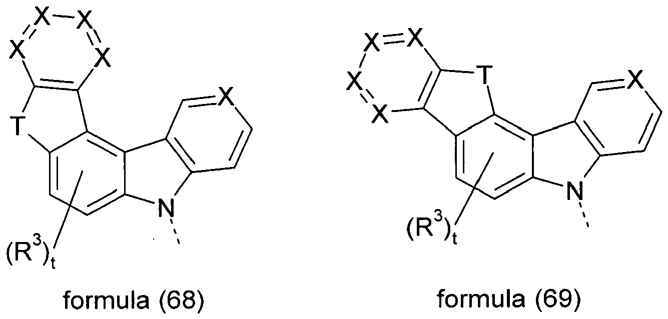 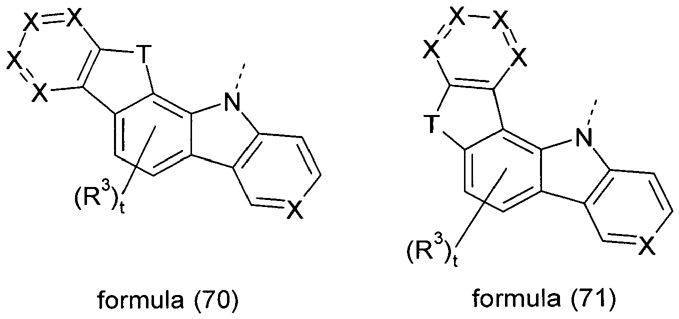 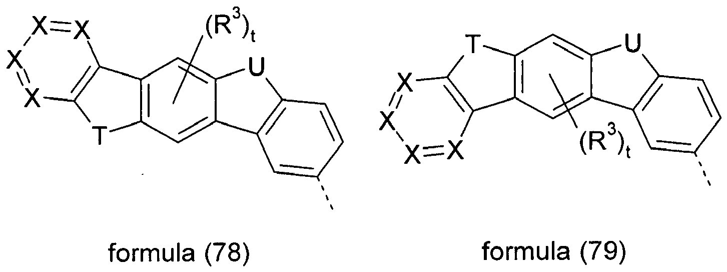 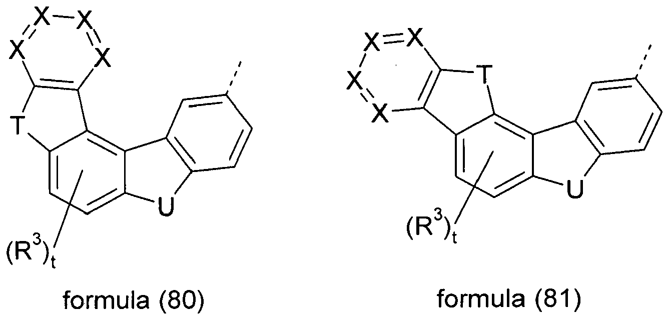 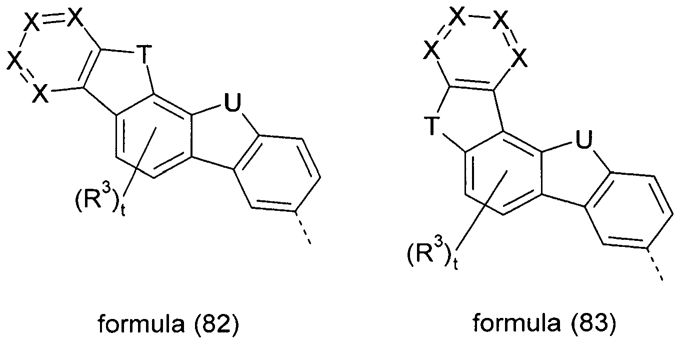 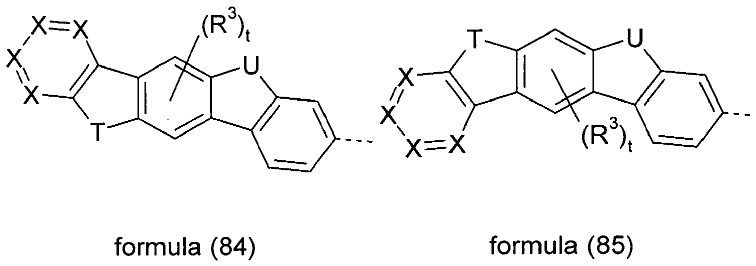 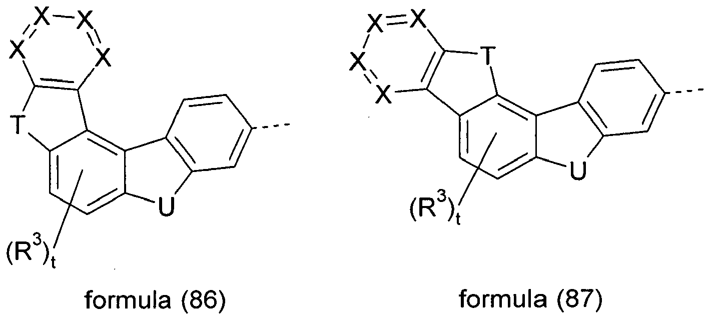 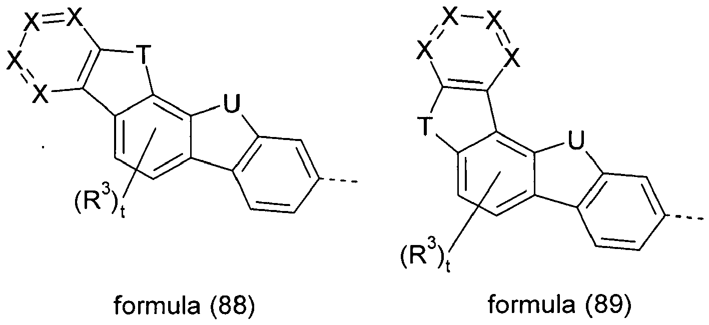 where the dotted lines stand for the connection between W and Y.

8. Compound according to one or more of Claims 1 to 7, **characterised in that** Y is, identically or differently on each occurrence, a single bond or an aliphatic, aromatic or heteroaromatic hydrocarbon having 5 to 60 atoms, which may in each case be substituted by one or more radicals R³.

9. Compound according to one or more of Claims 1 to 8, **characterised in that** the radical Y is selected from the compounds of the formulae (33) to (63) 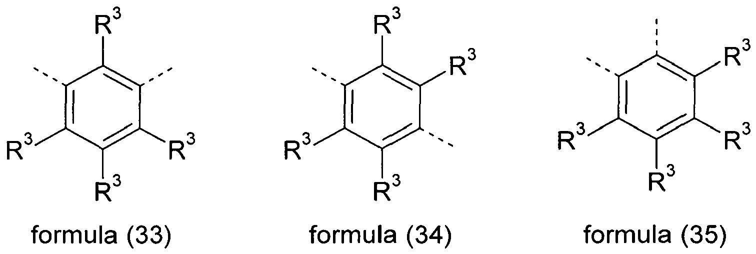 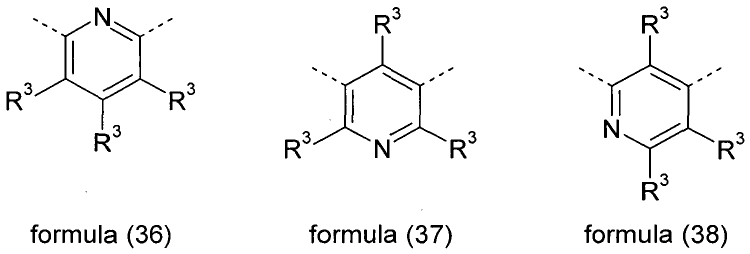 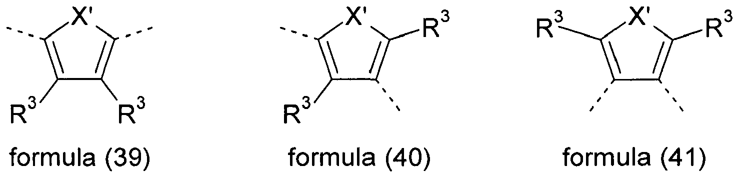 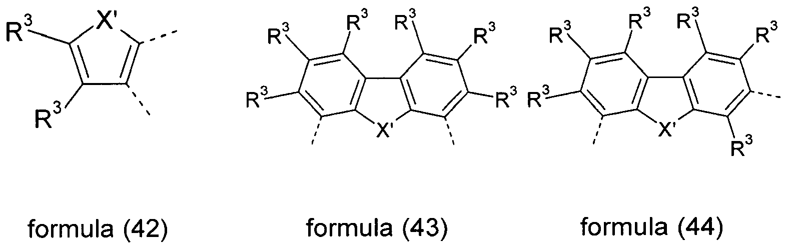 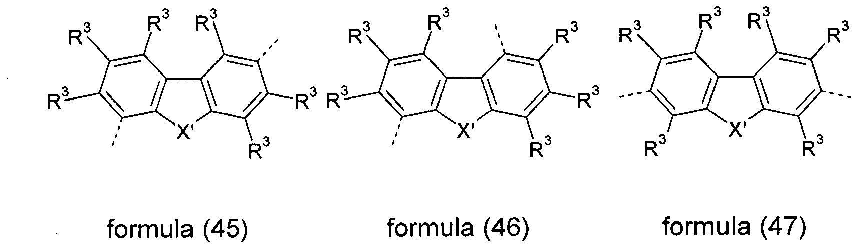 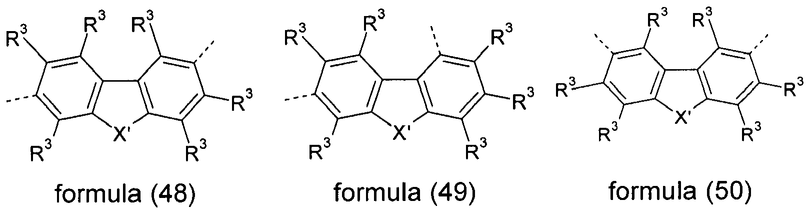 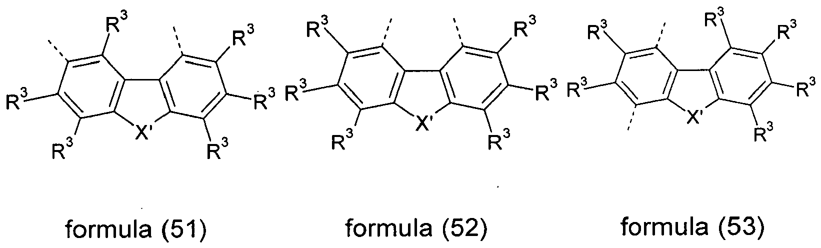 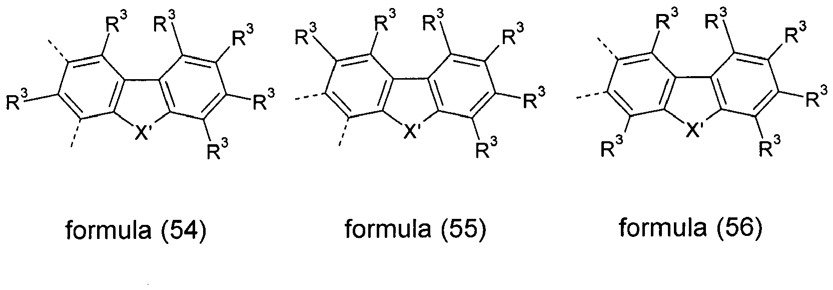 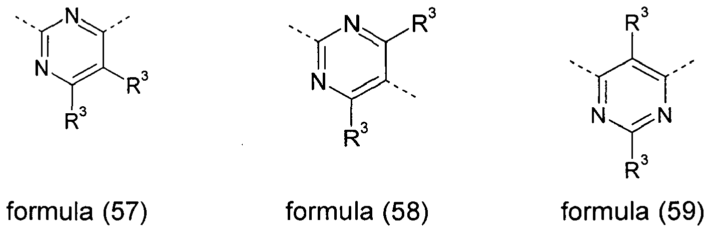 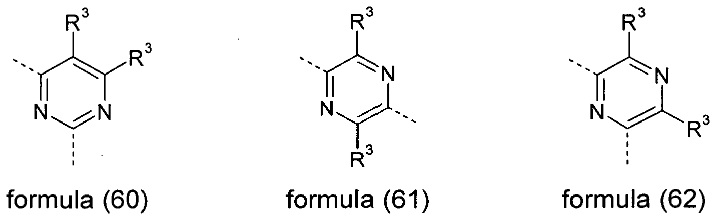 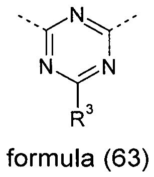 where X' is selected from the group O, S, Se, NR¹, C(R¹)₂ or S(=O)₂ and the dotted lines stand for the links to W or to the part of the organic ligand which is bonded directly to the metal M.

10. Process for the preparation of a compound according to one or more of Claims 1 to 9 by reaction of the corresponding free ligands with metal alkoxides of the formula (155), with metal ketoketonates of the formula (156) or with metal halides of the formula (157)
M(OR¹)ₙ formula (155) 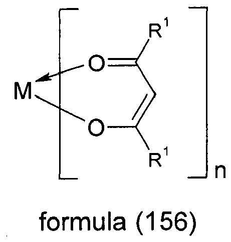
MHalₙ formula (157)
where the symbols M, n and R¹ have the meanings indicated above and Hal = F, Cl, Br or I.

11. Formulation comprising at least one compound according to one or more of Claims 1 to 9 and at least one solvent.

12. Use of a compound according to one or more of Claims 1 to 9 in an electronic device, preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers).

13. Electronic device comprising at least one compound according to one or more of Claims 1 to 9, preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers).

14. Organic electroluminescent device according to Claim 13, **characterised in that** the compound according to one or more of Claims 1 to 9 is employed as emitting compound in one or more emitting layers, preferably in combination with a matrix material, where the matrix material is preferably selected from the group consisting of ketones, phosphine oxides, sulfoxides, sulfones, triarylamines, carbazole derivatives, indolocarbazole derivatives, indenocarbazole derivatives, azacarbazole derivatives, bipolar matrix materials, silanes, azaboroles, boronic esters, triazine derivatives, zinc complexes, diaza- or tetraazasilole derivatives or diazaphosphole derivatives or mixtures of these matrix materials.

15. Compound according to one or more of Claims 1 to 9 or device according to Claim 13 or 14 for the therapy, prophylaxis and/or diagnosis of diseases and/or cosmetic conditions.

## Revendications

1. Composé de la formule (1) :
M(L)ₙ(L')ₘ formule (1)
dans laquelle le composé contient une moitié M(L)ₙ de la formule (2) : 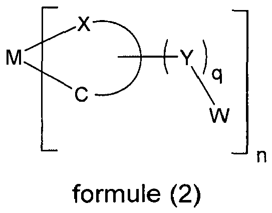 dans laquelle M est lié à n'importe quel ligand bidenté souhaité L via X et un atome de carbone C, et
où ce qui suit s'applique aux symboles et indices utilisés :
M est un métal choisi parmi le groupe constitué par iridium, rhodium, platine et palladium ;
X est, de manière identique ou différente pour chaque occurrence, CR¹ ou N ;
Y est, de manière identique ou différente pour chaque occurrence, une liaison simple ou un groupe divalent choisi parmi C(R¹)₂, C(=O), O, S, SO, SO₂, NR¹, PR¹ ou P(=O)R¹ ; un hydrocarbone aliphatique, aromatique ou hétéroaromatique comportant de 5 à 60 atomes, lesquels peuvent, dans chaque cas, être substitués par un radical ou plusieurs radicaux R³ ;
R¹ est, de manière identique ou différente pour chaque occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite comportant de 1 à 40 atomes de C ou un groupe alkényle ou alkynyle en chaîne droite comportant de 2 à 40 atomes de C ou un groupe alkyle, alkényle, alkynyle, alcoxy ou thioalcoxy ramifié ou cyclique comportant de 3 à 40 atomes de C, dont chacun peut être substitué par un radical ou plusieurs radicaux R², où un ou plusieurs groupes CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique comportant de 5 à 60 atomes de cycle aromatique, lesquels peuvent, dans chaque cas, être substitués par un radical ou plusieurs radicaux R², ou un groupe aryloxy ou hétéroaryloxy comportant de 5 à 60 atomes de cycle aromatique, lesquels peuvent être substitués par un ou plusieurs radicaux R², ou un groupe diarylamino, un groupe dihétéroarylamino ou un groupe arylhétéroarylamino comportant de 10 à 40 atomes de cycle aromatique, lesquels peuvent être substitués par un radical ou plusieurs radicaux R², ou une combinaison de deux de ces groupes ou plus ; deux radicaux R¹ ou plus peuvent ici également former un système de cycle monocyclique ou polycyclique, aliphatique, aromatique et/ou benzo-fusionné l'un avec l'autre ou les uns avec les autres ;
R² est, de manière identique ou différente pour chaque occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, un groupe alkyle, alcoxy ou thioalcoxy en chaîne droite comportant de 1 à 40 atomes de C ou un groupe alkényle ou alkynyle en chaîne droite comportant de 2 à 40 atomes de C ou un groupe alkyle, alkényle, alkynyle, alcoxy ou thioalcoxy ramifié ou cyclique comportant de 3 à 40 atomes de C, dont chacun peut être substitué par un radical ou plusieurs radicaux R³, où un ou plusieurs groupes CH₂ non adjacents peuvent être remplacé(s) par R³C=CR³, C=C, Si(R³)₂, Ge(R³)₂, Sn(R³)₂, C=O, C=S, C=Se, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, ou un système de cycle aromatique ou hétéroaromatique comportant de 5 à 60 atomes de cycle aromatique, lesquels peuvent, dans chaque cas, être substitués par un radical ou plusieurs radicaux R³, ou un groupe aryloxy ou hétéroaryloxy comportant de 5 à 60 atomes de cycle aromatique, lesquels peuvent être substitués par un radical ou plusieurs radicaux R³, ou un groupe diarylamino, un groupe dihétéroarylamino ou un groupe arylhétéro-arylamino comportant de 10 à 40 atomes de cycle aromatique, lesquels peuvent être substitués par un ou plusieurs radicaux R³, ou une combinaison de deux de ces groupes ou plus ; deux radicaux adjacents R² ou plus peuvent former un système de cycle monocyclique ou polycyclique, aliphatique ou aromatique l'un avec l'autre ou les uns avec les autres ;
R³ est, de manière identique ou différente pour chaque occurrence, H, D, F ou un radical hydrocarbone aliphatique, aromatique et/ou hétéroaromatique comportant de 1 à 20 atomes de C, où, en outre, un ou plusieurs atome(s) de H peut/peuvent être remplacé(s) par F ; deux substituants R³ ou plus peuvent ici également former un système de cycle monocyclique ou polycyclique, aliphatique ou aromatique l'un avec l'autre ou les uns avec les autres ;
L' est, de manière identique ou différente, pour chaque occurrence, n'importe quel co-ligand souhaité ;
W est égal à la formule (3) : 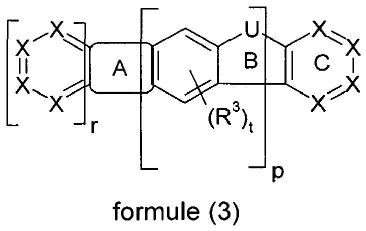
où le cycle A dans W est donné par le composé de la formule (3a), lequel peut être condensé avec les cycles adjacents de n'importe quelle manière possible 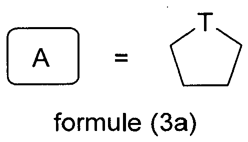 où
T est sélectionné parmi le groupe constitué par-C(R¹)₂, -N, -NR¹,-O, ou -S,
U est choisi, de manière identique ou différente pour chaque occurrence, parmi le groupe constitué par -C(R¹)₂, -Si(R¹)₂, N, -NR¹, -O,- S, -C(=O), -S(=O), -SO₂, -CF₂, -SF₄, -P,- P(=O)R¹, -PF₂, -P(=S)R¹, -As,- As(=O), -As(=S), -Sb, -Sb(=O) et -Sb(=S) ;
q, r sont, indépendamment l'un de l'autre, 0 ou 1 ;
p est supérieur ou égal à 1 ;
t est 0, 1 ou 2 ;
n est 1, 2 ou 3 pour M égal à iridium ou rhodium et est 1 ou 2 pour M égal à platine ou palladium ;
m est 0, 1, 2, 3 ou 4;
les indices n et m sont ici choisis de telle sorte que le nombre de coordinations sur le métal corresponde à 6 pour M égal à iridium ou rhodium et corresponde à 4 pour M égal à platine ou palladium ;
les ligands d'une pluralité de ligands L peuvent ici également être liés les uns aux autres ou L peut être lié à L' via n'importe quel pont souhaité Z et ainsi former un système de ligands tridenté, tétradenté, pentadenté ou hexadenté.

2. Composé selon la revendication 1, **caractérisé en ce que** la moitié M(L)ₙ est choisie parmi le groupe des composés des formules (4) à (20) : 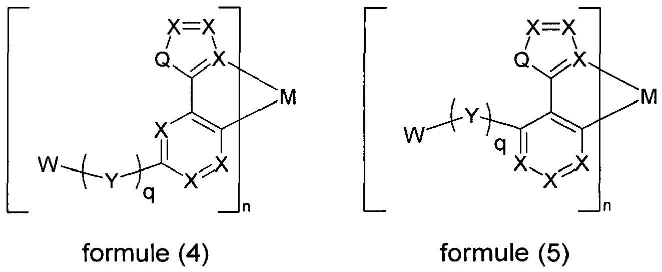 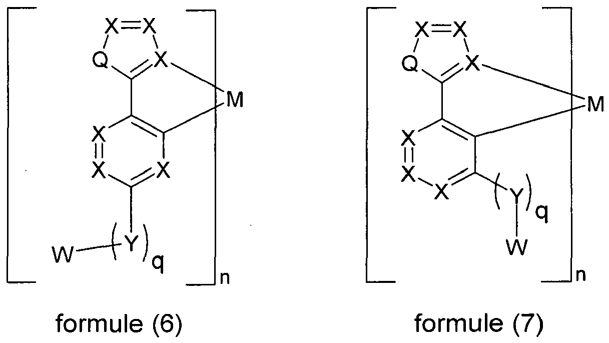 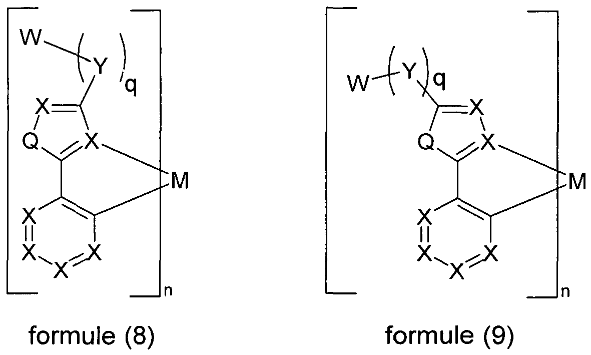 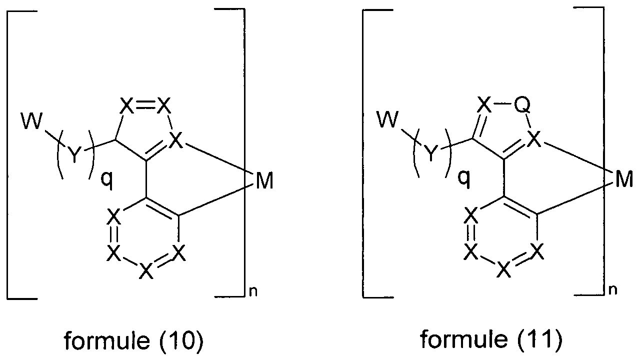 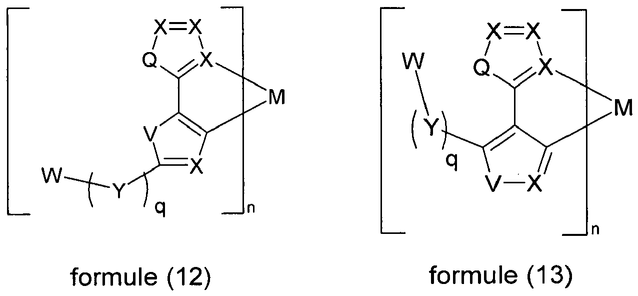 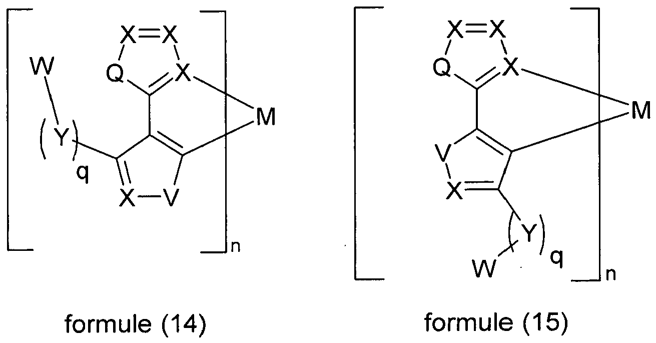 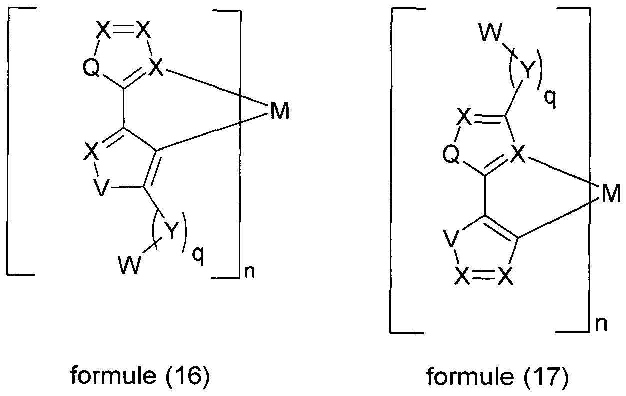 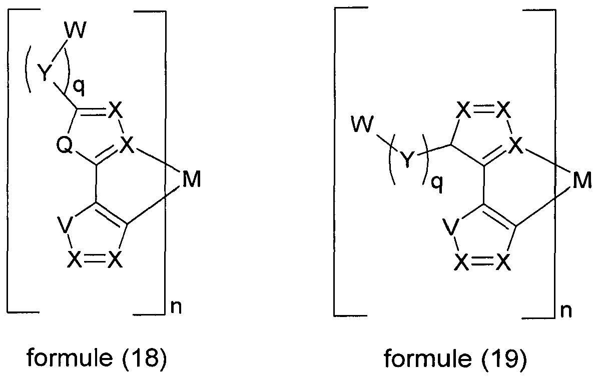 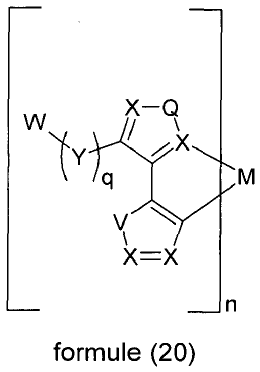 où ce qui suit s'applique aux symboles et indices utilisés :
Q est, de manière identique ou différente pour chaque occurrence, R¹C=CR¹, R¹C=N, O, S, Se ou NR¹ ;
V est, de manière identique ou différente pour chaque occurrence, O, S, Se, NR¹ ou C(R¹)₂.

3. Composé selon la revendication 1 ou 2, **caractérisé en ce que** la moitié M(L)ₙ est choisie parmi les formules (4), (9), (12) et (18) :

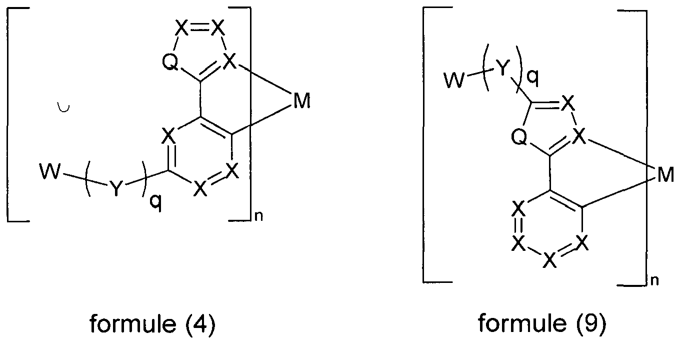

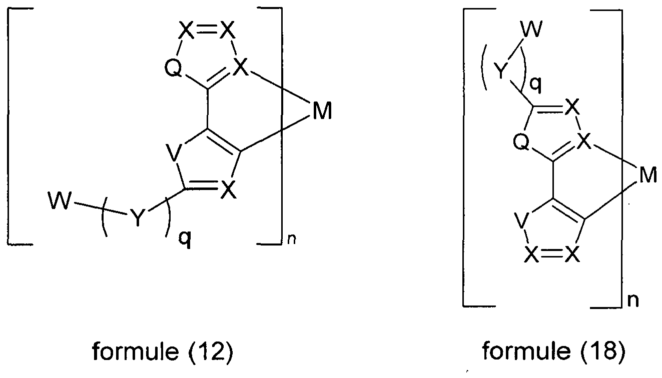

4. Composé selon une ou plusieurs des revendications 1 à 3, **caractérisé en ce que** la moitié M(L)ₙ est choisie parmi les formules (4) et (12) :

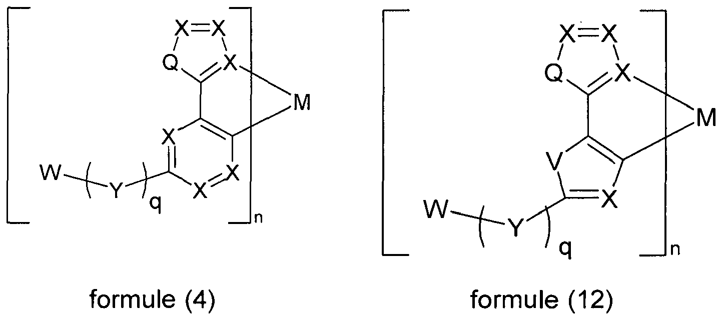

5. Composé selon une ou plusieurs des revendications 1 à 4, **caractérisé en ce que** le symbole X représente pour chaque occurrence CR¹ pour tous les atomes qui ne sont pas liés directement à M.

6. Composé selon une ou plusieurs des revendications 1 à 5, **caractérisé en ce que** la moitié M(L)ₙ est choisie parmi les formules (25) à (28) :

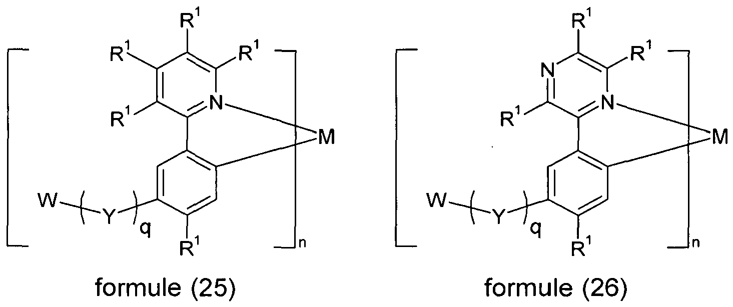

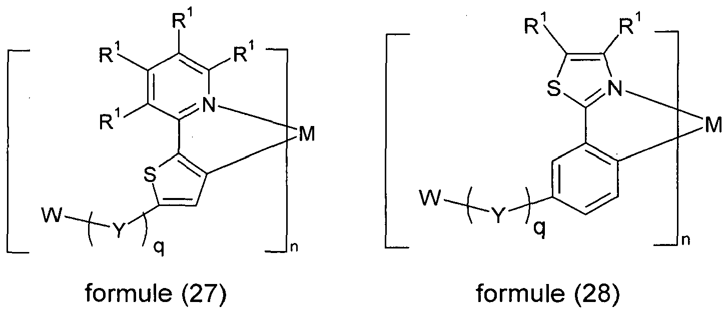

7. Composé selon une ou plusieurs des revendications 1 à 6, **caractérisé en ce que** W est choisi parmi les composés des formules (66) à (71) et (78) à (89) : 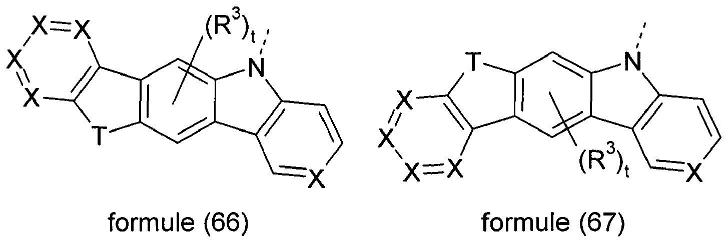 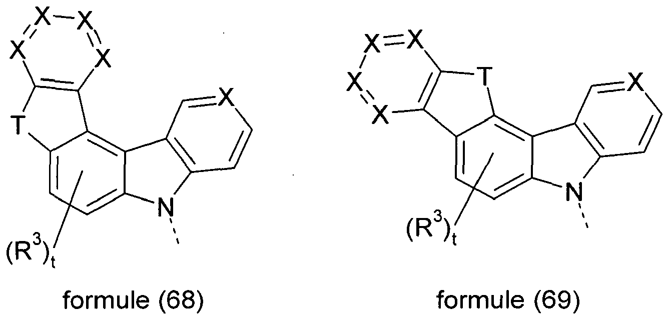 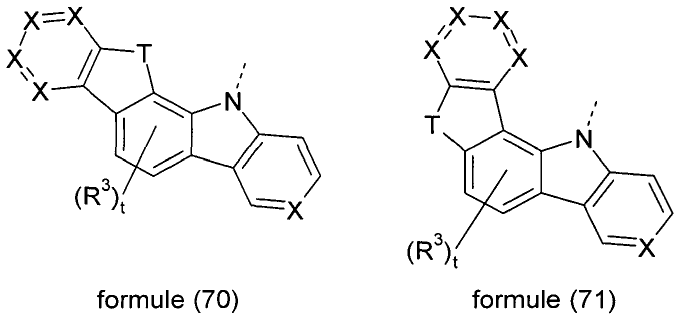 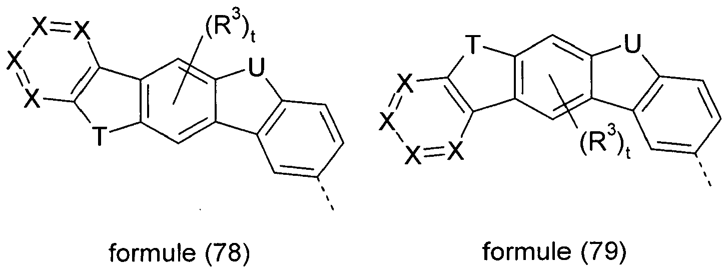 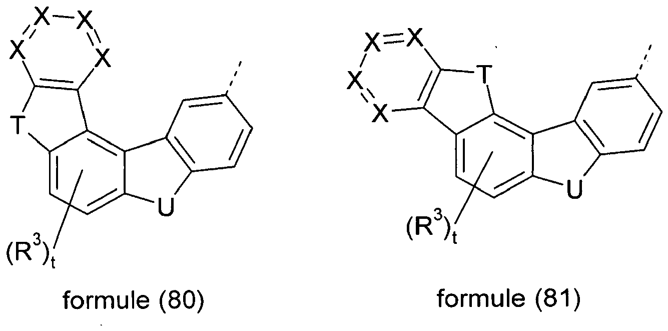 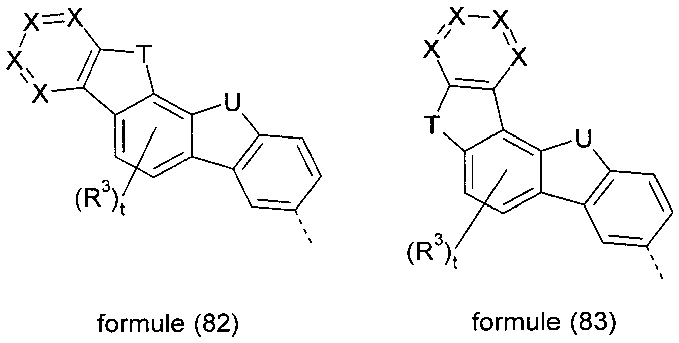 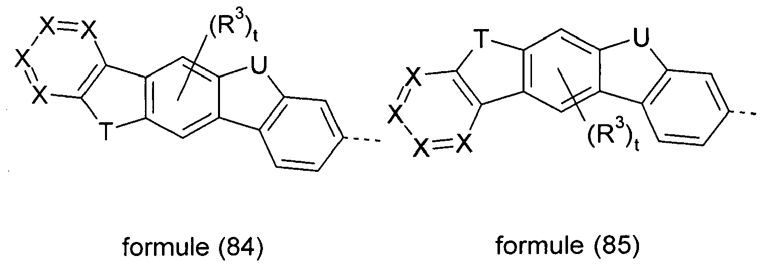 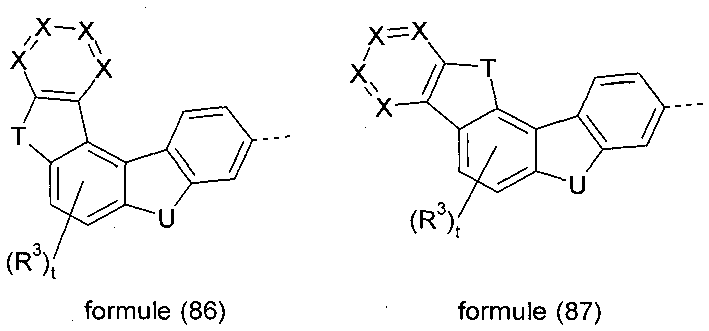 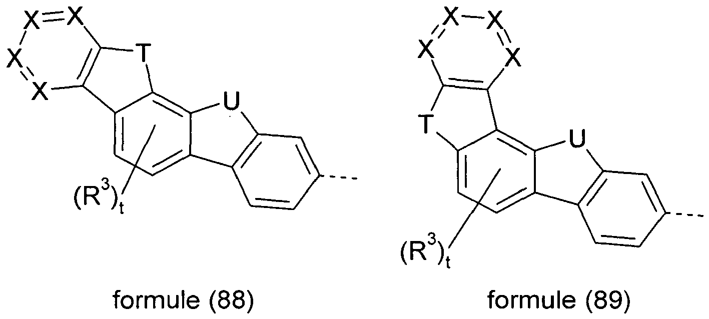 dans lesquelles les lignes en pointillés représentent la connexion entre W et Y.

8. Composé selon une ou plusieurs des revendications 1 à 7, **caractérisé en ce que** Y est, de manière identique ou différente pour chaque occurrence, une liaison simple ou un hydrocarbone aliphatique, aromatique ou hétéroaromatique comportant de 5 à 60 atomes, lesquels peuvent dans chaque cas être substitués par un ou plusieurs radicaux R³.

9. Composé selon une ou plusieurs des revendications 1 à 8, **caractérisé en ce que** le radical Y est choisi parmi les composés des formules (33) à (63) : 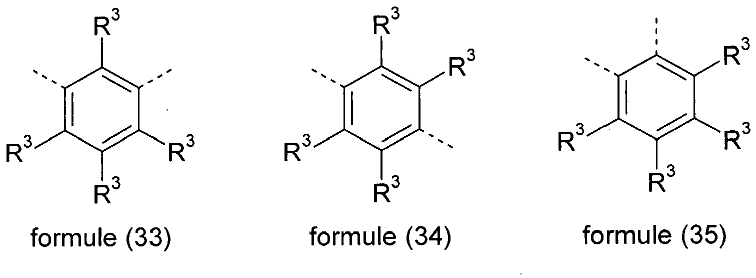 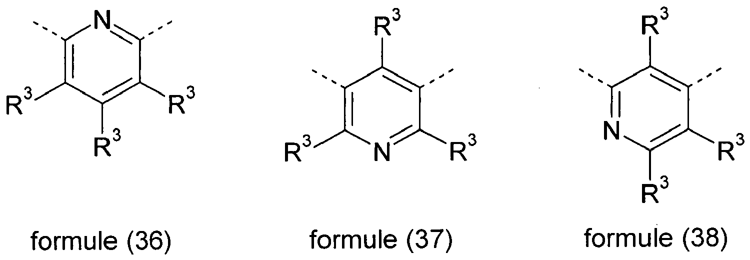 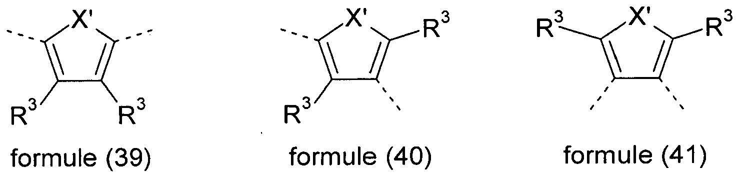 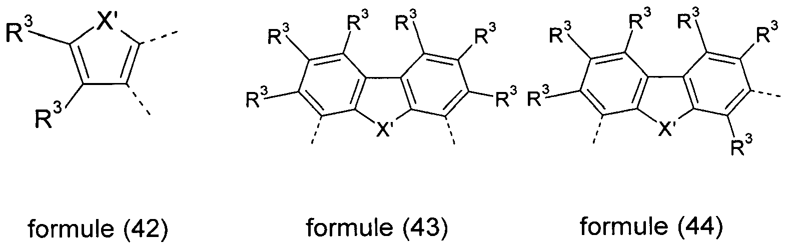 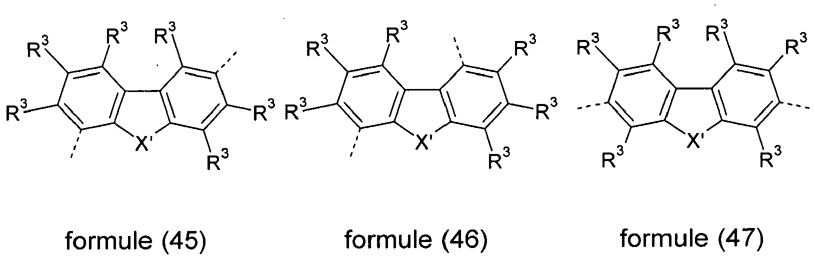 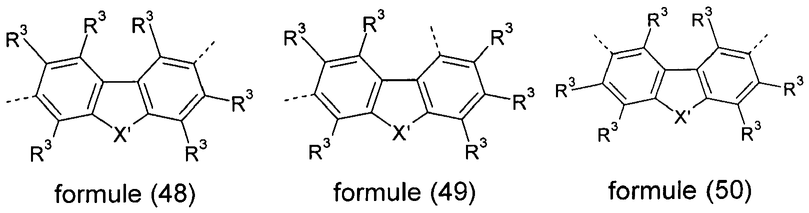 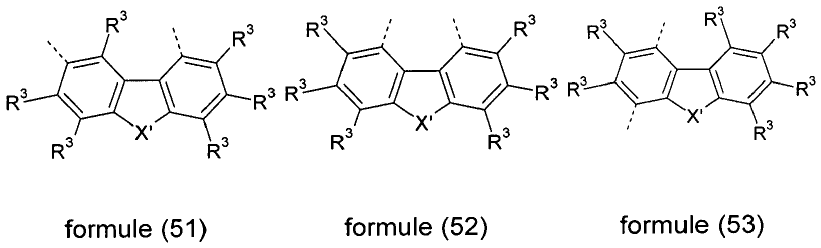 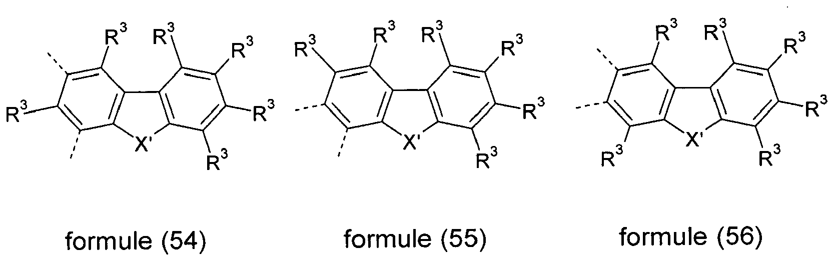 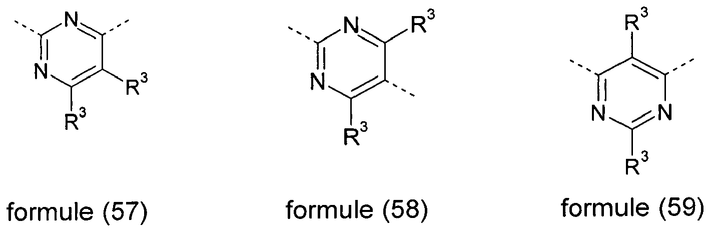 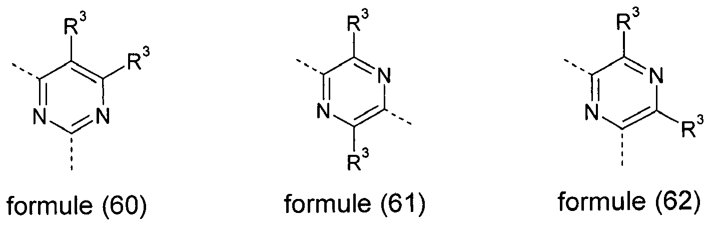 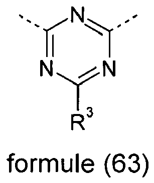 où X' est choisi parmi le groupe O, S, Se, NR¹, C(R¹)₂ ou S(=O)₂ et les lignes en pointillés représentent les liaisons sur W ou sur la partie du ligand organique qui est liée directement au métal M.

10. Procédé pour la préparation d'un composé selon une ou plusieurs des revendications 1 à 9 par réaction des ligands libres correspondants avec des alcoxydes métalliques de la formule (155), avec des cétocétonates métalliques de la formule (156) ou avec des halogénures métalliques de la formule (157) :
M(OR¹)ₙ formule (155) 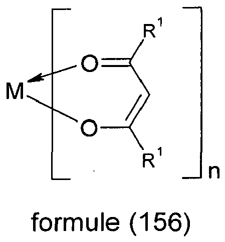
MHalₙ formule (157)
dans lesquelles les symboles M, n et R¹ présentent les significations indiquées ci avant et Hal = F, Cl, Br ou I.

11. Formulation comprenant au moins un composé selon une ou plusieurs des revendications 1 à 9 et au moins un solvant.

12. Utilisation d'un composé selon une ou plusieurs des revendications 1 à 9 dans un dispositif électronique, de façon préférable choisi parmi le groupe constitué par des dispositifs électroluminescents organiques (OLED, PLED), des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC) ou des diodes laser organiques (O-laser).

13. Dispositif électronique comprenant au moins un composé selon une ou plusieurs des revendications 1 à 9, de façon préférable choisi parmi le groupe constitué par des dispositifs électroluminescents organiques (OLED, PLED), des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC) ou des diodes laser organiques (O-laser).

14. Dispositif électroluminescent organique selon la revendication 13, **caractérisé en ce que** le composé selon une ou plusieurs des revendications 1 à 9 est utilisé en tant que composé d'émission dans une ou plusieurs couches d'émission, de façon préférable en combinaison avec un matériau de matrice, où le matériau de matrice est de façon préférable choisi parmi le groupe constitué par des cétones, des oxydes de phosphine, des sulfoxydes, des sulfones, des triarylamines, des dérivés de carbazole, des dérivés d'indolocarbazole, des dérivés d'indénocarbazole, des dérivés d'azacarbazole, des matériaux de matrice bipolaires, des silanes, des azaboroles, des esters boroniques, des dérivés de triazine, des complexes de zinc, des dérivés de diazasilole ou de tétraazasilole ou des dérivés de diazaphosphole ou des mélanges de ces matériaux de matrice.

15. Composé selon une ou plusieurs des revendications 1 à 9 ou dispositif selon la revendication 13 ou 14 pour la thérapie, la prophylaxie et/ou le diagnostic de maladies et/ou de conditions cosmétiques.
